# EUROPEAN PATENT SPECIFICATION

(11) **EP 0 805 678 B1**
(45) Date of publication and mention of the grant of the patent: **29.10.2003**
(21) Application number: 96903476.8
(22) Date of filing: 04.01.1996
(51) Int. Cl.: A61K 9/51

(54) **SURFACE-MODIFIED NANOPARTICLES AND METHOD OF MAKING AND USING SAME**
OBERFLÄCHEN-MODIFIZIERTE NANOPARTIKEL UND VERFAHREN FÜR IHRE HERSTELLUNG UND VERWENDUNG
NANOPARTICULES A MODIFICATION DE SURFACE ET LEURS PROCEDES DE FABRICATION ET D'UTILISATION

(30) Priority: 05.01.1995 US 369541; 16.02.1995 US 389893
(43) Date of publication of application: 12.11.1997
(73) Proprietor: THE BOARD OF REGENTS acting for and on behalf of THE UNIVERSITY OF MICHIGAN, Ann Arbor, MI 48109-1280 (US)
(72) Inventor: LEVY, Robert J., Ann Arbor, MI 48104 (US); LABHASETWAR, Vinod D., Ann Arbor, MI 48108 (US); SONG, Cunxian S., Ann Arbor, MI 48109-0576 (US)
(74) Representative: West, Alan Harry
(86) International application number: US9600476
(87) International publication number: WO96020698

(56) References cited:
- EP-A- 0 335 972
- EP-A- 0 336 964
- EP-A- 0 407 580
- EP-A- 0 529 711
- WO-A-90/10034
- WO-A-91/15193
- WO-A-92/17167
- WO-A-93/00076
- WO-A-94/18955
- WO-A-95/22963
- FR-A- 2 649 321
- JOURNAL OF CONTROLLED RELEASE, vol. 25, no. 1/02, 27 May 1993, AMSTERDAM (NL), pages 89-98, XP000361370 T. NIWA ET AL.: "PREPARATIONS OF BIODEGRADABLE NANOSPHERES OF WATER-SOLUBLE AND INSOLUBLE DRUGS WITH D.L.-LACTIDE/GLYCOLIDE COPOLYMER BY A NOVEL SPONTANEOUS EMULSIFICATION SOLVENT DIFFUSION METHOD, AND THE DRUG RELEASE BEHAVIOR"
- SCIENCE, vol. 263, 18 March 1994, WASHINGTON (US), pages 1600-1603, XP002013571 R. GREF ET AL.: "BIODEGRADABLE LONG-CIRCULATING POLYMERIC NANOSPHERES"
- JOURNAL OF CONTROLLED RELEASE, vol. 25, no. 1/02, 27 May 1993, AMSTERDAM (NL), pages 145-153, XP000361376 P.D. SCHOLES ET AL: "THE PREPARATION OF SUB-200NM POLY(LACTIDE-CO-GLYCOLIDE) MICROSPHERES FOR SITE-SPECIFIC DRUG DELIVERY"
- JOURNAL OF MICROENCAPSULATION, vol. 5, no. 2, 1988, LONDON (GB), pages 115-127, XP002013572 J. KREUTER: "POSSIBILITIES OF USING NANOPARTICLES AS CARRIERS FOR DRUGS AND VACCINES"
- JOURNAL OF CONTROLLED RELEASE, vol. 3, no. 1, January 1986, AMSTERDAM (NL), pages 15-23, XP002033952 S. J. DOUGLAS ET AL.: "poly(butyl 2-cyanoacrylate) nanoparticles with differing surface charges"

## Description

### Background of the Invention

### FIELD OF THE INVENTION

This invention relates to sustained release drug delivery systems and methods of making same. More particularly, the invention relates to surface-modified biodegradable nanoparticles for targeted delivery of bioactive agents, methods of making nanoparticles, novel polymeric compositions for making the nanoparticles, and methods of using same.

### DESCRIPTION OF THE RELATED ART

Site specific delivery of therapeutic agents for vascular diseases, or other local disorders such as cancer or infection, is difficult with systemic administration of drugs. Drugs administered orally, or by peripheral intravenous injection, are distributed throughout the patient's body and are subject to metabolism. The amount of drug reaching the desired site is frequently greatly diminished. Therefore, a larger dose of therapeutic agent is required, which in many cases, leads to unpleasant and unwanted systemic side effects. There is, therefore, a need for drug delivery systems which can be applied locally to treat regional disorders.

In many instances, intravascular administration of therapeutic agents would comprise a significant improvement in the art. However, there are special considerations which must be taken into account in the development of an intravascular drug delivery system. For example, For example, an intravascular drug delivery system must not cause clotting or thrombogenesis. Moreover, constant blood flow through the vasculature results in rapid dilution of the drug. There is, therefore, a need for a drug delivery system which can safely be delivered intravascularly and which can be retained at the site of administration to release therapeutic agent over a period of time.

Some of the foregoing and other disadvantages of the prior art can be overcome with injectable microparticles, and in particular, nanoparticles. Nanoparticles can enter cells and penetrate intracellular junctions. However, to date there have been no successful methods to confer antithrombogenic properties or cell adhesion properties to microparticles in order to enhance adhesion of the microparticles at the site of injection, such as the extracellular matrix in a vessel wall and the surrounding tissue, to facilitate drug retention.

Biodegradable sustained release nanoparticles for intravascular administration of therapeutic agents would be of extreme value in the treatment of cardiovascular disease such as restenosis, for example. Re-obstruction of coronary arteries or other blood vessels, after angioplasty, has generally been termed restenosis. Typically, within six months of coronary angioplasty, about 30% to 50% of the treated coronary lesions undergo restenosis. The processes leading to restenosis likely involve a combination of acute thrombosis following damage to the arterial wall imposed on a background of pre-existing arterial disease. The types of active agents which would be useful for site-specific treatment to mitigate and/or prevent restenosis cover a broad range, including antithrombogenic agents, growth factors, DNA, oligonucleotides, antiplatelet drugs, immune modulators, smooth muscle cell inhibitors, cytokines, anti-inflammatory agents, and anti-atherosclerosis agents (*e.g.*, antilipid agents or anticalcification agents).

Various drug delivery strategies have been devised for pharmacological intervention to prevent restenosis. One such strategy involves the invasive placement of periadventitial drug deliver systems, comprising controlled release polymer preparations, on the outside of blood vessels. Expandable balloon angioplasty stents having drug-polymer coatings have also been investigated. The stent devices are limited, however, to use in situations requiring stent angioplasty and suffer the further disadvantage that the amount of drug and polymer that can be contained in the system is limited to the surface area of the struts and wires comprising the stent. Another known approach for preventing restenosis is regional drug therapy involving segmental arterial infusions of drugs of interest to retard the events that lead to restenosis. The results achieved by the known system have been relatively ineffective due to rapid wash-out of drug by the blood flow. There is, thus, a need for sustained release drug delivery devices for local, regional, and/or targeted administration of a variety of therapeutic or bioactive agents, to sites, such as the vasculature. Of course, the same need exists in many diverse applications, such as gene therapy, cancer therapy, treatment of localized infections and inflammatory reactions, and diagnostic imaging.

One of the problems encountered in the development of sustained release drug delivery devices has been finding a suitable biocompatible, bioerodable polymer to serve as a matrix or depot for the therapeutic agent. A variety of biodegradable polymers have been synthesized and used in the practice of medicine. However, most of these biodegradable polymers are unsuitable for the manufacture of sustained release drug delivery systems, particularly nanoparticles. A commonly used polymer is the polyester, polylactic-polyglycolic acid copolymer (PLGA). While PLGA is biocompatible, it degrades relatively rapidly. Thus, the use of PLGA for long-term sustained release drug delivery systems has been limited. In addition, due to the limited number of hydroxyl groups on PLGA, it has been difficult to chemically link a significant amount of bioactive agent to the polymer chain. There is, therefore, a need for a means of providing PLGA, and other non-reactive polymers, with more reactive functional groups for subsequent chemical modification and/or linking with bioactive agents of interest. There is also a need in the art for biocompatible polymers which have long-term bioerosion characteristics.

Polycaprolactone, another biodegradable polymer used in the medical field, has long-term sustained release potential. In fact, polycaprolactones have been used for contraceptive systems incorporating hydrophobic agents, such as steroids. Unfortunately, polycaprolactones are not useful for hydrophilic agents, or for rapid release applications. Polycaprolactone also lacks reactive functional groups that can be used to derivatize, or chemically modify, the polymer. It would be advantageous to form a new biodegradable polymer, containing the hydrophobic polycaprolactone block, but with more desirable hydrophilic characteristics, rapid biodegradation kinetics, and the potential for further derivatization (*e.g*., through the addition of reactive epoxy groups).

Some researchers have synthesized polylactone-polyether block copolymers by initiating polymerization of lactone monomers using a poly-glycol as an alcoholic-type initiator. However, this technique results in the formation of a BAB-type block copolymer wherein the hydrophilic segment is in the middle of the block copolymer. This technique has the further disadvantage that only low molecular weight polymers can be formed. There is a need for a technique which chemically links hydrophobic and hydrophilic copolymer blocks in ABA, BAB, as well as (AB)ₐ, form so that hydrophobicity and molecular weight of the block copolymers can be tailored as desired. There is an even greater need for block copolymers having reactive functional groups, such as hydroxyl groups, on both ends for ready chemical modification, such as coupling to heparin, albumin, vaccines, or other biomolecules of interest.

It is, therefore, an object of this invention to provide a biocompatible biodegradable sustained release drug delivery system for local and/or targeted administration of a variety of therapeutic or bioactive agents.

It is another object of this invention to provide a sustained release drug delivery system for catheter-based local drug delivery at any site which can be accessed through the vasculature, or by other interventional means.

It is also an object of this invention to provide methods of making sustained release drug delivery systems which comprise biocompatible biodegradable polymers, and nanoparticles in particular.

It is a further object of this invention to provide methods of making sustained release drug delivery systems which comprise biocompatible biodegradable nanoparticles having improved properties, such as targeting ability, retention capability, anti-thrombogenicity, and the like.

It is yet an additional object of this invention to provide a method of making ultrasmall nanoparticles (*e.g*., 20 to 35 nm in diameter).

It is additionally an object of this invention to provide an improved biocompatible, biodegradable polymer having, hydrophobic and hydrophilic characteristics, which is suitable for making sustained release drug delivery systems.

It is yet a further object of this invention to provide an improved biocompatible, biodegradable polymer having reactive functional groups on the surface which are suitable for chemical modification and/or linking with bioactive agents of interest.

It is also another object of this invention to provide a method to confer reactivity, or to activate, the surface of biocompatible, biodegradable polymers which are otherwise relatively inert.

### Summary of the Invention

The foregoing and other objects are achieved by this invention which provides a sustained release drug delivery system comprising surface-modified nanoparticles. The nanoparticles are a core of biodegradable, biocompatible polymer or biomaterial. At least one multifunctional epoxide linking compound is covalently linked to the biodegradable polymer. The average diameter of the nanoparticles of the present invention is typically less than 300 nm, preferably in the range of 100 nm to 150 nm, and more preferably 10 nm to 50 nm, with a narrow size distribution.

The polymeric core may have a bioactive, or bioinactive, agent or combination of agents, incorporated, embedded, entrained, or otherwise made part of the polymer matrix comprising the nanoparticle core. The incorporated bioactive agent is released as the polymer hydrolyzes and dissolves, thereby biodegrading.

As used herein, the terms "biocompatible polymer" or "biomaterial" denote any synthetic or naturally-derived polymeric material which is known, or becomes known, as being suitable for in-dwelling uses in the body of a living being, *i.e*., is biologically inert and physiologically acceptable, non-toxic, and, in the sustained release drug delivery systems of the present invention, is biodegradable or bioerodable in the environment of use, *i.e*., can be resorbed by the body.

Illustrative biomaterials suitable for use in the practice of the invention include naturally-derived polymers, such as acacia, chitosan, gelatin, dextrans, albumins, alginates/starch, and the like; or synthetic polymers, whether hydrophilic or hydrophobic.

Biocompatible, biodegradable synthetic polymers which may be used to formulate nanoparticles include, but are not limited to, polyesters, such as polylactides, polyglycolides, and polylactic polyglycolic copolymers (PLGA); polyethers, such as such as hydroxy-terminated poly (∈-caprolactone)-polyether or polycaprolactone (PCL); polyanhydrides; polyalkylcyanoacrylates, such as n-butyl cyanoacrylate; polyacrylamides; poly(orthoesters); polyphosphazenes; polyamino acids; and biodegradable polyurethanes. It is to be understood that the term polymer is to be construed to include copolymers and oligomers.

In a preferred embodiment, the biocompatible, biodegradable synthetic polymer is polylactic polyglycolic acid co-polymer (PLGA; available from Birmingham Polymers, Inc, Birmingham, Alabama). PLGA, for example, is FDA approved and currently used for surgical sutures. Additionally, PLGA is commercially available in a wide range of molecular weights with various biodegradation characteristics. PLGAs suitable for use in the practice of the invention have molecular weights in the range of from about 30,000 to 700,000, typically 30,000 to 90,000, with an intrinsic viscosity ranging from 0.5 to 10.5.

In another preferred embodiment of the invention, the biocompatible, biodegradable synthetic polymer is a polycaprolactone; specifically, novel polycaprolactone-based multiblock copolymers which contain hydrophobic and hydrophilic segments. In a particularly preferred embodiment, the multiblock copolymer is epoxy-derivatized and surface-activated as will be discussed more completely hereinbelow.

JOURNAL OF CONTROLLED RELEASE, vol. 25, no. 1/02, 27 May 1993, AMSTERDAM (NL), pages 145-153, XP000361376 P.D. SCHOLES ET AL: "THE PREPARATION OF SUB-200NM POLY(LACTIDE-CO-GLYCOLIDE) MICROSPHERES FOR SITE-SPECIFIC DRUG DELIVERY" discloses the production of poly(lactide-co-glycolide) particles having diameters as low as 90nm.

As used herein, the term "bioactive agent" means a chemical compound, or combination of compounds, naturally-occurring or synthetic, which possess the property of influencing the normal and pathologic behavior of living systems. A bioactive can be therapeutic, diagnostic, prophylactic, cosmetic, nutritional, *etc*. In some cases, the bioactive agent can be bioinactive in the broad sense; an excipient or filler, an adjuvant, which will act in conjunction, or combination, with one or more other bioactive agents; or a surface modifying agent as will be defined more completely hereinbelow.

Of course, the term "bioactive agent" includes pharmaceutical agents, alone or in combination with other pharmaceutical agents and/or bioactive agents.

In preferred embodiments of the invention, the pharmaceutical agent is a cardiovascular agent, particularly a cardiovascular agent which is useful for the treatment of restenosis of vascular smooth muscle cells. The cardiovascular agent may be a stimulator, such as platelet derived growth factor, endothelial cell growth factor, fibroblast growth factor, smooth muscle cell-derived growth factors, Interleukin 1 and 6, transforming growth factor-β, low density lipoprotein, vasoactive substances (Antiotension II, epinephrine, nortpinephrine, -5HT, neuropeptide substances P&K, endothelin), thrombin, leukotrins, prostaglandins (PGE₂, PGL₂), epidermal growth factors, oncogenes (c-myb, c-myo, fos), or proliferating cell nuclear antigen; inhibitors such as transforming growth factor-β, heparin-like factors, or vasorelaxant substances; antithrombins, such as heparin, hirudin, or hirulog; antiplatelet agents, such as aspirin, dipyridamole, sulfinpyrazone, salicylic acid, eicosapentaenoic acid, ciprostene, and antibodies to platelet glycoprotein IIb/IIIa; calcium channel blockers, such as nifedipine, verapamil, diltiazem; antitensin converting enzyme (ACE) inhibitors, such as captopril or cilazapril; immunosuppressants, such as steroids or cyclosporin; fish oils; growth factor antagonists, such as angiopeptin or trapidil; cytoskeletal inhibitors, such as cytochalasins; antiinflammatory agents, such as dexamethasone; thrombolytic agents, such as streptokinase or urokinase; and antiproliferatives, such as colchicine or U-86983 (provided by the Upjohn Company, Kalamazoo, MI; hereinafter "U86"); genetic material suitable for the DNA or anti-sense treatment of cardiovascular disease; protein kinase inhibitors, such as staurosporin or the like; smooth muscle migration and/or contraction inhibitors such as the cytochalasins, suramin, and nitric oxide-releasing compounds, such as nitroglycerin, or analogs or functional equivalents thereof. In a particularly preferred embodiment, directed to the treatment of restenosis, the bioactive agent is the cytoskeletal inhibitor, cytochalasin B.

Of course, genetic material for the DNA or anti-sense treatment of cardiovascular disease is specifically included. Illustrative examples are platelet-derived growth factor, transforming growth factors (alpha and beta), fibroblast growth factors (acidic and basic), angiotensin II, heparin-binding epidermal growth factor-like molecules, Interleukin-1 (alpha and beta), Interleukin-6, insulin-like growth factors, oncogenes, proliferating cell nuclear antigen, cell adhesion molecules, and platelet surface antigens.

In still other embodiments of the invention, the bioactive agent is a protein or peptide-based vaccine, such as bacterial vaccines, including tetanus, cholera toxin, Staphylococcus enterotoxin B, Pertussis, pneumococcus, Staphylococcus and Streptococcus antigens, and others, E. Coli (enteropathogenic); and viral vaccine proteins, such as all AIDS antigens, viral proteins (*e.g*., influenza virus proteins, adenovirus, and others), live virus in microcapsules (*e.g*., attenuated poliovirus), Hepatitis viral components, and Rotavirus components; viral and bacterial polysaccharides; and DNA-based vaccines. In a particularly preferred embodiment, the protein-based vaccine is Tetanus-Toxoid.

In other embodiments, directed to the treatment of cancer, the bioactive agent is an anticancer agent, illustratively alkylating agents, such as mechiorethamine, cyclophosphamide, ifosfamide, mephalan, chlorambucil, hexamethylmelamine, thiotepa, busulfan, carmustine, lomustin, lomustine, semustine, steptozocin, dacarbazine; antimetabolites, such as methotrexate, fluorouracil, floxuridine, cytarabine, mercaptopurine, thioguanine, pentostatin; natural products, such as alkaloids (*e.g*., vinblastine or vincristine), toxins *(e.g*., etoposide or teniposide), antibiotics (*e.g*., such as dactinomycin, daunorubicin, bleomycin, plicamycin, mitomycin), and enzymes, (*e.g*., L-asparaginase); biological response modifiers, such as Interferon-α; hormones and antagonists, such as adrenocortocoids (*e.g*., dexamethasone), progestins, estrogens, anti-estrogens, androgens, gonadotropin releasing hormone analogs; miscellaneous agents, such as cisplastin; mitoxantrone, hydroxyurea, procarbazine or adrenocortical suppressants (*e.g*., mitotane or aminoglutethimide). Other examples, specifically include, anticancer genes, such as tumor suppressor genes, such as Rb and P⁵³, cytokine-producing genes, tumor necrosis factor α-cDNA, carcinoembryonic antigen gene, lyphokine gene, toxin-mediated gene therapy, and antisense RNA of E6 and E7 genes.

Bioactive agents useful in the practice of the invention, include, without limitation, enzymes, such as coagulation factors (prothrombin), cytokines (platelet-derived growth factor, fibroblast growth factor), cell adhesion molecules (I-Cam, V-Cam, integrin); transport proteins, such as albumin, ferritin, transferrin, calmodulin; and biologically active peptides, such as those containing arginine-glycine-alanine (RGD sequence); biopolymers, such as nucleic acids (DNA, RNA, oligonucleotides (sense and antisense DNA and RNA), protamine, collagen, elastin, matrix proteins (*e.g*., glycoproteins, agrican, glycan); carbohydrates, such as mono- and polysaccharides, dextran, agar, agarose derivatives, monomeric and polymer-crosslinked polysaccharides; protoglycans, such as heparin, heparan, dermatan-sulfate, and related macromolecules; lipids, such as phospholipids, cholesterol, triglycerides, lipoproteins, apolipoproteins; synthetic agents, such as detergents, pharmaceuticals (specifically including bisphosphonates, ion channel agents, and calcium channel blockers), imaging agents, and polymers, such as cyanoacrylates, polyamine acids; and crystalline salts, such as osteoconductive salts which are conducive to bone-mineral formation, such as calcium phosphates, hydroxyapatite, octacalcium phosphate, tricalcium phosphate, or trace metals, such as ferric chloride, alumina, aluminum chloride, or zinc, magnesium, or cobalt salts.

In still further embodiment, the bioactive agent is a nucleic acid, specifically an RNA, DNA, oligonucleotides of RNA or DNA (sense and antisense). Specifically, included are osteotropic gene or gene segments, such as bone morphogenic proteins (BMP2 and 4 and others), transforming growth factor, such as TGF-β1-3, activin, phosphoproteins, osteonectin, osteopontin, bone sialoprotein, osteocalcin, vitamin-k dependent proteins, glycoproteins, and collagen (at least I and II).

As used herein, the term "surface modifying agent" is defined as any chemical or biological compound, which may be a bioactive agent, having the property of altering the surface of nanoparticles so as to perform one or more of the following functions: to target binding of the nanoparticles to tissues or cells of living systems, to enhance nanoparticle sustained release properties, including retention at the site of administration, to protect nanoparticle-incorporated bioactive agents, to impart antithrombolytic effects, to improve suspendibility, and to prevent aggregation.

Surface modifying agents include, but are not limited to, various synthetic polymers, biopolymers, low molecular weight oligomers, natural products, and surfactants.

Synthetic polymers which are useful as surface modifying agents include carboxymethyl cellulose, cellulose, cellulose acetate, cellulose phthalate, polyethylene glycol (Carbowax), polyvinyl alcohol (PVA), hydroxypropylmethyl cellulose phthalate, hydroxypropyl cellulose, sodium or calcium salts of carboxymethyl cellulose, noncrystalline cellulose, polaxomers such as Pluronic™ F68 or F127 which are block copolymers of ethylene oxide and propylene oxide available from BASF, Parsippany, NJ, poloxamines (Tetronic 908, *etc.),* dextrans, swellable hydrogels which are mixtures of dextrans, such as diethyl amino-ethyl dextran (DEAE-dextran), polyvinyl pyrolidone, polystyrene, and silicates, such as Bentonite or Veegum.

Natural products, include proteins and peptides, such as acacia, gelatin, casein, albumins (ovalbumin, human albumins, *etc*.), myoglobins, hemoglobins, and sugar-containing compounds, such as tragacanth, sugars, such as sorbitol or mannitol, polysaccharides (*e.g*., ficoll), and pectin. Various lipids are specifically included, such as lecithin, phospholipids, cholesterol, beeswax, wool fat, sulfonated oils, and rosin soap.

Proteins and peptides specifically contemplated to be within the invention include vascular smooth muscle binding proteins, illustratively, monoclonal and polyclonal antibodies, F(ab')₂, Fab', Fab, and Fv fragments of antibodies, growth factors, cytokines, polypeptide hormones, macromolecular recognizing extracellular matrix receptors (such as integrin and fibronectin receptors and the like); peptides for intracellular stroma and matrix localization, such as any peptide having an affinity for extracellular glycoprotein (*e.g*., tenascin), collagen, reticulum, or elastic fibers.

In embodiments directed to cancer therapy, for example, surface modifying agents include tumor cell binding proteins, such as those associated with epitopes of myc, ras, bcr/Abl, erbB, mucin, cytokine receptors (*e.g*., IL-6, EGF, TGF, myc) which localize to certain lymphomas (myc), carcinomas, such as colon cancer (ras), carcinoma (erbB), adenocarcinoma (mucins), breast cancer and hepatoma (IL-6 receptor), breast cancer (EGF and TGF), respectively.

In embodiments directed to immunization, surface modifying agents include toxins and toxoids, such as cholera toxin or toxoid, or fragments of same B-chain) to enhance its uptake or increase immunogenicity. Other surface modifying agents specifically include immunostimulants, such as muramyl dipeptide, block co-polymers (*e.g*., Pluronics), lipid A, and the vaccine antigen of the entrapped vaccine.

Illustrative non-ionic surfactants which serve a surface modifying agents are polyoxyethylene sorbitan fatty acid esters (available commercially from Hercules, Inc., Wilmington, DE under the trademark Tween), sorbitan fatty acid ester (available commercially from Hercules, Inc. under the trademark Span, fatty alcohols, such as cetyl alcohol or stearyl alcohol, alkyl aryl polyether sulfonate (available from Sigma Chemicals, St. Louis, MO under the trademark Triton X), dioctyl ester of sodium sulfonsuccinic acid (available from Atlas Powder Company, Wilmington, DE under the trademark Aerosol OT®). Anionic surfactants include sodium dodecyl sulfate, sodium and potassium salts of fatty acids (sodium oleate, sodium palmitate, sodium stearate, *etc.*), polyoxyl stearate (Mryj®, Atlas Powder Company), polyyoxylethylene lauryl ether (Brij®, Atlas Powder Company), sorbitan sesquioleate (Aracel®, Atlas Powder Company) triethanolamine, fatty acids, such as palmitic acid, stearic acid, and glycerol esters of fatty acids, such as glycerol monostearate. Exemplary cationic surfactants include didodecyldimethyl ammonium bromide (DMAB), cetyl trimethyl ammonium bromide, benzalkonium chloride, hexadecyl trimethyl ammonium chloride, dimethyldodecylaminopropane and N-cetyl-N-ethyl morpholinium ethosulfate (Atlas G-263, Atlas Powder Company).

The aforementioned bioactive agents and surface modifying agents are illustrative only. Any bioactive agent and/or surface modifying agent which can be incorporated into a biocompatible, biodegradable matrix and/or attached to the surface of polymer, such as by coating or covalent attachment, is within the contemplation of the invention herein. Broadly, the classification of bioactive agents has been broken down into categories depending on the method used to incorporate them in nanoparticles based on the hydrophobicity/hydrophilicity *of* the agent.

In accordance with a method embodiment of the present invention, nanoparticles may be prepared by what is generically termed herein as an "in-solvent emulsification-evaporation" technique using single (oil-in-water) or multiple emulsifications (water-in-oil-in-water) depending upon whether the incorporated bioactive agent is hydrophobic or hydrophilic, or a protein/peptide-based hydrophilic agent, such as DNA-containing agents. For a semipolar bioactive agent, a co-solvent system using a combination of polar and nonpolar solvents is used to form a single organic phase to dissolve both the bioactive agent and polymer which, when emulsified in an aqueous phase, forms an oil-in-water emulsion.

For hydrophobic bioactive agents, the polymer and hydrophobic active agent(s) are dissolved in an organic solvent. The organic solution is added drop-wise to an aqueous solution of a detergent, surfactant, or other emulsifying agent, with sonification (15 to 65 Watts energy output over a period of 30 seconds to 20 minutes, preferably about 10 minutes) to form a stable emulsion. The sonification takes place over an ice bath in order to keep the polymer from melting. Emulsifying agent is typically present in the aqueous solution in an amount ranging from about 0.1% to 10% w/v, and preferably about 1% to 3% w/v. The organic solvent is evaporated from the emulsion. The nanoparticles are separated from the remaining aqueous phase by centrifugation, or preferably ultracentrifugation (120,000 to 145,000 g), washed with water, and re-centrifuged and decanted.

The washed nanoparticles are resuspended in water by sonication (illustratively, 65 Watts for one minute over an ice bath) and, in some embodiments, lyophilized for storage and/or subsequent processing. Lyophilization is done by first freezing the nanoparticle suspension over dry ice for 30-60 minutes and then lyophilizing in a lyophilizer (such as Model FM 3SL plus, sold by The Virtis Company, Inc., Gardiner, NY) at temperatures of from about ranging from bout -30° C to -55° C under a vacuum of 500 millitorr or less for a period of time of at least 24-48 hours. In specific embodiments herein, lyophilization was conducted at a temperature of -55° C and vacuum at 55 millitorr for 24-48 hours. The lyophilized nanoparticles are stored at 4° C in an anhydrous environment.

The nanoparticles are stored in a desiccated form inasmuch as water can erode the polymer. The nanoparticles may be sterilized by radiation, such as gamma radiation (2.5 Mrad) or electron beam technology, as is known in the art. In the alternative, the nanoparticles may be prepared in a sterile environment, using sterile components. Of course, other means of sterilizing the nanoparticles can be employed. In addition, the nanoparticles may be stored at room temperature, but are preferably stored at 4° C.

Suitable surfactants useful in the practice of method embodiments of the present invention, for making oil-in-water emulsions (*e.g*., Examples 1, 8, and 20), include without limitation, polyvinyl alcohol; polyoxyethylene sorbitan fatty acid esters sold commercially under the trademark Tween (Hercules, Inc, Wilmington, DE); polyethylene glycols; triethanolamine fatty acid esters, such as triethanolamine oleate; sodium or potassium salts of fatty acids, such as sodium oleate; sodium lauryl sulphate; cellulose acetate; polaxomers such as Pluronic™ F68 or F127 which are block copolymers of ethylene oxide and propylene oxide available from BASF; and quaternary ammonium compounds, such as didodecyldimethyl ammonium bromide (DMAB). For making water-in-oil emulsions (*e.g*., first emulsion in multiple emulsion Examples 5 and 10), sorbitan esters of fatty acids, such as those marketed under the trademark Span by Hercules, Inc., fatty alcohols, fatty acids, and glycerol esters of fatty acids, such as glycerol monostearate, are preferred.

For hydrophilic bioactive agents, a technique using a co-solvent system has been developed. The polymer is dissolved in a nonpolar organic solvent, such as methylene chloride, chloroform, ethyl acetate, tetrahydrofuran, hexafluoroisopropanol, or hexafluoroacetone sesquihydrate. The water-soluble bioactive agent is dissolved in a semipolar organic solvent, such as dimethylacetamide (DMAC), dimethylsulfoxide (DMSO), dimethylformamide (DMF), dioxane, and acetone. When combined, the result is an organic phase incorporating both polymer and bioactive agent. The organic phase is emulsified in an aqueous solution of an emulsifying agent as described with respect to the technique for hydrophobic bioactive agents.

In some embodiments, an agent can be added to the organic solution to favor partitioning of the hydrophilic bioactive agent into the organic phase upon solidification of the nanoparticles. As an example, a fatty acid salt, such as sodium palmitate, an anionic agent which forms a complex with a cationic drug, such as ibutilide, to force the ibutilide into the organic phase. Other agents which favor partitioning into the organic phase include agents that affect the pH of the aqueous phase, or that increase the viscosity of the aqueous phase. Specific examples of agents favor partitioning, include without limitation, cationic and anionic lipids (depending upon the charge of the bioactive agent), and multivalent, polycationic agents, such as protamine or polyamino acids, including polylysine and polyarginine.

While proteins and vaccine antigens, for example, are highly water-soluble, a multiple emulsion technique was developed for forming protein-containing nanoparticles. In this technique, the water-soluble proteins are dissolved in distilled water to form a first aqueous phase. The polymer is dissolved in a nonpolar organic solvent such as chloroform or methylene chloride. The protein-containing aqueous solution is emulsified in the organic solution with sonification to form a water-in-oil primary emulsion. A secondary emulsion is formed by emulsifying the primary emulsion into an aqueous solution of an emulsifying agent to form a water-in-oil-in-water emulsion. The organic solvent is then evaporated from the water-in-oil-in-water emulsion. The resulting nanoparticles are separated from the remaining aqueous phase by centrifugation, washed, and lyophilized as previously described.

The surface of the pre-formed biocompatible, biodegradable nanoparticle core may be modified to obtain various advantages. For intravascular targeting of local drug therapy, for example, it would be useful to enhance retention of the nanoparticles by the arterial wall by incorporating fibronectin, for example. For use as a vaccine, it would be useful to enhance the immunogenicity of the particles for better adjuvant properties. In this case, immunostimulants, such as muramyl dipeptide, Interleukin-2, Lipid A, and the vaccine antigen, such as cholera toxin or the B-chain of cholera toxin, could be incorporated and/or adsorbed to the surface of the nanoparticles. Of course, the possibilities are numerous, and specifically include antithrombogenic agents and mucoadhesives, for example.

Other advantages include targeting to cells, proteins, or matrix, protection of the incorporated bioactive agent, and enhancement of sustained release characteristics. In addition to the foregoing, the surface can be modified to increase shelf life, such as by building-in a desiccant to prevent aggregation. Moreover, placing a surfactant or detergent on the surface, such as DMAB, or a sugar or polysaccharide, such as mannitol, ficoll, or sucrose, mitigates against the need to sonicate when the stored, desiccated nanoparticles are resuspended prior to use.

Surface modification of pre-formed nanoparticles is particularly advantageous since it avoids complications with chemical compatibility which could lead to failure of particle formation, In a method aspect, the surface of pre-formed nanoparticles can be modified by adsorbing, or physically adhering, at least one surface modifying agent to the nanoparticles, without chemical bonding.

One advantageous method for adsorbing a surface modifying agent to the nanoparticles comprises the steps of suspending the nanoparticles in a solution of the surface modifying agent, or agents, and freeze-drying the suspension to produce a coating on the nanoparticles. In this preferred method embodiment, the pre-formed nanoparticles are suspended in a solution of surface modifying agent in distilled water, in a concentration ranging from about 0.5% to 15% w/w, and preferably about 5%. Typically the suspension contains about 100 mg to 1 gram of nanoparticles, and in the embodiments presented herein, about 200 mg.

The surface modifying agent is covalently linked to the pre-formed nanoparticles. In a preferred advantageous embodiment of the invention, a method has been developed to incorporate reactive epoxide side chains into the polymeric material comprising the nanoparticles, which reactive side chains can covalently bind other molecules of interest for various drug delivery applications. This technique is particularly useful inasmuch as the polylactic polyglycolic acid co-polymers widely used in drug delivery research for biodegradable formulations inherently lack reactive groups, and therefore, are difficult to derivatize.

In a method aspect, the nanoparticles are subjected to at least partial hydrolysis to create reactive groups on the surface which, in the case of PLGA, are hydroxyl groups. However, it is to be understood that the reactive functional groups on the polymer may also be amino, anhydrides, carboxyl, hydroxyl, phenol, or sulfhydryl. After reactive functional groups are created, the nanoparticles are then contacted with reactive multifunctional epoxide compounds to form epoxy-activated nanoparticles. The epoxy-activated nanoparticles will chemically bond to reactive groups on bioactive agents, which reactive groups may be amino, anhydrides, carboxyl, hydroxyl, phenol, or sulfhydryl.

The epoxy compounds suitable for the practice of the present invention may be monomers, polyepoxide compounds, or epoxy resins. Illustrative reactive epoxides suitable for use in the practice of the invention include, without limitation, 1,2-epoxides such as ethylene oxide or 1,2-propylene oxide; and bifunctional or polyfunctional compounds, such as butane and ethane di-glycidyl ethers, such as diglycidyl butanediol ether, ethanediol diglycidyl ether, or butanediol diglycidyl ether (available from Aldrich Chemical, St. Louis, MO); erythritol anhydride; the polyfunctional epoxides sold under the trademark Denacol by Nagase Chemicals, Osaka, Japan; epichlorhydrin (Aldrich Chemical, St. Louis, MO); enzymatically-inducible epoxides available from Sigma Chemicals, St. Louis, MO; and photo-polymerizable epoxides (Pierce, Rockford, IL). In preferred embodiments, the epoxy compounds are Denacol epoxides which are polyfunctional polyglycerol polyglycidyl ethers. For example, Denacol EX512 has 4 epoxides per molecule and Denacol EX521 has 5 epoxides per molecule.

In a specific preferred embodiment, the polymer is contacted with the multifunctional cpoxide compound in the presence of a catalyst. Suitable catalysts include, but are not limited to, tertiary amines, guanidine, imidazole, boron trifluoride adducts, such as boron trifluoride-monoethylamine, bisphosphonates, trace metals *(e.g.*, Zn, Sn, Mg, Al), and ammonium complexes of the type PhNH₃ + AsF₆₋. In other embodiments, the reaction can be photoinitiated by UV light, for example, in the presence of an appropriate catalyst, which may be titanium tetrachloride and ferrocene, zirconocene chloride, carbon tetrabromides or iodoform.

In yet another method aspect of the invention, the surface modifying agent may be incorporated as part of the polymer matrix comprising the nanoparticle. In a specific illustrative embodiment of this aspect of the invention, nanoparticles having an incorporated surface modifying agent which is a bioadhesive, specifically cyanoacrylate, are formed by including a cyanoacrylate-containing polymer, such as isobutyl cyanoacrylate, in the organic phase. When the nanoparticles are formed by an in-solvent emulsification-evaporation technique (see Example 14), the cyanoacrylate becomes part of the polymer core. Other polymers which would impart a bioadhesive effect include hydrogels and Pluronics.

In yet another embodiment of this aspect of the invention, the polymer core is a novel epoxy-derivatized and activated polycaprolactone. Block copolymers having hydrophobic and hydrophilic segments are synthesized by multiple reactions between hydroxyl end groups and epoxide groups in an illustrative reaction scheme comprising at least the following steps:
(a) dissolving a first polymer-diol in an organic solvent;
(b) adding a multifunctional epoxide in excess to the dissolved first polymer-diol so that one of the epoxide groups of the multifunctional epoxide reacts with hydroxyl groups on the ends of the first polymer-diol to form an epoxide end-capped first polymer (block A);
(c) adding an excess of a second polymer-diol (block B) to the epoxide end-capped first polymer block A to form a hydroxy-terminated BAB-type triblock copolymer.

The multifunctional epoxide suitable for use in the practice of this aspect of the invention include butane and ethane di-glycidyl ethers, erythritol anhydride, polyfunctional polyglycerol polyglycidyl ethers, and epichlorhydrin.

In some embodiments, the first polymer-diol is hydrophobic, illustratively polycaprolactone, polylactides, polyglycolides, and polylactic-polyglycolic acid copolymer. The second polymer-diol, therefore, is hydrophilic. Illustrative hydrophilic polymer-diols include, but are not limited to polyethylene glycol, polaxomers, and poly(propylene oxide). In other embodiments, the first polymer diol is hydrophilic and the second polymer diol is hydrophobic.

Advantageously, the molecular weight of the first polymer-diol can be expanded by epoxide reaction prior to combination with the second polymer-diol in order to control the physical properties of the resulting multiblock polymer. Further, the method steps outlined above can be repeated to produce multiblock polymers of any desired chain length. In a preferred embodiment, hydroxy-terminated polymers can be further reacted with a multifunctional epoxide to form an epoxide end-capped polymer. Multiblock copolymers in accordance with the present invention have hydrophobic and hydrophilic segments connected by epoxy linkages and are hydroxy-terminated or epoxide-terminated with a molecular weight between about 6,000 to 100,000 as measured by gel permeation chromatography and intrinsic viscosity.

An epoxide-terminated multiblock polymer can then be reacted with bioactive agent(s) having at least one functional group thereon which reacts with epoxide groups, such as amino, anhydrides, carboxyl, hydroxyl, phenol, or sulfhydryl. Of course, hydroxy-terminated polymers can react with bioactive agents either through the terminal hydroxy groups, or through the polyfunctional epoxide groups present in the polymer chain.

In order to use the nanoparticles in a practical embodiment, they may be reconstituted into a suspension with distilled water or normal saline at physiological pH and osmolarity. Other suitable suspending media include triglycerides, physiologic buffers, serum or other serum/plasma protein constituents, or tissue culture media with or without serum. Of course, excipients and additives of the type well known in the art for use in conjunction with pharmaceutical compositions may be added. Such excipients specifically include complexing agents and permeation enhancers, such as cyclodextrans, and osmolarity adjusting agents such as mannitol, sorbitol, and ficoll.

In an alternative embodiment, nanoparticles may be provided in an injectable suspending medium which gels after application to the region of injection. For example, the suspending medium may be a poloxamer, such as those sold under the trademark Pluronic by BASF, or collagen (Type I, Type II or procollagen) which are liquid at 4° C, but solidify at 37° C. Other exemplary suspending media for this embodiment, include hydrogels, such as prepolymeric acrylamides which may be catalyzed to form a water-containing gel, cyanoacrylates, and fibrin glue (a fibrinogen solution which turns to fibrin after it is injected; commercially available from multiple sources, including Ethicon, Somerville, NJ).

Typically, the nanoparticles are present in the injectable suspension at a concentration ranging from 0.1 mg nanoparticles per ml suspending fluid to 100 mg nanoparticles per ml suspending fluid. For the embodiments containing U86, a hydrophobic antiproliferative agent, for example, 15 mg nanoparticles per ml is a preferred upper limit since a higher amount causes arterial damage. The dosage of bioactive agent carried by the nanoparticles in suspension, of course, depends on the amount incorporated in the process. A person of ordinary skill in the art would be able to ascertain the dosage for efficacy and the requisite amount of nanoparticle-containing suspension required to administer the required dosage. It is to be understood that the nanoparticles may be adapted for administration by other routes, such as orally or to the mucous membrane, or may be administered intramuscularly or subcutaneously.

Nanoparticles made in accordance with the principles of the invention biodegrade in periods of time ranging are 30 days or less to 6 months or more. Based on prior experience with PCL in sustained release dosage forms, it is anticipated that embodiments where the biodegradable polymer is PCL can provide sustained release of bioactive agent for up to 3 years.

### Brief Description of the Drawing

Comprehension of the invention is facilitated by reading the following detailed description, in conjunction with the annexed drawing, in which:
Fig. 1 is a graphical representation of the *in vitro* release of a hydrophobic bioactive agent, U86, from nanoparticles made in accordance with the present invention which have been subjected to sterilizing gamma radiation;
Fig. 2 which is a graphical representation of the effect of surface modification and suspension media on the uptake of U86-containing nanoparticles expressed a µg nanoparticles per 10 mg artery specimen in the *ex vivo* canine model;
Fig. 3 is a plot of neointimal/media area ratios (NI/M) plotted against the total injury index as a measure of vascular as induced in porcine arteries by an overinflated catheter balloon following administration of U86-containing nanoparticles of the present invention;
Fig. 4 is a graphic representation of the inhibition of restenosis, expressed as the NI/M ratio, following the local administration of dexamethasone-containing PLGA nanoparticles after triple angioplasty-induced injury in rats;
Fig. 5 is a schematic representation of a synthetic procedure for coupling an epoxide compound to an hydroxyl end-group of polymeric nanoparticles, specifically PLGA nanoparticles, and subsequent coupling of the resulting epoxide-terminated polymer with heparin;
Fig. 6 which is a graphical representation of the *in vitro* release of heparin from nanoparticles of the type shown as compound 25 in Fig. 5, as measured by radioactivity, expressed as a percent of bound heparin released over time (days);
Fig. 7 is an illustrative reaction scheme for the production of block copolymers having a hydrophobic PCL segment and a hydrophilic segment, which may be a hydrophilic polyether;
Figs. 8-11 show the spectra of starting materials for making the block copolymers in accordance with the illustrative reaction scheme of Fig. 7, specifically a polycaprolactone-diol (PCL-diol), the hydrophilic polaxomer Pluronic F68 (F68), polyethylene glycol (PEG E4500), and a multifunctional epoxide (Denacol EX252), respectively;
Fig. 12 is the spectrum of an hydroxy-terminated block copolymer having hydrophobic (PCL) and hydrophilic (F68) segments are linked by an epoxide (EX252);
Fig. 13 is the spectrum of an hydroxy-terminated block copolymer having hydrophobic (PCL) and hydrophilic (PEG) segments linked by an epoxide (EX252) with a 75:25 molar ratio of PCL to PEG;
Fig. 14 is the spectrum of an hydroxy-terminated block copolymer of the type shown in Fig. 13, but having a 60:40 molar ratio of PCL to PEG, and therefore, a greater proportion of hydrophilic polymer than the copolymer shown in Fig. 13;
Fig. 15 is a graphic representation of the percent of albumin (BSA) remaining in hydroxy-terminated PCL/F68/PCL nanoparticles made in accordance with Example 18 as function of time, in days, as compared to the amount of albumin remaining in a physical mixture, or dispersion, of BSA with the PCL/F68/PCL nanoparticles;
Fig. 16A through 16C are graphical representations of the stability of the heparin coupled to nanoparticles comprising the triblock copolymers of Table 15 expressed as % bound heparin remaining in the nanoparticles over time in days, specifically the triblock copolymers are an expanded PCL homopolymer (PCL/PCL/PCL), and hydroxy-terminated ABA triblock copolymers of polycaprolactone and Pluronic F68 (PCL/F68/PCL) or polyethylene glycol (PCL/PEG/PCL) as compared to intimate physical mixtures of heparin and the triblock copolymers;
Fig. 17 is a graphical representation of the *in vitro* release of the hydrophobic bioactive agent U86 from heparin-coupled nanoparticles of triblock copolymers as in Fig. 16, expressed as the percent of U86 released over time in days, as compared to the *in vitro* release of U86 from PLGA heparin-coupled nanoparticles;
Fig. 18 is a graphical representation of the *in vitro* release of dexamethasone, as a percent released over time in days, for nanoparticles of triblock copolymers as in Fig. 16 (Table 17);
Fig. 19 which is a graphical representation of the *in vitro* release of albumin (BSA) released from ABA triblock copolymer films having 15% BSA loading and a thickness of 150 µm expressed as the % BSA released over time in days;
Fig. 20 is a graphic representation of the *in vitro* release of cytochalasin-B from PLGA nanoparticles prepared in accordance with a method of the invention expressed as the percent of total cytochalasin-B released over time (in days); and
Fig. 21 is a graphical representation of the immune response resulting from subcutaneous immunization of rates with Tetanus Toxoid loaded nanoparticles, as measured by IgG (µg/ml), at 21 days and 30 days post-immunization, as compared to the immune response in rats following subcutaneous immunization with conventional Alum-Tetanus Toxoid conjugate; and
Fig. 22 is a plot of luciferase activity (CPM/µg protein) in COS cells transfected with specimens of DNA (luciferase)-containing PLGA nanoparticles made in accordance with the present invention.

### Detailed Description

In order to form nanoparticles in accordance with the present invention, it is important to reduce the interfacial energy at the liquid-liquid interface during processing. The reduction in interfacial energy results in formation of a spontaneous and stable emulsion. Reduction in interfacial energy can be attained by addition of appropriate emulsifiers to either one, or both, of the aqueous or organic phases.

In addition to the use of appropriate surfactant(s), optimization of different formulation factors, such as the relative volume of the two liquid phases (1:9 is optimal as the internal to external phase ratio, however, ratios ranging to about 4.5:5.5 are suitable), and the concentration of the polymer and bioactive agent in each, contributes to the overall particle size. The input of external energy during the emulsification procedure, such as by an homogenizer or sonicator, results in the formation of extremely small droplets of one liquid in the other liquid phase. Evaporation of the organic solvent solidifies the liquid droplets into small solid particles, termed the "polymeric core" in this application. Bioactive agent dissolved in either an aqueous or organic phase becomes part of the polymeric core matrix.

The following are specific examples of nanoparticles and methods of making same in accordance with the invention:

### I. Methods of Making Nanoparticles

### A. Method for Incorporating Hydrophobic Bioactive Agents

### Example 1:

In a typical procedure for incorporating a hydrophobic bioactive agent into nanoparticles in accordance with the above-described method aspect, 200 mg of polymer and 60 mg drug are dissolved in 10 ml of an organic solvent, such as distilled methylene chloride. The organic drug/polymer solution is added drop-wise over a period of one minute (with sonication at 55 Watts of energy output from a probe-type sonicator) to 40 ml 2% w/v aqueous PVA solution (average molecular weight 30,000 to 70,000) that had been saturated with methylene chloride and filtered. The PVA solution was saturated with methylene chloride because methylene chloride, which is partially soluble in water, would cause the polymer to separate from the drug/polymer solution immediately upon its addition into the aqueous phase because of diffusion of methylene chloride into water. Avoiding premature precipitation aids the creation of an emulsion having a relatively uniform particle size distribution. Filtration of the PVA solution prior to use is helpful since commercially available PVA (Sigma, St. Louis, MO) contains a small fraction of high molecular weight PVA molecules (>70,000) which are not soluble in water. Sonication is continued for a total of 10 minutes at 55 Watts. This results in the formation of an oil-in-water emulsion. After 18 hours of stirring at room temperature over a magnetic stir plate to evaporate the solvent, nanoparticles are recovered by centrifugation at 145,000 g. The recovered nanoparticles are washed three times with distilled water, resuspended again by sonication in 10 ml distilled water over an ice bath, and lyophilized at - 60°under 100 millitorr vacuum for 48 hours. The lyophilized nanoparticles are dried in a dessicator for another 48 hours and stored at 4° C in a dessicator until use.

### Example 2:

PLGA-lipid nanoparticles were made by dissolving 130 mg PLGA in 10 ml methylene chloride. A lipid solution (4 ml; available in chloroform at a concentration of 10 mg lipid per/ml from Sigma, St. Louis, MO) was added to the PLGA solution to form an organic phase. In this embodiment, the lipid is L-α-dioleoylphosphatidylethanolamine. A hydrophobic drug, which in this example is U86 (60 mg), is dissolved in the organic phase. The organic phase was emulsified by sonication into 40 ml 2.5% w/v aqueous PVA to form an oil-in-water emulsion. The organic solvent was evaporated by stirring the emulsion in an open container for 16 hours. Nanoparticles were recovered by ultracentrifugation at 140,000 g, washed three times with water, and lyophilized. The PLGA-lipid nanoparticles were recovered in about 60% yield, with U86 loading of 26%. The mean particle diameter was 100 ± 39 nm.

In this example, the second bioactive agent which is a lipid, functions both as a partitioning agent and a surface modifying agent.

### Example 3:

The hydrophobic drug, dexamethasone is formulated into PLGA nanoparticles by the following illustrative procedure.

600 mg PLGA is dissolved in 24 ml methylene chloride. Dexamethasone (200 mg) is dissolved separately in a combination of 4 ml acetone and 2 ml ethanol. The dexamethasone solution is added to the polymer solution to form an organic phase. The organic phase is emulsified into 120 ml 2% PVA solution to form an oil-in-water emulsion. Organic solvents are evaporated at room temperatures with stirring over a stir plate for 18 hours. Nanoparticles, thus formed, are recovered by ultracentrifugation, washed three times with water, resuspended and lyophilized. This procedure forms nanoparticles in 60% yield, with a drug loading of 15.5% w/w, and average particle size of about 160 nm.

### Example 3A:

PLGA nanoparticles containing ciprostene, a hydrophobic prostaglandin antagonist (Upjohn, Kalamazoo, MI), were made in accordance with the techniques of Example 1 relating to hydrophobic agents, but using a co-solvent system comprising a polar and semipolar organic solvent.

In a typical procedure, 300 mg PLGA is dissolved in a mixture of 7 ml methylene chloride and 3 ml acetone. Ciprostene (70 mg) is dissolved separately in 3 ml dimethyl acetamide and mixed with the polymer solution to form an organic phase. The organic phase is emulsified in 30 ml of 2% PVA solution, adjusted to pH 4.5 with monobasic sodium phosphate, using a probe sonicator set at 65 Watts of energy output for 10 minutes to form an oil-in-water emulsion. The emulsion is stirred for 18 hours. Nanoparticles are recovered by ultracentrifugation. washed three times with water, resuspended and lyophilized. The pH was adjusted to favor partitioning of the drug into the organic phase to improve entrapment efficiency.

The ciprostene-loaded nanoparticles had a small mean particle size. At 21.6% w/w drug loading, the mean particle diameter was 97.4 ± 38 nm. Another batch of nanoparticles, having 15.5% drug loading, had a mean particle diameter of 82.8 ± 54 nm. When subjected to *in vitro* release studies (phosphate buffer at pH 7.4, 37° C), 100% of the incorporated drugs were released by 65 days and 40 days, respectively.

Using standard *in vitro* platelet aggregometry techniques, a dose-response curve was first developed with free ciprostene to determined its inhibitory potency against standard ADP-induced platelet aggregation. The IC₅₀ for the drug in this experiment was roughly 0.28 µg/ml. Concentrations of PLGA nanoparticles ranging from 0.3 to 30 µg/ml (actual ciprostene concentration of 0.06 to 6 µg/ml due to 20% drug loading) were added to platelet rich plasma samples heated to 37° C. The platelet inhibitory effects were monitored after 1 minute. The IC₅₀ for the polymer-incorporated ciprostene was 0.59 µg/ml. Non-drug containing PLGA nanoparticles, as controls, had no obvious effects on the aggregation profile of pig platelets to the agonist ADP. A comparison of the IC₅₀s of the free ciprostene and the nanoparticle-incorporated ciprostene suggests that roughly 39% of the polymer-loaded drug becomes available to the platelets in the *in vitro* system.

### B. Method for Incorporating Hydrophilic Bioactive Agents

### Example 4:

An illustrative example of incorporating a hydrophilic bioactive agent, ibutilide, into PLGA nanoparticles is given below.

In a typical example, a fatty acid solution is formed by dissolving 93 mg of palmitic acid sodium salt in a co-solvent system consisting of 2.25 ml dimethyl acetamide and 3 ml methylene chloride. The fatty acid solution is warmed over a water bath (temperature <40° C) until a clear solution is formed. PLGA (275 mg) and ibutilide (25 mg; molar ratio of fatty acid to ibutilide is 5:1) are added to the fatty acid solution and are stirred until the solution forms a clear gel. While still warm, the clear gel-like solution is added to 20 ml 2% PVA solution prepared in borate buffer saturated with methylene chloride (50 mM, pH 9.0, prepared by adjusting the pH of boric acid with 5 N HCl). The combination is sonicated at 65 Watts of energy for 10 minutes to form an oil-in-water emulsion. The emulsion is stirred over a magnetic stir plate for 18 hours. Nanoparticles are recovered by ultracentrifugation at 145,000 g, washed three times with water, resuspended in water and lyophilized for 48 hours. In this particular embodiment, the nanoparticles were produced in 60% yield with an average particle diameter of 144 nm and 7.4% w/w drug loading (Sample 22 on Table 3).

A partitioning agent, which in this case is an anionic fatty acid (palmitic acid) forms a complex with the cationic drug, ibutilide, due to ionic interaction. The complex thus formed is hydrophobic and, therefore, partitions into the organic phase. Since the complex is also ionic, it will separate again, during bioerosion, into drug and fatty acid to release drug from the nanoparticles,

The ratio of semipolar to nonpolar solvents in the co-solvent system depends upon the solubility of the drug and the polymer. The proportion must be adjusted so that the co-solvent system dissolves both drug and polymer. A person of ordinary skill in the art would be able to select the right combination of solvents on the basis of their polarity for any given drug/polymer combination.

### C. Method for Incorporating Protein/Peptide Hydrophilic Bioactive Agents

### Example 5:

A multiple water-in-oil-in-water emulsion technique used to incorporate an exemplary protein, bovine serum albumin (BSA), into nanoparticles.

In a typical preparation BSA (50 mg) is dissolved in 500 µl water. A polymer solution is prepared consisting of PLGA (150 mg) dissolved in 5 ml methylene chloride. The BSA solution is emulsified into the polymer solution with 65 Watts of energy output from a probe sonicator to form a primary water-in-oil emulsion. The primary emulsion is further emulsified into a PVA solution (2.5% w/w, 40 ml, 30,000 to 70,000 M. Wt.) by sonication at 65 Watts for 10 minutes to form a multiple water-in-oil-in-water emulsion. The multiple emulsion is stirred over a stir plate for 18 hours to remove organic solvent. Nanoparticles are recovered by ultracentrifugation, washed three times with water, resuspended, and lyophilized. The yield of BSA-containing nanoparticles made by this technique was 57%. The average particle diameter was 160 nm with 18% w/w drug loading.

### D. Method for Making Ultrasmall Nanoparticles

### Example 6:

In another preferred specific illustrative embodiment, ultrasmall nanoparticles are formed in accordance with the principles of the invention by a technique using a co-solvent system which has been developed to further reduce the interfacial energy so that ultrasmall emulsion droplets are formed. Ultrasmall nanoparticles are defined herein as having a mean diameter of between about 10 nm to 50 nm, and more preferably 20 nm to 35 nm. In addition to the co-solvent system, increasing the amount of energy applied with the sonicator probe from 35 to 65 Watts contributes to the smaller size of the particles. Also, the use of certain emulsifying agents, particularly DMAB, contribute to the production of ultrasmall nanoparticles. Other cationic detergents, notably cetyl trimethyl ammonium bromide (CTAB), hexyldecyl trimethyl ammonium chloride (CTAC), have been found to produce similar results.

In a typical example, the co-solvent system is a combination of a nonpolar organic solvent, such as methylene chloride, chloroform, or ethyl acetate, and a semi-polar organic solvent, such as acetone, dimethyl sulfoxide (DMSO), or dimethyl acetamide.

Polylactic polyglycolic acid copolymer (100 mg) and bioactive agent are dissolved in 5 ml of an organic co-solvent system of dichloromethane and dimethylacetamide (2:3 by volume) to comprise an organic phase. The organic phase is emulsified in an aqueous phase (20 ml) containing 2.0% w/v PVA (9,000-10,000 molecular weight, 80% hydrolyzed) by sonication using a probe sonicator with an energy output of 65 Watts for 10 minutes in an ice bath. The emulsion is stirred for 18 hours at room temperature. Then, the emulsion is dialyzed for 18 hours using dialysis tubing of molecular weight cut-off 12,000 to 14,000. The particles are then lyophilized for 48 hours and desiccated.

While Example 6 is directed to making ultrasmall nanoparticles incorporating a hydrophobic agent, the technique is applicable to hydrophilic agents. A multiple emulsion technique (water-in-oil-in-water), similar to Example 5, may be used wherein the hydrophilic bioactive agent is dissolved in the aqueous phase.

### II. Surface Modification Techniques

Table 1 below is partial listing of surface modifying agents, their intended results, and suggested methods of incorporating the surface modifying agent to the nanoparticles. This list is intended to be illustrative, and in no way should be construed as limiting the types of surface modifying agents contemplated in the practice of the invention. A person of ordinary skill in the art would be able to select appropriate modifying agents for a given purpose.

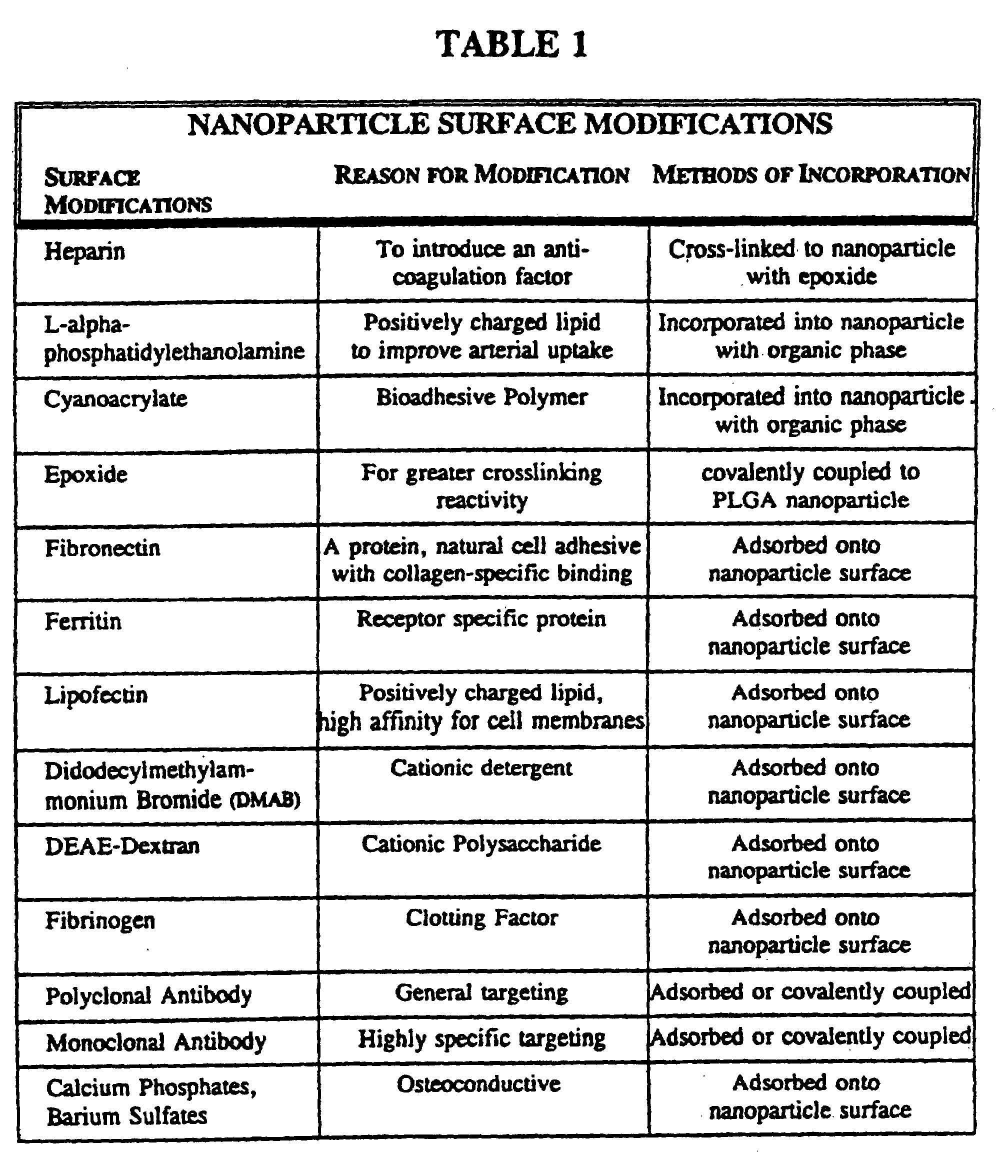

As is evident from Table 1, the invention herein contemplates multiple methods of modifying the surface.

### A. Adsorption of Surface Modifying Agent

In one technique, the surface of pre-formed nanoparticles is modified by providing a coating of a surface modifying agent which is physically adhered or adsorbed.

In a typical method for providing an adsorbed coating, the surface modifying agent is dissolved in a solvent to form a solution and the pre-formed nanoparticles are suspended in the solution. The suspension is then freeze-dried to form a coating which is physically adhered, but not chemically bonded. More particularly, nanoparticles are suspended in water (usually at a concentration of 10 mg/ml) by sonication. Then, a measured amount of surface modifying agent, either in solution or in dry form is added to the suspension. If the surface modifying agent is provided in solution, the solvent should not dissolve the nanoparticles. Suitable solvents include polar solvents, such as water, aqueous buffer, saline, ethanol-water, glycerol-water, or combinations thereof. In a typical case, the measured amount is 5% w/w of surface modifying agent per mass of nanoparticles. However, it is contemplated that amounts of surface modifying agent may range from 0.5% to 15%. The surface modifying agent-containing suspension of nanoparticles is lyophilized in a lyophilizer at 0° C to -55° C in a vacuum of 500 millitorr or less for at least 24-48 hours.

It should be noted that the concentration range for the bound surface modifier is given for purposes of illustration only, and can be varied by those of skill in the art because it is greatly in excess of the therapeutically effective amount. The ability to irreversibly bind a high concentration of surface modifier to the biomaterial, thereby targeting the bioactive agent to the site of use and/or conferring advantageous properties to the biomaterial, is a significant advantage of this invention over the prior art.

### Example 7:

In a typical procedure, the surface modifying agent DMAB is dissolved in 10 ml water by gentle vortexing. Nanoparticles (95 mg, U-86 loaded PLGA nanoparticles made in accordance with Example 8) are suspended in the aqueous DMAB solution by sonication for 30-60 seconds over an ice bath. The surface-modified nanoparticle suspension is then lyophilized as usual.

### B. Incorporation of the Surface Modifying Agent Into the Polymer Matrix

If the surface modifying agent is water insoluble, it preferably is incorporated info the organic phase of the emulsion while formulating the nanoparticles.

### Example 8:

A method of in-solvent emulsification-evaporation is used to incorporate hydrophobic bioactive agents into nanoparticles. In the specific illustrative embodiments herein, U86 or the adrenocortocoid, dexamethasone, are model hydrophobic bioactive agents. PLGA and drug are dissolved in 5 ml methylene chloride. The PLGA-drug mixture is emulsified in 40 ml 2.5% w/v aqueous PVA (M. Wt. 30,000-70,000) with sonication using a microtip probe sonicator (Heat Systems, Model XL 2020, Misonix Inc., Farmingdale, NY) at an energy output of 65 Watts, over an ice bath for 10 minutes. The emulsion is stirred for 16 hours at room temperature to permit the methylene chloride to evaporate. The nanoparticles are recovered by ultracentrifugation at 141,000 g. The recovered nanoparticles are washed three times with water and lyophilized for 48 hours. The nanoparticles are stored in desiccated form. The U86-containing nanoparticles were obtained in 80% yield, contained 15.5% w/w drug, and had an average particle diameter of 110 nm. The dexamethasone-containing nanoparticles were obtained in 80% yield, contained 16.05% w/w drug, and had an average particle diameter of 108 nm.

Additional formulations of U86-containing nanoparticles, and surface modified nanoparticles, made in accordance with Example 8, are given in Table 2 below. Table 2 also gives data relating to yield, percent drug-loading, and size in nm. All of the surface modifying agents shown on Table 2 were incorporated as part of the polymer matrix of the PLGA nanoparticles, *i.e*., were added into the polymer solution during formulation in accordance with the procedures of this example. The surface modifiers are palmitic acid (PA), beeswax (Wax), both hydrophobic materials, isobutyl cyanocrylate (IBCNA), a bioadhesive, and dioleoylphosphatidylethanolamine (DOPE), a phospholipid to enhance uptake of the nanoparticles. The numbers which appear in conjunction with the identification of the surface modifying agents are the weight, in mg, of surface modifying agent used in the formulation, *e.g.*, sample 11 contained 108 mg of IBCNA.

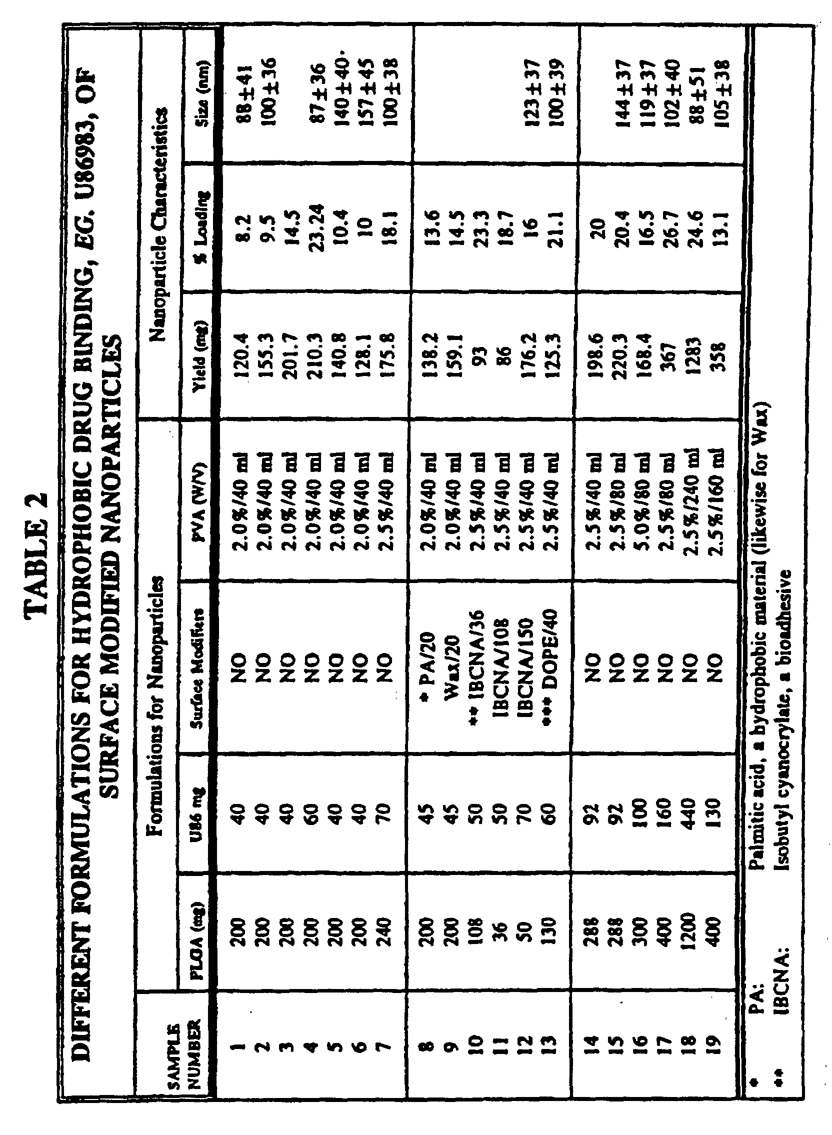

Hydrophilic-drug loaded nanoparticles were prepared in accordance with the method of Example 4. Table 3 gives several formulations for the ibutilide-containing PLGA nanoparticles, as well as yield, percent drug-loading, and size in nm. The additive set forth in Table 3, palmitic acid, functions as a partitioning agent as described in Example 4.

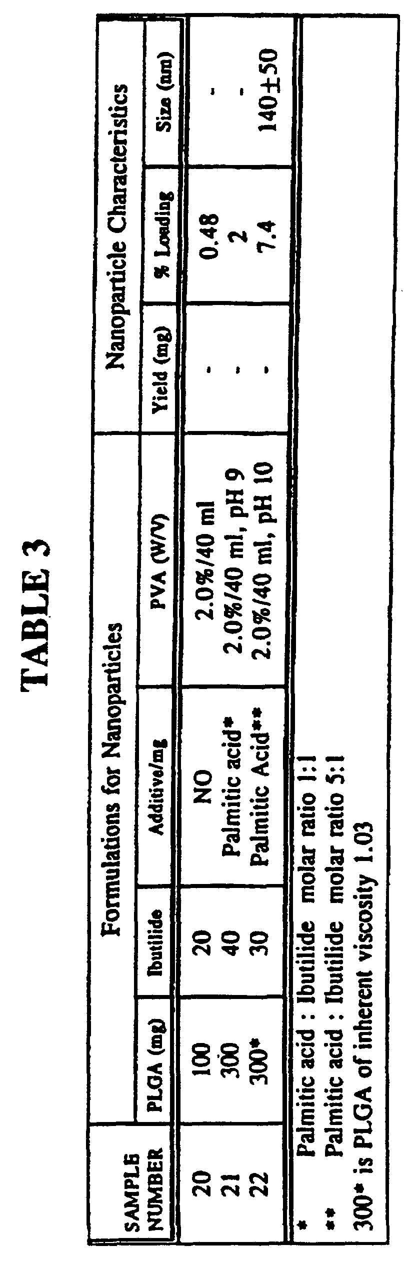

### Example 9:

In a typical procedure to incorporate heparin in PLGA nanoparticles, 30 mg heparin is dissolved in 500 µl water and the solution is cooled to 4° C. Pluronic F-127 (10 mg) is added to the heparin solution as a viscosity enhancing agent to favor entrapment of heparin in the nanoparticle matrix core. The mixture is emulsified with sonication (55 Watts energy output for 10 minutes over an ice bath) with a solution of PLGA (150 mg) in methylene chloride (5 ml) to form a water-in-oil emulsion. The water-in-oil emulsion is further emulsified into 20 ml 2.5% aqueous PVA solution by sonification for 10 minutes at 55 Watts. The result is a water-in-oil-in-water multiple emulsion. The multiple emulsion is stirred over a magnetic stir plate for 18 hours to evaporate the organic solvents. Nanoparticles may be recovered by ultracentrifugation or use of an Amicon® (Amicon Inc., Beverly, MA) filtration system. The recovered nanoparticles are washed free of un-entrapped heparin and lyophilized. The yield for the instant method is 45% with an average partice size of 90 nm and 4.8% w/w drug load. Evaluation of the heparin-containing nanoparticles by standard APTT testing for andcoagulation activity demonstrated that the heparin-containing nanoparticles had a coagulation time of >200 seconds as compared to 13.7 second for control nanoparticles which were PLGA nanoparticles without heparin.

### Example 10

Nanoparticles containing tetanus toxoid were prepared in an identical procedure to Example 5 except that the tetanus toxoid (TT) solution (500 µl) contained 11 mg TT and 1 mg surfactant, Pluronic F-127. The yield of TT-containing nanoparticles was 60% with an average particle size of 241 nm and drug loading of 4% w/w (sample 28 on Table 4).

Additional formulations of BSA and/or TT-containing nanoparticles, with a Pluronic F-127 additive are set forth in Table 4. In this case, Pluronic F-127 performs a dual function. It acts as a viscosity enhancing agent to favor partitioning and contributes to the formation of a stable emulsion. In the case of vaccines, such as in the TT-containing nanoparticles, Pluronic F-127 also acts as an adjuvant to enhance immune response.

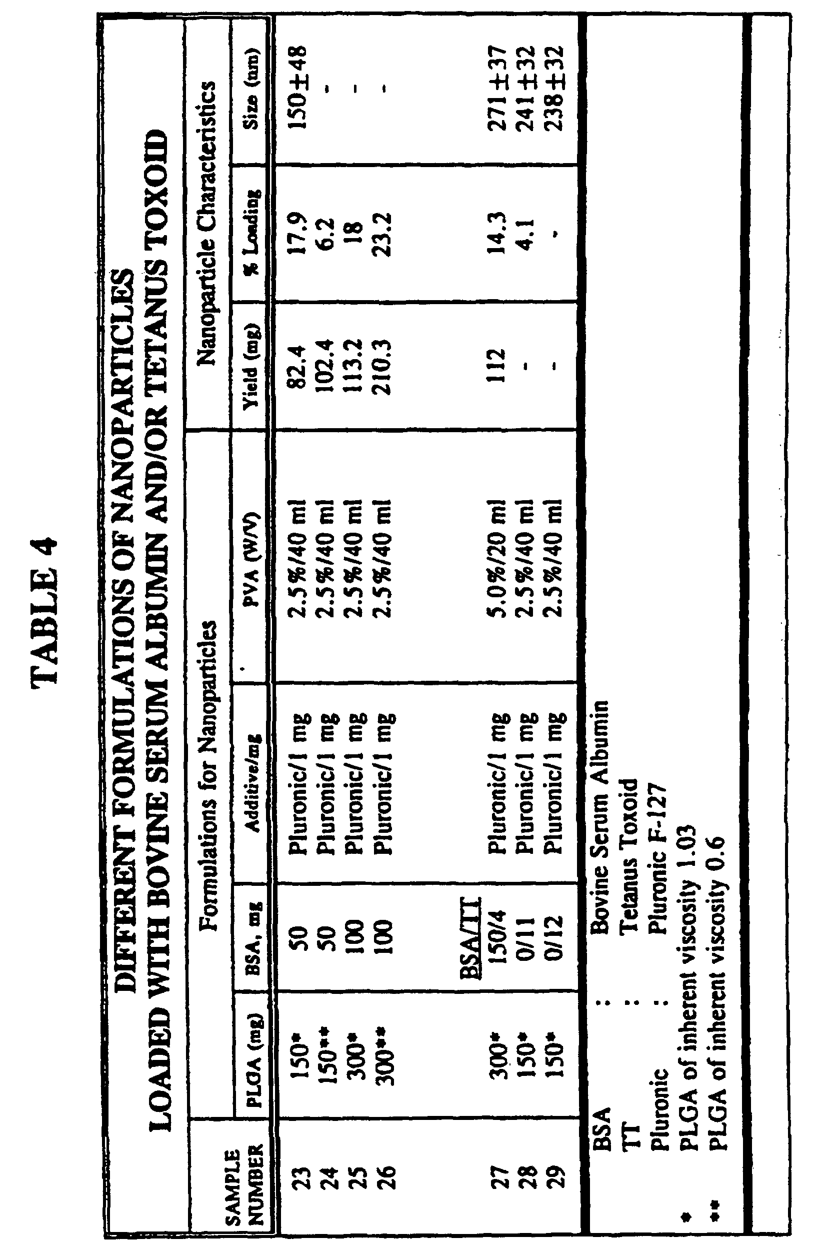

### Experimental Results:

### in vitro Release Studies

*in vitro* Release studies were conducted on the nanoparticles made in accordance with Examples 8, 4, and 5 using a double diffusion chamber wherein the two compartments of the diffusion chamber are separated by a Millipore (100 nm pore size; Millipore Corp., Bedford, MA) membrane. The donor side of the chamber was filled with a nanoparticle suspension (5 mg nanoparticles per ml physiological phosphate buffer (pH 7.4, 0.154 mM). The receiver side was filled with the same buffer. The diffusion cells were placed on a shaker (110 rpm) in a 37° C room. Periodically, a sample of buffer was withdrawn from the receiver side and replaced with an equal quantity of fresh buffer. The drug levels in the receiver buffer were quantitated by HPLC or other analytical methods. The data was used to calculate the percent drug released from the nanoparticles over time.

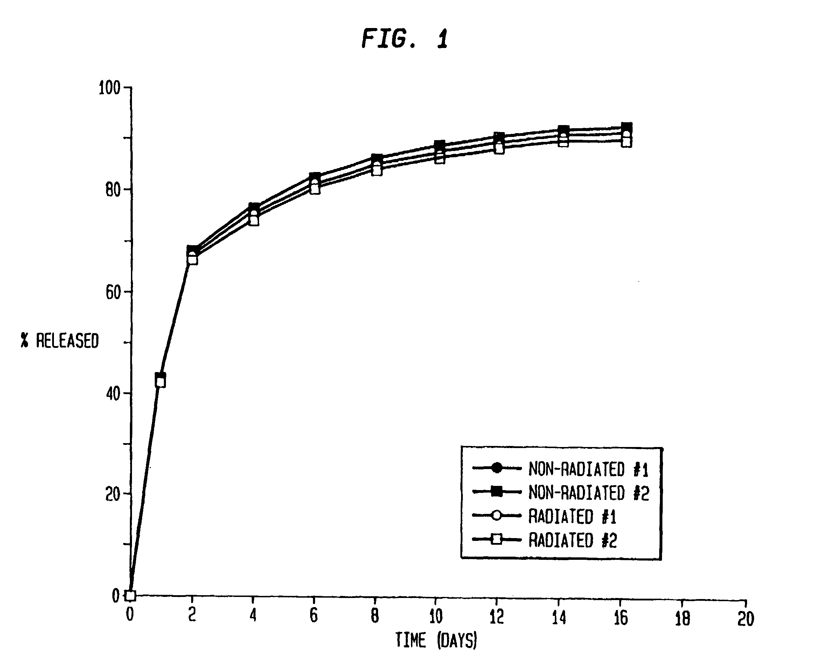

The *in vitro* release studies of nanoparticles containing U86 showed an initial burst effect, followed by release at an exponentially decreasing rate. Similar release rates were observed for hydrophilic and/or protein-containing nanoparticles. Gamma sterilization (2.5 Mrad) did not affect the *in vitro* release characteristics of U86 from the nanoparticles as shown in Fig. 1 which is a graphical representation of the *in vitro* release of a hydrophobic bioactive agent, U86, from nanoparticles made in accordance with the present invention which have been subjected to sterilizing gamma radiation.

Size distribution may be measured by a laser defractometer, such as the Nicomp 370 Dynamic Laser Light Scattering Autocorrelator (Nicomp Particle Sizing Systems, Santa Barbara, CA) or similar equipmcnt. A suspension of nanoparticles (1 mg/ml) in water of normal saline is prepared by sonication just prior to analysis. Nanoparticles prepared in accordance with the invention were typically less than 200 nm, and generally in the range of 80-160 nm. The particle size distribution analysis of the nanoparticles revealed a uniform and narrow size distribution.

Scanning electron micrographs were taken of nanoparticles which had been mounted and sputtered with gold. The results demonstrated that the particles are of uniform dimensions a with smooth surfaces and the absence of any free drug granules.

### ex vivo Arterial Uptake Studies in a Dog Model

Nanoparticles made in accordance with the principles of the invention were evaluated, *ex vivo* for arterial uptake as a result of surface modification. A dog carotid artery was removed, flushed with normal saline to remove blood, and held taut (2.7 cm length) by tying the ends to two glass capillary tubes separated by a distance of 2.1 cm on a glass rod. The bottom end of the arterial segment was temporarily ligated so that a nanoparticle suspension (2.5 to 10 mg/ml) introduced into the top end under 0.5 psi pressure was retained in the artery segment. After 30 seconds, the bottom end of the artery was opened and a lactated-Ringers solution was passed through the artery segment from the top end for 30 minutes at a flow rate of 40 ml/hour. A 2 cm segment of the artery was cut from the device, homogenized, extracted, and quantitated for drug levels by HPLC. Knowing the extraction efficiency and drug loading of the particles, the amount of nanoparticles retained by the artery segment was calculated.

In a specific example, PLGA nanoparticles loaded with U86 were manufactured accordance with the method of Example 8. The unmodified embodiment (sample 15, Table 2), was used as a control for comparative purposes, *i.e*., to illustrate the greater degree of retention achieved with the various surface-modified particles. Surface-modified nanoparticles, as identified on Table 5, were prepared in accordance with the techniques set forth herein (sample 17 on Table 2).

Coatings of either 5% DMAB (samples 40-43) or 5% DEAE-Dextran (samples 44-46) were placed on the sample nanoparticles by the freeze-drying technique described hereinabove. The results of arterial retention of the surface-modified nanoparticles in the *ex vivo* dog model are shown on Table 5. Nanoparticles modified with 5% w/v didodecyldimethyl ammonium bromide (DMAB-5%) were the most effective, resulting in 11.4 times more retention of nanoparticles as compared to the unmodified nanoparticles (PLGA).

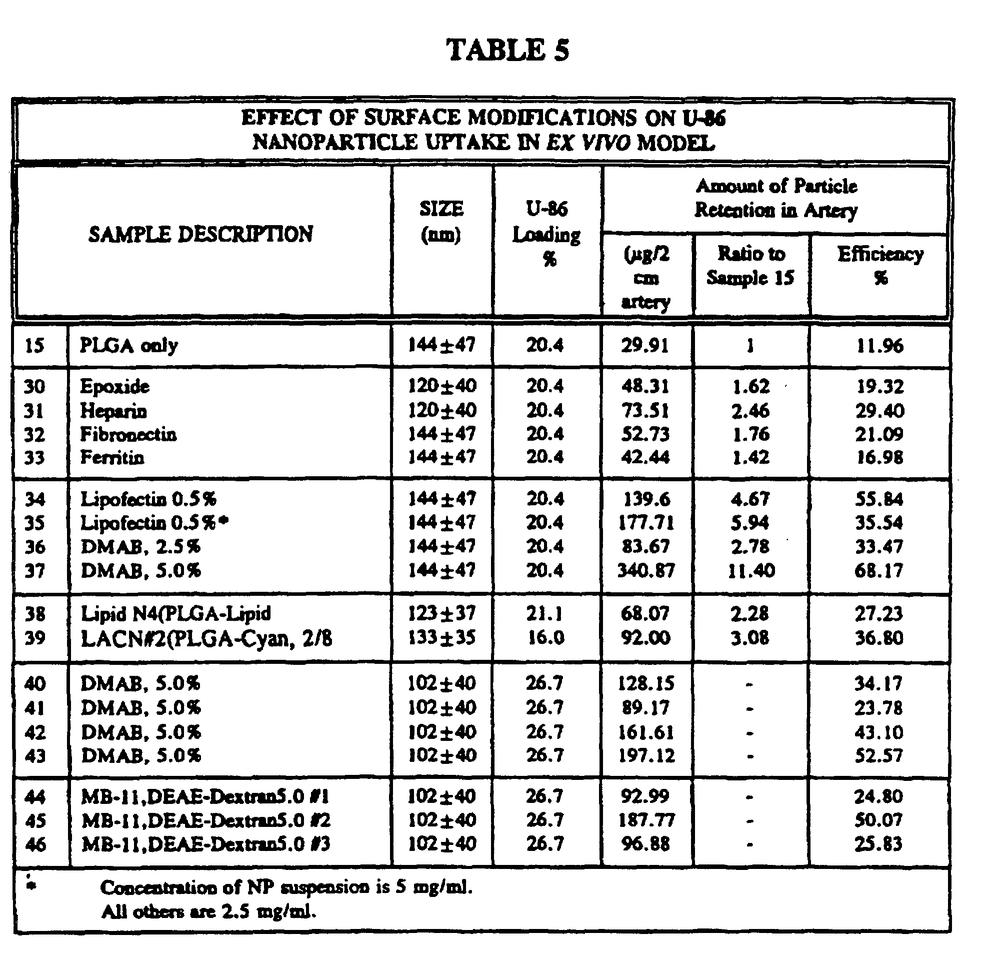

In addition to DMAB and DEAE-dextran, 5% fibrinogen was placed on PLGA nanoparticles by the freeze-drying technique. The PLGA nanoparticles had a mean particle diameter of 130 ± 35 nm and a 14.6% drug loading prior to the application of the fibrinogen. The particles were suspended in normal saline or a 1:1 mixture of serum and saline and injected into the *ex vivo* dog experiments, the mean ± SE uptake of nanoparticles in a 10 mg segment of artery was 38.03 ± 2.42 µg, 39.05 ± 3.33 µg, and 52.30 ± 4.0 µg, respectively, for 5% DMAB, 5% DEAE-dextran, and 5% fibrinogen.

To summarize the results, surface modification of nanoparticles with DMAB improves retention to tissue. DEAE-dextran modified nanoparticles have an increased viscosity in suspension. Fibrinogen-modified nanoparticles facilitate thrombus formation, thereby aggregating the spheres and significantly improving arterial uptake. A combination of DMAB and fibrinogen, for example, would cause initial adhesion, followed by thrombus formation, to secure the nanoparticles to the arterial wall for long-term effect.

In addition to surface modification, the concentration of nanoparticles in the infusion suspension affected the retention of nanoparticles to the arterial wall in the *ex vivo* canine model as shown in Table 6 for samples 31 and 34 of Table 5 suspended in normal saline at the listed concentrations.

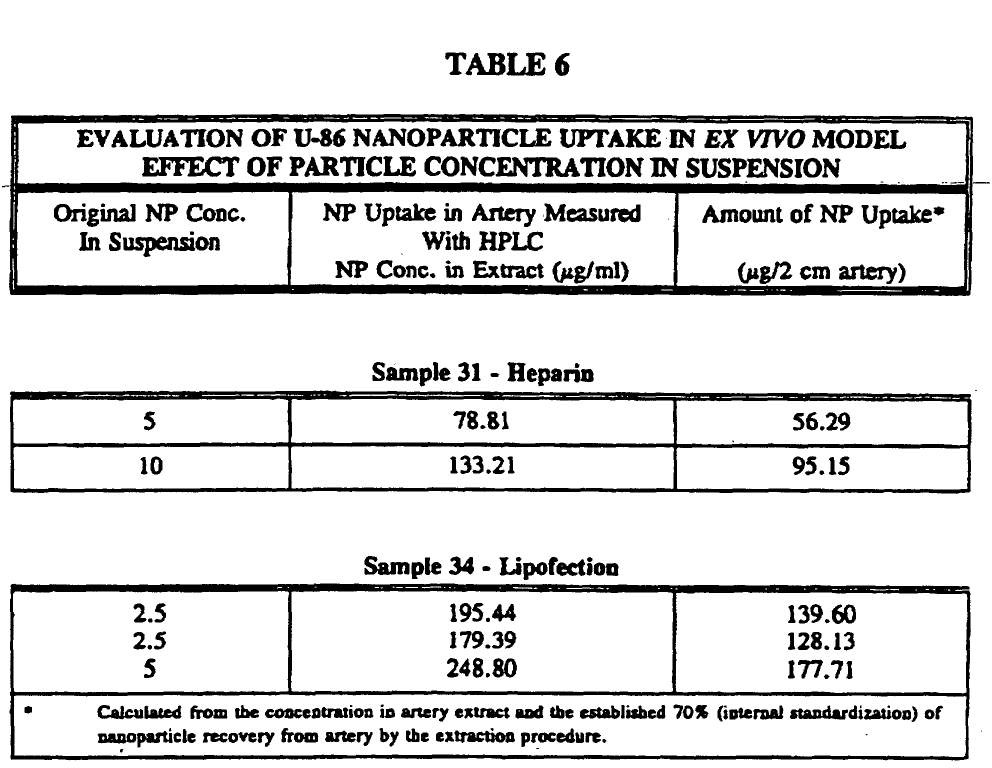

Table 6 shows that an increase in nanoparticle concentration in the suspension enhances the uptake of nanoparticles by the arterial wall.

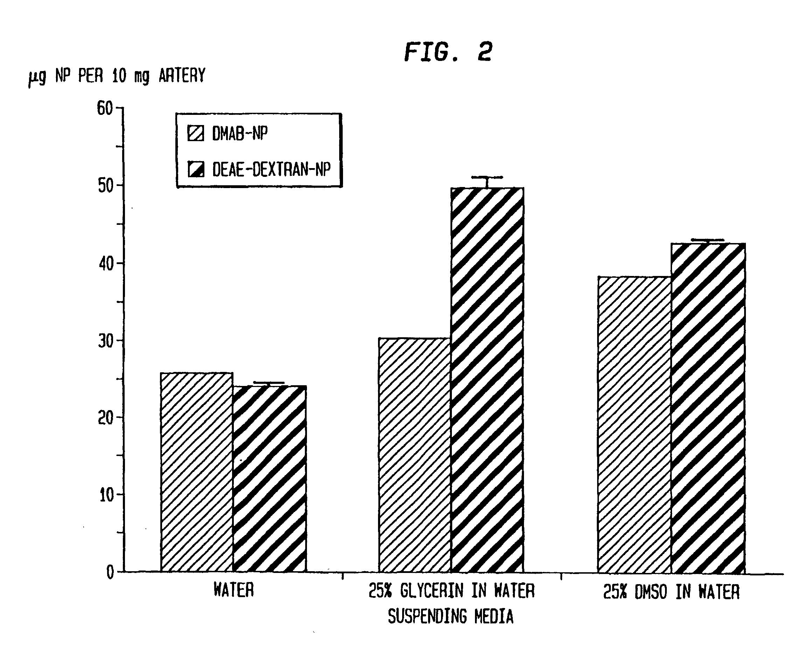

Various suspending media were investigated in the *ex vivo* canine model for their effect on nanoparticle retention. Nanoparticles (Sample 19 on Table 2) were surface modified with DMAB and DEAE-Dextran. Samples of the surface-modified particles were suspended in distilled water, 10% v/v aqueous DMSO, or 25% v/v aqueous glycerin. DMSO was used to enhance permeability of the arterial wall and glycerin was used to induce transient hypertonic shock at the site of administration to enhance drug delivery across the biologic membranes. The results are shown in Fig. 2 which is a graphical representation of the effect of surface modification and suspension media on the uptake of U86-containing nanoparticles expressed a µg nanoparticles per 10 mg artery specimen. As shown in Fig. 2, an osmotic shock, such as induced by a hypertonic solution (glycerin-water), or the inclusion of a tissue permeability enhancing agent (DMSO) in the suspending medium improves uptake of the nanoparticles by the arterial wall.

The entrapment efficiency for nanoparticles made in accordance with the methods at 8, 4, and 5 is about 70-80% for hydrophobic drugs, about 45% for hydrophilic drugs, and, 57-67% for proteins and vaccines. Typical drug-loading for the various types of nanoparticles are 4% to 28%. The effect of drug-loading on retention was studied with DMAB-modified nanoparticles and the results are shown on Table 7. Interestingly, higher drug loading resulted in lower retention. This phenomenon very likely reflects a critical change in the hydrophilicity/hydrophobicity characteristics of the nanoparticles which affects their ability to reside in the arterial wall. It is hypothesized that higher loadings of the hydrophobic drug U86 gives the particles less affinity with the highly hydrophilic arterial wall. However, reducing the loading of U86 allows a more favorable, or overall, hydrophilic reaction.

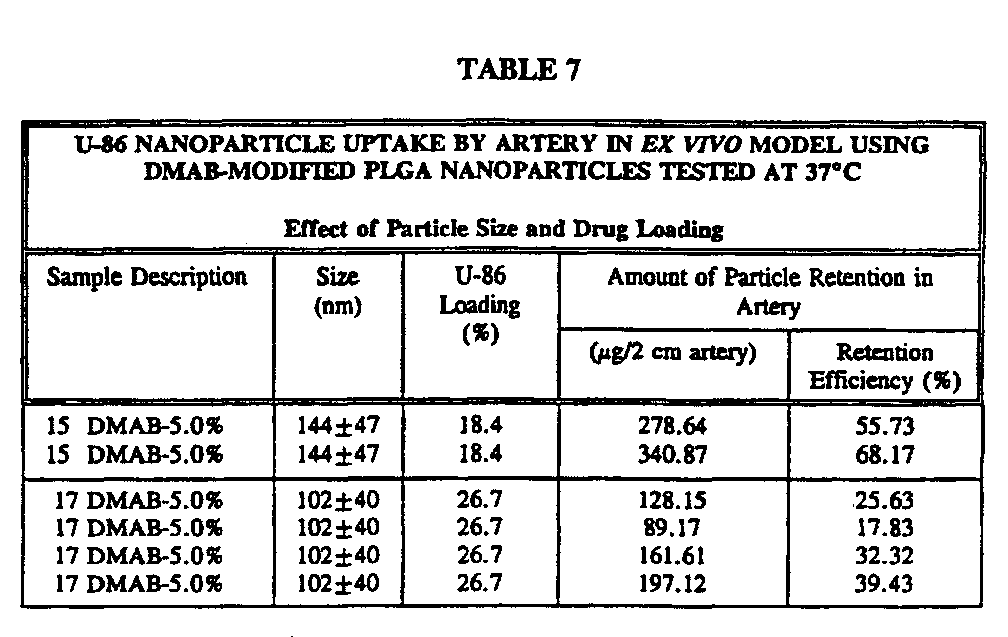

### in vivo Arterial Uptake Studies in a Rat Model

Nanoparticles made in accordance with the principle of the invention were evaluated for *in vivo* uptake and retention. The left carotid artery of rats, male Sprague-Dawley weighing 400-500 g, were exposed. A 2F Fogarty embolectomy balloon catheter (BSI, Minneapolis, MN) was used to remove the endothelial layer of the exposed artery. A 1 mm incision (also known as an arteriotomy) was made with an arteriotomy scissors in the rat's left or right common carotid artery, which was restrained by 3-0 silk ligatures to prevent bleeding. A Fogarty catheter (sized 2-0 French) was inserted into the incision and advanced into the arterial segment to the distal ligature. The balloon tip of the catheter was inflated with carbon dioxide and the catheter was pulled back and forth three times to create an arterial injury by denuding the endothelium. The catheter was then removed. At the same arterial incision, a catheter was inserted into the artery for infusing a nanoparticle suspension (200 µl) into the injured section of the carotid artery while the distal end of the artery was temporarily ligated. The catheter was removed after 60 seconds and the port was closed. The distal end of the carotid artery was opened to resume normal blood flow. After 2 hours, both left and right carotid arteries were harvested. The drug level in the artery samples was quantitated to evaluate nanoparticle retention *in vivo*. In a second set of experiments, nanoparticles were prepared so as to contain a fluorescent dye, Rhodamine B. The harvested carotid arteries were frozen and cross-sectioned to study the histology and location of the particles in the arterial walls.

DMAB and DEAE-Dextran modified nanoparticles, Samples 40 and 43 on Table 5, were used in this *in vivo* rat model to demonstrate that nanoparticles are preferentially taken up at the location of infusion (left carotid artery) as compared to the right carotid artery. The results for 10 mg segments of left carotid artery (n=11 rats) as compared to right carotid artery are: 7.77 ± 1.46 µg nanoparticles as compared to 2.98 ± 0.27 µg nanoparticles. Similar results were observed with dexamethasone-loaded nanoparticles (2.7 ± 1.3 µg nanoparticles per 10 mg segment of left carotid artery as compared to an undetectable amount in the right carotid artery (n=9 rats; detection limit of 0.1 µg/mg).

Histological examination of fluorescent-labeled nanoparticles which were loaded with dexamethasone (15% w/w; Example 3) also revealed significant presence in the arterial wall. Dexamethasone-PLGA nanoparticles containing Rhodamine B as a fluorescent marker were suspended in normal saline (50 mg/ml) and infused into rat carotid artery after triple balloon angioplasty denudation as described hereinabove. Multiple (four) infusions are made with each infusion consisting of 75 µl nanoparticle suspension. Arterial segments were harvested at different time periods (24 hours, 3 days, 7 days, and 14 days) and cryosectioned to observe the presence of nanoparticles with a fluorescence microscope. Fluorescent activity was observed in the artery until 7 days post-infusion.

### Long Term in vivo Arterial Uptake Studies in Animal Models

### (1) Pigs

In addition to the *in vivo* studies with rats, the nanoparticles were tested on pigs, weighing between about 30-40 lbs. In each subject pig, the elastic lamina of the coronary artery was ruptured by over inflation of a balloon tip catheter. A nanoparticle suspension (2.5 to 10 mg/ml in normal saline) was infused at the location of the injury by a Wolinksy (28 or 96 hole) or D-3 balloon catheter (Sci-Med, Minneapolis, MN) at 1-3 atmosphere pressure over 1 to 5 minutes. After 2-6 hours, the coronary arteries were harvested and quantitated for drug levels to calculate nanoparticle retention.

The results are given in Table 8 for nanoparticles loaded with U86, having the indicated surface modification. Nanoparticles with DMAB surface modification were retained in higher amounts than unmodified nanoparticles. The increased binding demonstrates the tissue specific increase in affinity for the surface modified nanoparticles.

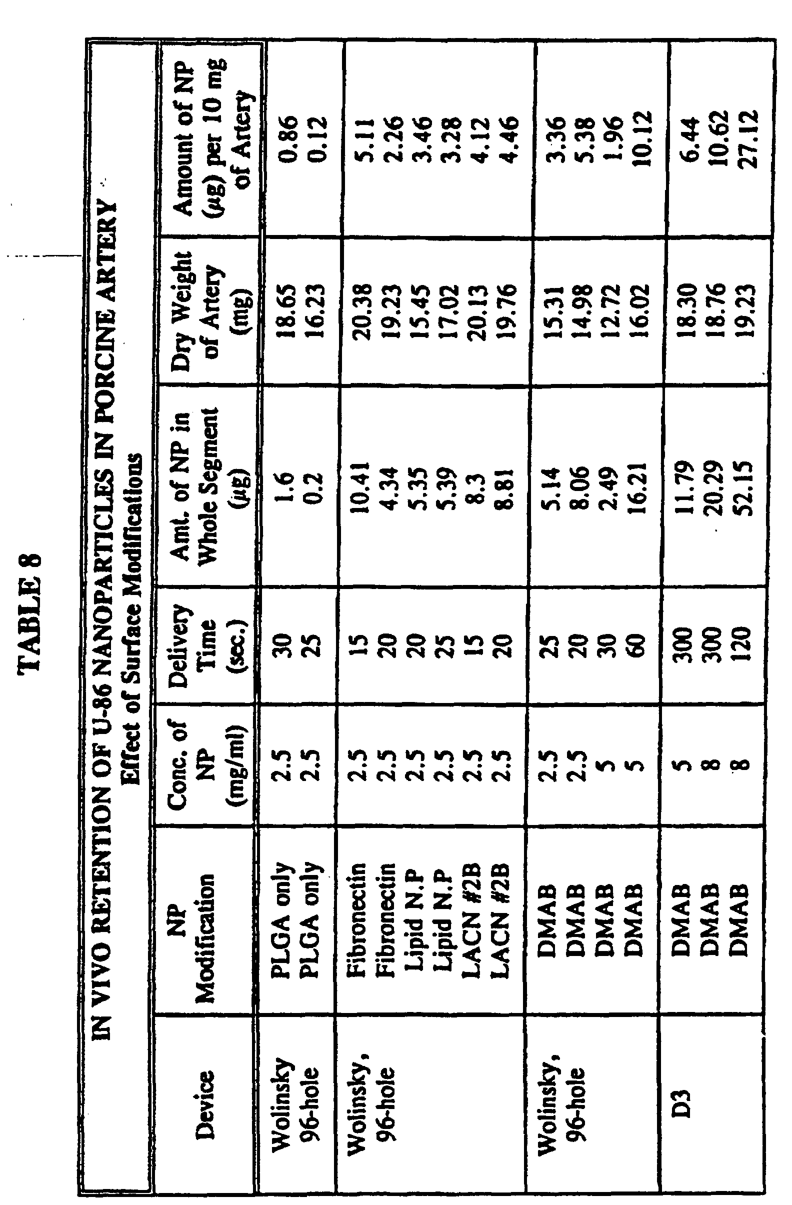

The amount of nanoparticles in the artery after one hour of blood circulation was not detectably different from the amount in arteries which were harvested immediately. This result indicates that the nanoparticles have penetrated into the tissue and/or cells and can not be washed away easily. The fluorescence microscope examination confirmed the retention. No significant difference was seen between the results of delivery with the two types of catheters (Wolinsky and Dispatch). Lower and relatively steady plasma U86 levels were observed after the local delivery of nanoparticles as compared to an iv injection of U86 solution.

U86 loaded-PLGA nanoparticles (15% w/w) with 5% DMAB surface modification and a particle size between 100-149 nm were suspended in normal saline at a concentration 15 or 30 mg/ml. The nanoparticles (NP) were administered to pigs which were sacrificed at 30 minutes or after one hour. The results are shown on Table 9.

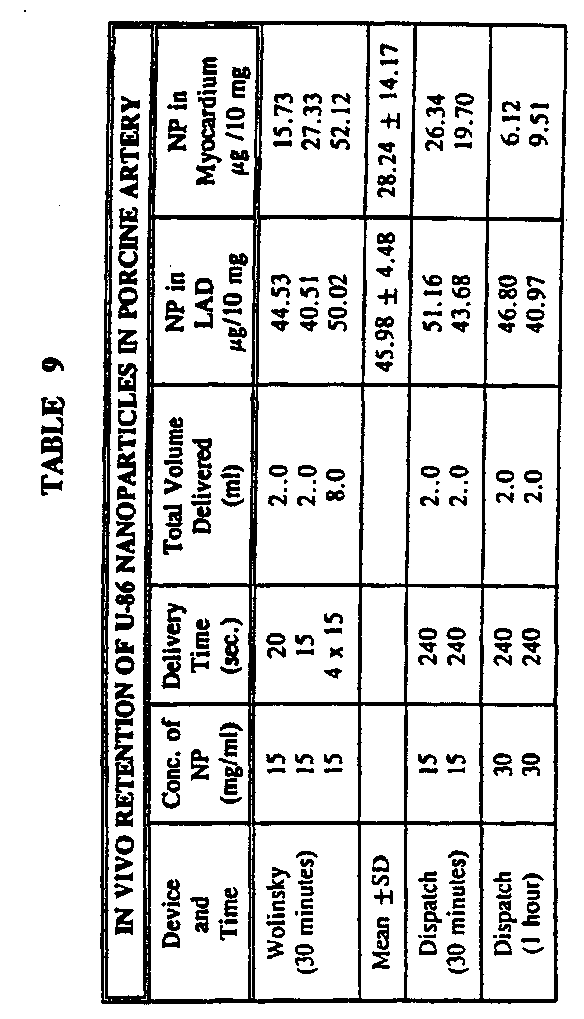

Delivery of a high concentration (30 mg/ml) of nanoparticles showed an average of about 45 µg uptake per 10 mg dry artery in *in vivo* pig studies. The length of the left anterior descending coronary artery (LAD) segment utilized for this measurement is about 1.5 cm and weighs about 15 mg (dry). Therefore, roughly 1 cm of treated LAD will be able to uptake about 45 µg nanoparticles by local delivery. There is about 7 µg net U86 in 1 cm of treated artery.

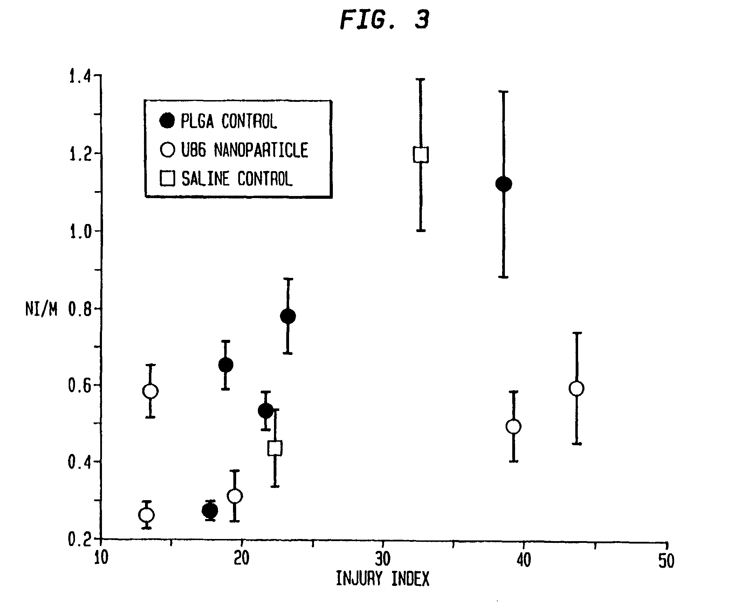

In addition to the foregoing, controlled release of U-86 from PLGA nanoparticles locally administered to pigs following balloon angioplasty-induced injured with a Sci-Med Dispatch catheter resulted in significant inhibition of restenosis as compared to saline and non-drug containing PLGA controls. Fig. 3 is a plot of neointimal area divided by medial area ratios (NI/M) plotted against the total injury index for the artery as standardized by Upjohn laboratories (Am. Heart J., Vol. 127, pages 20-31, 1994). The Upjohn test quantifies the severity of vascular damage (injury index) and the extent of neointimal (NI) hyperplasia (proliferation index) induced by over-inflation of the balloon. The injury index is the internal elastic lamina fracture length divided by the internal elastic lamina circumference x 100. The data shown on Fig. 3 demonstrate a statistically significant reduction in restenosis with regional release of U86 from nanoparticles or the present invention.

### (2) Rats

Similar long-term *in vivo* studies were conducted using rats. DMAB-modified U86-containing PLGA nanoparticles (U86 at 14.6% loading; mean particle size 130 ± 35; suspension concentration of 10 mg/ml of normal saline were infused into the left carotid artery of rats and subsequently harvested at 2 hours, 1 day, and 2 days post-injection. The amount of nanoparticles (µg) in a 10 mg segment of left artery was 9.00 ± 0.28; 9.19 ± 0.28; and 7.95 ± 0.41, respectively. The right carotid artery of each rat was used as the control. The amount of nanoparticles (µg) in a 10 mg segment of right carotid artery was 1.01 ± 1.55; 2.77 ± 0.24; and 0.51 ± 0.60, respectively.

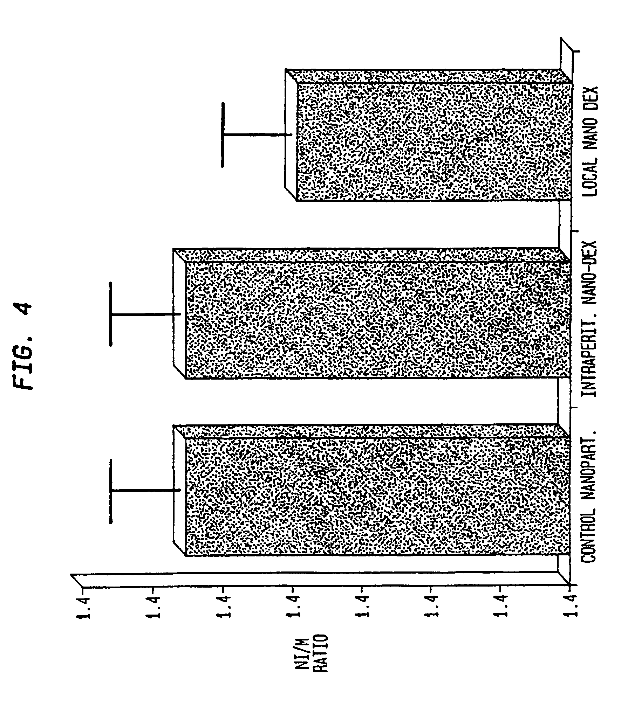

In studies employing PLGA nanoparticles incorporating dexamethasone (15% w/w), rats were subjected to triple angioplasty injury of the carotid artery. The rats were divided into three experimental groups: controls (nanoparticles with no bioactive agent), animals receiving intraperitoneal injection of nanoparticles containing dexamethasone, and animals to which dexamethasone-loaded nanoparticles were injected into the site of injury. After two weeks, the injured arteries were harvested and analyzed. Fig. 4 is a graphic representation of the inhibition of restenosis following the local administration of dexamethasone-containing nanoparticles (statistically significant; p>0.006). The data is expressed as the NI/M ratio as described hereinabove.

### Acute in vivo Studies of Arterial Uptake in Dogs

*in vivo* Experiments were conducted with dogs, using the DMAB, DEAE-dextran, and fibrinogen (5%) surface-modified PLGA nanoparticles made in accordance with the method of Examples 8 and 7.

Dogs under general anesthesia were subjected to a triple balloon angioplasty of both femoral arteries using a Bard angioplasty catheter. Following denuding of the endothelium, the damaged femoral segment was isolated with ligatures and filled with a small volume (200 µl) of a 5 mg/ml suspension of nanoparticles in normal saline at one atm pressure. The arterial wall was repaired to prevent bleeding, and after 60 seconds, blood was permitted to flow through the artery. After 30 minutes, the animal was euthanized and the both the damaged artery and the contralateral artery were retrieved for analysis by HPLC. The results show that fibrinogen enhances uptake somewhat as compared to control in both the *ex vivo* and *in vivo* studies. Between 40 and 50% of the nanoparticles suspended in the artery for the one minute isolation period were actually taken up by the arterial wall. Virtually no nanoparticles were detected in the contralateral artery. Moreover, the fibrinogen coated nanoparticles had nearly one and a half time more uptake than the DMAB-coated nanoparticles.

The results for U86-loaded PLGA nanoparticles which had been surface-modified with fibrinogen and DMAB (5%) in accordance with Example 7 are shown below in Table 10. The PLGA nanoparticles had a mean particle diameter of 130 ± 35 nm and a 14.6% drug loading prior to the application of the named coating. The right femoral artery of dog # 2 was analyzed as a control to evaluate the systemic distribution of nanoparticles *in vivo*. The "CONTROL" listed in Table 10 was an artery from a non-treated dog.

**TABLE 10**

| Treatment | Amount of NP in Segment (µg) | Dry Weight of Artery (mg) | NP (µg) in 10 mg artery | Mean ± SE |
|---|---|---|---|---|
| **Fibrinogen:** | | | | |
| Left Femoral #1 | 125.57 | 30.07 | 41.76 | 32.20 ± 3.22 |
| Left Femoral #2 | 95.65 | 29.3 | 32.65 | |
| Right Femoral #2 (as control) | 3.69 | 40.47 | 0.91 | 0.91 |
| **DMAB:** | | | | |
| Left Femoral #1 | 87.54 | 37.93 | 23.08 | |
| Left Femoral #2 | 43.19 | 18.74 | 23.05 | 25.13 ± 1.19 |
| Left Femoral #3 | 70.57 | 24.12 | 29.26 | |
| **CONTROL** | -0.21 | 32.37 | -0.06 | -0.06 |

A similar *in vivo* dog experiment was conducted using different delivery techniques. The data in Table 10 was obtained following a one-minute residence time in an ligated artery segment. PLGA nanoparticles of average particle size 161 ± 42 nm and 15.5% loading of U86 were coated with 5 % DMAB and suspended in normal saline and administered to dogs as a 15 second exposure, or as a series of four 15 second exposures separated by one minute of blood flow. Referring to Table 10, the DMAB-coated nanoparticles were retained in a 10 mg segment of femoral artery in an average amount of 25.13 ± 1.19 µg. A 15 second exposure resulted in nearly the same amount of retention, specifically 21.46 ± 0.73 µg. However, a series of four 15 second exposures resulted in more than double the amount of retention, 49.11 ± 2.42 µg.

A similar experiment was conducted with rats using DMAB-modified PLGA nanoparticles loaded with U86 (15.5%; particle size 161 ± 42 nm) in normal saline at a concentration of 10 mg/ml. The DMAB-coated nanoparticles administered in a single, 60 second exposure were retained in a 10 mg segment of left carotid artery in an average amount of 9.00 ± 0.28 µg. However, a series of four 15 second exposures resulted in more than double the amount of retention, 20.37 ± 1.37 µg. Controls for this experiment comprised 10 mg segments of untreated right carotid artery which contained only 1.01 ± 1.55 µg and 2.08 ± 0.40 µg, respectively.

The higher the suspension concentration, the higher the arterial wall content of U86 in the acute *in vivo* dog studies reported herein. Nanoparticles, which were U86-loaded PLGA nanoparticles of particle size 120 nm with 15% drug loading and 5% DMAB surface modification (prepared as in Examples 8 and 7) were administered to dogs in concentrations ranging from 5 mg/ml to 100 mg/ml over 15 seconds. Table 11 shows the amount of nanoparticles (µg) retained in a 10 mg segment of artery as a function of nanoparticle concentration (mg/ml) in normal saline.

**TABLE 11**

| NP conc. (mg/ml) | Amount of NP in Segment (µg) | Dry Weight of Artery (mg) | NP (µg) in 10 mg artery | Mean ± SE |
|---|---|---|---|---|
| 5 mg/ml | 162.96 | 71.33 | 22.85 | 24.5 ± 3.38 |
| | 106.87 | 30.31 | 21.24 | |
| | 115.73 | 39.52 | 29.88 | |
| | 102.11 | 47.23 | 21.62 | |
| | 93.63 | 45.65 | 20.51 | |
| | 138.58 | 138.58 | 19.73 | |
| 10 mg/ml | 138.09 | 36.84 | 37.48 | 38.95 ± 2.07 |
| | 195.43 | 48.36 | 40.41 | |
| 15 mg/ml | 282.11 | 46.7 | 60.41 | 59.48 ± 0.66 |
| | 288.87 | 49.26 | 58.85 | |
| 20 mg/ml | 298.87 | 38.39 | 77.85 | 69.41 ± 5.97 |
| | 288.37 | 34.67 | 60.97 | |
| 30 mg/ml | 377.45 | 44.55 | 84.73 | 83.73 ± 1.38 |
| | 435.48 | 52.61 | 82.77 | |
| 50 mg/ml | 611.26 | 62.3 | 98.11 | 96.05 ± 2.92 |
| | 405.07 | 43.1 | 93.98 | |
| 100 mg/ml | 649.74 | 58.44 | 111.18 | 111.18 |

### C. Covalent Attachment of Surface Modifying Agent By Epoxy

In still other embodiments of the invention, the surface modifying agent is covalently linked to the pre-formed nanoparticles. In a preferred advantageous embodiment of the invention, a method has been developed to incorporate reactive epoxide side chains into the polymeric material comprising the nanoparticles, which reactive side chains can covalently bind other molecules of interest for various drug delivery applications. This embodiment is discussed in greater detail hereinbelow in Examples 11 and 12.

The polylactic polyglycolic acid co-polymers widely used in drug delivery research for biodegradable formulations inherently lack reactive groups, and therefore, are difficult to derivatize. A method has been developed to incorporate reactive epoxide side chains, which can covalently bind other molecules of interest for various drug delivery applications. In addition to PLGA, any polymer containing free hydroxyl, amino, sulfhydryl, carboxyl, anhydride, phenol, or the like, groups can be derivatized by this method aspect of the invention.

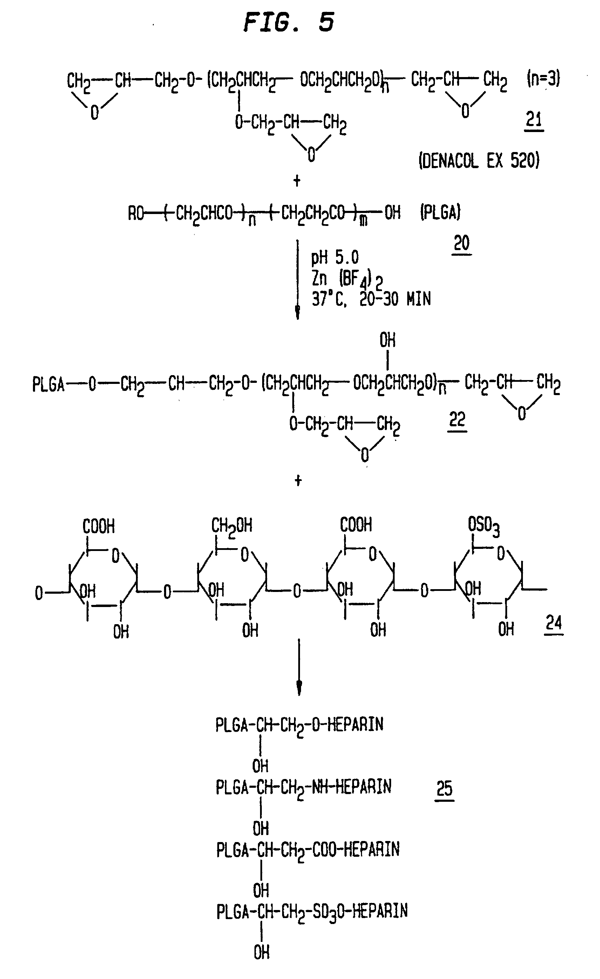

Fig. 5 is a schematic representation of a synthetic procedure for coupling an epoxide compound to an hydroxyl end-group of polymeric nanoparticles. In the specific embodiment shown in Fig. 5, the nanoparticles comprise PLGA (compound 20) and are made by an in-solvent emulsification-evaporation technique, for example, such as that described in Example 1. Of course, the PLGA, nanoparticles may be formed by any technique prior to epoxide derivatization in accordance with this aspect of the invention.

The pre-formed PLGA nanoparticles are suspended in a liquid, illustratively a buffer to which a catalyst has been added. In the embodiment shown on Fig. 5, the suspending media is a borate buffer at pH 5.0 and the catalyst is zinc tetrafluoroborate hydrate, Zn(BF₄)₂. Suitable catalysts include, but are not limited to, tertiary amines, guanidine, imidazole, boron trifluoride adducts, such as boron trifluoride-monoethylamine, bisphosphonates, trace metals (*e.g.*, Zn, Sn, Mg, Al), and ammonium complexes of the type PhNH₃ + AsF₆₋. In other embodiments, the reaction can be photoinitiated by UV light, for example, in the presence of an appropriate catalyst, which may be titanium tetrachloride and ferrocene, zirconocene chloride, carbon tetrabromides or iodoform.

An epoxide compound dissolved in a suitable solvent, such as the buffer, is added to the nanoparticles suspension and permitted to react to form an epoxide-coupled polymer (compound 22). Referring to Fig. 5, the epoxy compound is a polyfunctional epoxide sold under the trademark Denacol (Nagasi Chemicals, Osaka, Japan; compound 21).

The epoxy compounds suitable for the practice of the present invention may be monomers, polyepoxide compounds, or epoxy resins. Illustrative reactive epoxides suitable for use in the practice of the invention include, without limitation, 1,2-epoxides such as ethylene oxide or 1,2-propylene oxide; and bifunctional or polyfunctional compounds, such as butane and ethane di-glycidyl ethers, such as diglycidyl butanediol ether, ethanediol diglycidyl ether, or butanediol diglycidyl ether (available from Aldrich Chemical, St. Louis, MO); erythritol anhydride; the polyfunctional epoxides sold under the trademark Denacol by Nagase Chemicals, Osaka, Japan; epichlorhydrin (Aldrich Chemical, St. Louis, MO); enzymatically-inducible epoxides available from Sigma Chemicals, St. Louis, MO; and photo-polymerizable epoxides (Pierce, Rockford, IL). The Denacol epoxides are polyfunctional polyglycerol polyglycidyl ethers. For example, Denacol 512 has 4 epoxides per molecule and Denacol EX521 has 5 epoxides per molecule.

The reactive epoxide groups of the epoxide-coupled polymer (compound 22) can then be reacted with various types of bioactive agents having functional groups which react with the epoxy linkage, such as alcohol, phenol, amines, anhydrides, *etc*. The result is a covalent link between the functionalized polymer and the bioactive agent(s) of interest (*e.g*., compound 24).

In the embodiment of Fig. 5, the bioactive agent of interest is heparin (compound 24). Heparin is a highly sulfated polyanionic macromolecule comprising a group of polydiverse straight-chain anionic mucopolysaccharides called glycosaminoglycans (molecular weight ranges from 5,000 to 30,000 daltons). Heparin contains the following functional groups, all of which are susceptible to reaction with an epoxide group: -NH₂, -OH, -COOH, and -OSO₃. If the reaction between the epoxide-coupled polymer and heparin is carried out at an acidic pH (5.0-9.0), the main reaction will be with the -NH₂ groups. The result is PLGA nanoparticles to which heparin is covalently bound (compound 25). Of course, the -OH groups in heparin may react with the epoxide groups at this pH.

The following are specific illustrative embodiments of the epoxy-derivatization technique. Although Example 12 is directed to the binding of heparin to the surface of epoxy-derivatized nanoparticles, it is to be understood that the epoxy-derivatization technique can be used to react various types of bioactive agents having functional groups which react with the epoxy linkage, such as alcohol, phenol, amines, anhydrides, *etc*., to nanoparticles. Even proteins and peptides, including antibodies, can be attached to epoxy-modified nanoparticlcs to achieve antibody-mediated drug delivery systems. Specific examples include heparin, bisphosphonate, DNA, RNA, and virtually any agent which contains hydroxy or amino groups, or which may be derivatizable to contain reactive groups.

### Example 11:

PLGA nanoparticles were prepared by an in-solvent emulsification-evaporation technique (similar to Example 8). PLGA (150 mg) was dissolved in 5 ml methylene chloride which was emulsified in aqueous PVA (2.5% w/v, 20 ml), over an ice bath, using a probe sonicator with an energy output of 65 Watts. The emulsion was stirred with a magnetic stirring bar at room temperature for 18 hours to permit the methylene chloride to evaporate. The nanoparticles were recovered by ultracentrifugation, washed three times with water, and resuspended in water by sonication for 3 minutes. The resulting suspension was lyophilized.

The lyophilizad PLGA nanoparticles (40 mg) were suspended in 5 ml borate buffer (50 mM, pH 5) by sonification for 3 minutes. A catalyst, which is in this specific embodiment, was zinc tetrafluoroborate hydrate (12 mg) was added to the nanoparticle suspension. A polyfunctional epoxide, Denacol 520 (3 epoxides per molecule, 14 mg) was dissolved in 2 ml borate buffer. The epoxide solution was added to the nanoparticle suspension with stirring at room temperature (37°C). After 30 minutes, the nanoparticles were separated by ultracentrifugation and washed three times with water to remove unreacted Denacol. The resulting product was epoxy-activated nanoparticles. The reaction of the PLGA nanoparticles and the epoxide was confirmed by proton NMR.

### Example 12:

In a specific illustrative embodiment, heparin is reactively bound to the epoxy-activated nanoparticles of Example 11 using the immobilized polyfunctional epoxide as the coupling agent. An excess of heparin is used so that only one site on each heparin molecule will react with the epoxide group. If a lesser amount of heparin is used, more sites on each heparin molecule will react with epoxy groups which will result in loss of anticoagulation ability.

PLGA nanoparticles (40 mg) made in accordance with Example 11 were resuspended in 20 ml borate buffer. A solution of heparin (14 mg) in borate buffer (4 ml; pH 5.0) was added to the nanoparticles with stirring at 37° C. The heparin solution and the nanoparticles were permitted to react for two hours, with gentle stirring. The nanoparticles were separated from the unreacted heparin by ultracentrifugation and dialyzing against normal saline over a 26 hour period. The resulting heparinized nanoparticles were then lyophilized. The heparin content of the nanoparticles of this specific embodiment was measured by Toluidine Blue metachromatic assay and found to be 7.5 µg/mg nanoparticle.

The antithrombogenic effect of the bound heparin was evaluated by the activated partial thromboplastin (APTT) test. Dog plasma (0.5 ml) was mixed with 5 mg heparin-coupled nanoparticles and incubated at 37° C for 1 hour with shaking. The thrombin time of the test plasma was determined using a BBL Fibrosystem Fibrometer (Becton Dickinson Microbiology Systems, Cockeysville, Maryland) following a standard procedure. Plasma from the same dog was incubated with PLGA nanoparticles as a control. The heparinized PLGA nanoparticles showed significant anticoagulation activity since no clot formation occurred over more than 200 seconds. Control particles which were not reacted with heparin, on the other hand, permitted clotting in 16.7 seconds.

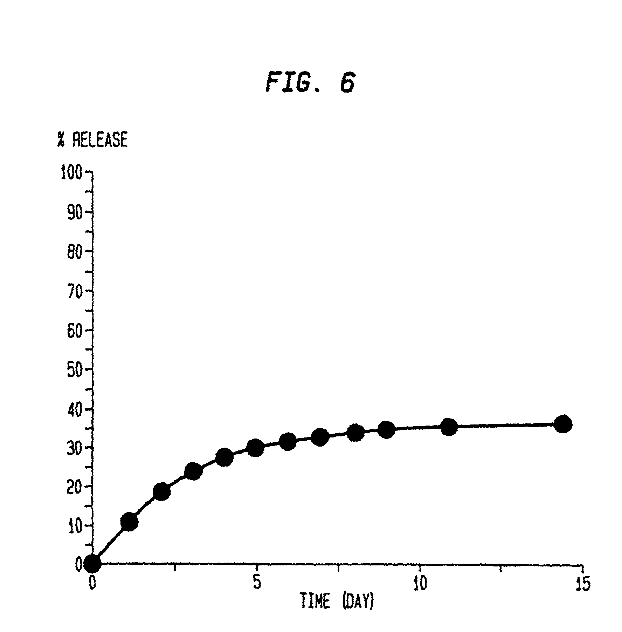

The stability of the bound heparin was tested with radiolabeled ¹⁴C heparin at 37° C for 15 days. The results are shown on Fig. 6 which is a graphical representation of the *in vitro* release of heparin as measured by radioactivity expressed as a percent of bound heparin. About 30% of the bound heparin was released from the nanoparticles during the first 5 days. The remaining 70% was bound with a high level of stability. About 65% of the heparin remained bound to the nanoparticles after 15 days of release at 37° C. This indicates a stable chemical coupling of heparin to the nanoparticles.

### Example 13:

PLGA nanoparticles were prepared and epoxy-activated in accordance with the method of Example 11. The epoxy-activated nanoparticles (70 mg) were suspended in 5 ml bicarbonate buffer, pH 9.2. BSA (30 mg) was separately dissolved in 5 ml of the same buffer, and mixed with the nanoparticle suspension. The reaction was allowed to take place for 24 hours at 37° C with stirring on a magnetic stir plate. The resulting nanoparticles were collected by ultracentrifugation, and washed three times with either water or phosphate buffered saline (pH 7.4) containing 0.05% Tween-80.

The amount of BSA bound to epoxy-activated nanoparticles (PLGA/BSA+EP) washed in either (H₂O) or buffer is compared to the amount of BSA bound to non-activated PLGA nanoparticles (PLGA/BSA) in Table 12. Plain un-activated PLGA nanoparticles, containing no BSA, were used as controls. Table 12 demonstrates significantly better binding of BSA on epoxy-activated nanoparticles.

**Table 12**

| Samples | Abs. 605 nm | BSA (µg) | Weight of NP (mg) | BSA (µg/mg NP) | Net BSA (µg/mg NP) |
|---|---|---|---|---|---|
| PLGA | 0.156 | 15.92 | 9.35 | 1.70 | 0 |
| PLGA | 0.202 | 22.32 | 10.07 | 2.22 | 0 |
| PLGA/BSA+EP/H₂O | 0.857 | 113.49 | 5.74 | 19.77 | 17.87 |
| PLGA/BSA+EP/Buffer | 0.943 | 125.47 | 8.14 | 15.41 | 13.51 |
| PLGA/BSA/H₂O | 0.350 | 42.92 | 7.26 | 5.91 | 4.01 |
| PLGA/BSA/Buffer | 0.250 | 29.00 | 3.72 | 7.80 | 5.90 |

It should be noted that, while pre-polymerized and pre-formed nanoparticles were epoxy-activated and derivatized by the method described hereinabove, the monomers comprising the polymer, for example, can be functionalized prior to polymerization with the reactive epoxide groups without departing from the spirit and scope of the present invention.

### D. Incorporation of Surface Modifiers Into Polymer Core Matrix

In yet another alternative technique for providing surface modification, the surface modifying agent is incorporated into the matrix of the biocompatible, biodegradable polymer comprising the nanoparticle core.

### (1) Co-incorporation of a Surface-Modifying Polymer

In this one aspect of the facet of the invention, the nanoparticle polymer core may comprise, at least partially, a biodegradable, biocompatible polymer which has a surface modifying property. In a specific illustrative embodiment detailed below in Example 14, isobutyl cyanoacrylate is combined with PLGA as the organic phase of an in-solvent emulsification-evaporation technique. The result is nanoparticles having a PLGA-cyanoacrylate polymer core. The cyanoacrylate imparts a bioadhesive property to the nanoparticles. Of course, the amount of cyanoacrylate relative to PLGA can be modified.

Other polymers, such as hydrogels or Pluronics, can be co-incorporated with PLGA or another biodegradable, biocompatible polymer in accordance with the principles of the invention, to impart a bioadhesive property. Further, it is to be clearly understood that this example is illustrative only, and that many other polymers can be co-incorporated with biodegradable, biocompatible polymers to form combinations having various improved properties, including those properties attributed to "surface modifying agents" as used herein.

### Example 14:

In a typical preparation, 108 mg PLGA and 36 mg isobutyl cyanoacrylate (Polyscience, Inc., Warrington, PA) were separately dissolved in 5 ml methylene chloride and then combined to make an organic phase. U86 (67 mg) was dissolved in the solution comprising the organic phase. The organic phase was emulsified into 25 ml of 2.5% wlv aqueous PVA with sonication, at 55 Watts of energy output for 10 minutes over an ice bath. The organic phase was evaporated from the emulsion at room temperature for 40 hours. The resulting nanoparticles were recovered by ultracentrifugation at 140,000 g, washed three times with water, and lyophilized. The PLGA-cyanoacrylate nanoparticles were recovered in about 65% yield, with U86 loading of 25%, The mean particle diameter was 123 ± 37 nm. In yet another embodiment of this aspect of the invention, the biocompatible, biodegradable polymer is a novel epoxy-derivatized and activated polycaprolactone. Polycaprolactone, a biodegradable polymer used in the medical field, has long-term sustained release potential. However, conventional polycaprolactones are not useful as carriers for hydrophilic active agents, or for rapid release applications. In addition, polycaprolactones lack reactive functional groups that can be used to derivatize, or chemically modify, the polymer.

### (2) Polycaprolactone-containing Multiblock Copolymers

In this embodiment, hydrophilic segments, such as poly(ethylene glycol), are introduced into a PCL polymer chain to form novel biodegradable hydroxy-terminated poly (ε-caprolactone)-polyether multi-block copolymers useful as carriers for biologically active agents. The novel polycaprolactone-based polymers, therefore, have more desirable hydrophilic characteristics than conventional polycaprolactone, controllable biodegradation kinetics, and the potential for further derivatization, such as through the addition of reactive epoxy groups as described hereinabove.

Advantageously, it is possible to form nanoparticles from the novel polycaprolactone-based polymers of the present invention without the addition of a detergent or emulsifying agent. When an organic solution of poly(ethylene glycol)-polycaprolactone, for example, or other similar types of polymers having both hydrophilic and hydrophobic moieties in a single molecule, is added into an aqueous phase, the hydrophilic portion of the polymer molecule (PEG) will orient towards the aqueous phase and the hydrophobic portion (PCL) will orient towards the center of the emulsion droplet. Thus, a nanoparticle core consisting of a hydrophobic portion with a hydrophilic surface will be formed. The outwardly facing PEG is a very good emulsifier and will assist in the formation of an emulsion. Moreover, PEG will also stabilize the emulsion and prevent aggregation of the emulsion droplets.

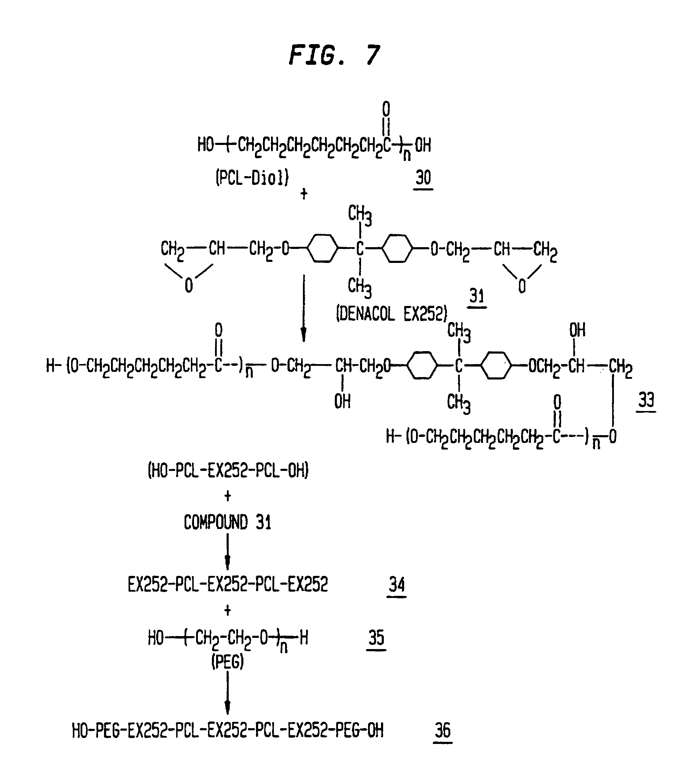

Block copolymers of the hydrophobic PCL segment and a hydrophilic segment, which may be a hydrophilic polyether, may be synthesized by multiple reactions between hydroxyl end groups and epoxide groups in a reaction scheme illustrated in Fig. 7. The illustrative reaction scheme of Fig. 7 can be used to chemically link copolymer blocks in ABA, BAB, as well as (AB)ₐ, form, so that hydrophobicity and molecular weight of the block copolymers can be tailored as desired. Placing hydroxyl groups on both ends of the block copolymers permits ready chemical modification of the polymer, such as coupling to heparin, albumin, vaccine, antibodies, or other biomolecules.

Referring to Fig. 7, compound 30 is polycaprolactone diol (PCL-Diol). The highest weight PCL-diol commercially available has a molecular weight of 3000 which is not long enough to serve as a main segment in a copolymer used as a sustained release biodegradable nanoparticle. In order to get a higher molecular weight PCL-diol which will be a solid at the contemplated temperatures of use, PCL-diol (compound 30) is reacted with a difunctional epoxide compound, such as Denacol EX252 (compound 31) in a 2.5:1 molar ratio. An excess of PCL-diol was used in this particular case so that the PCL-diol would be an end group in the polymer chain. If the ratio is reversed, *i.e*., there is an excess of EX252, then the epoxide compound will be an end group in the polymer chain. The unreacted PCL is removed by gradient precipitation. The result is an expanded PCL-diol, which in this specific embodiment has the structure: HO-PCL-EX252-PCL-OH (compound 33).

Although the difunctional epoxide, Denacol EX252 has been used in this specific embodiment, it is to be understood that any polyfunctional epoxide, herein defined as a di- or multifunctional epoxide, such as Denacol EX521 and EX512, or compounds having a single epoxide functionality such as ethylene oxide or 1,2-propylene oxide, can be used in the practice of the invention.

The expanded PCL-diol compound 33 is reacted with excess difunctional epoxide compound to achieve end-capping of the PCL-diol with epoxide groups. Referring to Fig. 7, one of the two epoxide groups in the difunctional epoxide compound 31 reacts with the hydroxyl ends of the PCL-diol compound 33 and leaves the other epoxide group free so that both ends of the PCL-diol are capped by an epoxide group. The excess epoxide compound is removed by precipitation and washing. The result is an epoxide-capped PCL, EX252-PCL-EX252-PCL-EX252, compound 34.

Compound 34 (Block A) is reacted with an excess of a polyether diol (Block B). In the embodiment shown in Fig. 7, the polyether diol is polyethylene glycol (PEG; M. Wt. 4500), compound 35. Block A is reacted with Block B in a 1:4 molar ratio in this specific embodiment. The resulting copolymer is collected by precipitation and the excess of polyether is removed by washing with water. The final copolymer is a BAB triblock copolymer linked with epoxides and terminated at both ends by hydroxyl groups, compound 36. In this specific example, compound 36 is HO-PEG-EX252-PCL-EX252-PCL-EX252-PEG-OH.

To make an ABA triblock copolymer, the reaction sequence is reversed, *i.e*., the polyether-diol is used to form Block A and the PCL-diol is used as Block B. In addition, multi-block copolymers may be made using ABA or BAB triblock copolymers as a pre-polymer (analogous to compound 33). In other words, the ABA prepolymer is end-capped with epoxide compound and reacted with B block which results in a BABAB copolymer or A block for a ABABA copolymer. A person of ordinary skill in the art can devise a multiplicity of hydroxy- and/or epoxy-terminated polymers using the techniques of the present invention.

Of course, other hydrophobic polymers may be used for Block A/B, for example, such as polylactides, polyglycolides, PLGA, polyanhydrides, polyamino acids, or biodegradable polyurethanes. Other hydrophilic polymers suitable for block B/A include polaxomers, such as Pluronic F68 and Pluronic F127, and poly(propylene oxide) (PPO).

In choosing A and B polymers, a person of ordinary skill in the art would choose an optimal balance of hydrophilic and hydrophobic molecules for a particular application. More hydrophilic polymers will have faster drug releasing properties and *vice versa*. Physical properties, such as shape and stability of the drug system, as well as the molecular weight of the polymer will affect the release kinetics. The lower the molecular weight of the polymer, of course, the more rapid the rate of release.

The molecular weight of block copolymers made in accordance with the invention is in the range of 30,000 to 700,00 as measured by gel permeation or intrinsic viscosity, with approximately 90,000 to 100,000 being preferred for drug delivery applications.

### Example 15:

In a specific, illustrative embodiment, PCL-diol (1.5 g; 0.5 mMol.; Polyscience, Inc., Warrington, PA; M. Wt. 3,000) was reacted with Denacol EX252 (0.21 g; 0.55 mMol.) in 15 ml THE in the presence of Zn(BF₄)₂ catalyst (2% by weight according to epoxide compound) at 37° C under stirring for 28 hours. To separate the expanded PCL-diol from the non-expanded diol, gradient precipitation was carried out using heptane and the precipitated, higher molecular weight PCL was collected by centrifugation. The product, which is an expanded PCL-diol, HO-PCL-EX252-PCL-OH, was washed with 5 ml of heptane to remove free epoxide molecules and dried.

The expanded diol (0.75 g) was reacted with Denacol EX252 (0.42 g; molar ratio of PCL to EX252 was 1:4) ) in 10 ml THF, in the presence of Zn(BF₄)₂, at 37° C with stirring for 5 hours. The polymer was precipitated with 30 ml heptane. The collected product, which is an epoxide end-capped expanded PCL, specifically EX252-PCL-EX252-PCL-EX252, was washed with 10 ml of heptane to remove the excesses of epoxide compound and dried.

### Example 16:

The PEG-terminated compound 36, HO-PEG-EX252-PCL-EX252-PCL-EX252-PEG-OH, can be made as follows:

Compound 34 (1 g) is dissolved in 15 ml THF to which 2 g of PEG (compound 35; 1:3 molar ratio of compound 34 to PEG) and 20 mg Zn(BF₄)₂ had been added. The reaction is permitted to proceed for 48 hours, on a shaker table, at 37° C. The polymer HO-PEG-EX252-PCL-EX252-PCL-EX252-PEG-OH (compound 36) is precipitated with heptane, centrifuged, and washed twice with 50 ml of water.

### Example 17:

ABA triblock copolymers were made in accordance with the illustrative general reaction scheme of Fig. 7, using the following polyethers as Block A: PEG E4500, the polaxomers Pluronic F68 (F68) and Pluronic F127 (F127), and polypropylene oxide) (PPO). The various polyethers were incorporated into ABA triblock copolymers with PCL to obtain polymer specimens with varying hydrophilicity and mechanical properties. PPO is a hydrophobic polyether polymer of M Wt. 4000. The Pluronics are diblock copolymers with PPO as the hydrophobic block and poly(ethylene oxide) (PEO) as the hydrophilic block. Pluronic F127 has a molecular weight of about 12,600 and is 70% PPO and 30% PEO. Pluronic F68 has a molecular weight of about 6,000 and is 80%PPO and 20% PEO, and hence, less hydrophilic than Pluronic F127. PEG is the most hydrophilic polyether in the group.

In a specific illustrative embodiment, Pluronic F68 (1.5 g; 0.25 mMol.) was reacted with Denacol EX252 (0.42 g) in 15 ml THF in the presence of 40 mg Zn(BF₄)₂ (1:4 molar ratio of F68 to EX252), at 37° C with stirring for 6 hours. The reaction mixture was precipitated in 20 ml heptane. The collected product was washed with 5 ml of heptane twice to remove the excess unreacted epoxide, and dried. The result was an epoxide end-capped Pluronic F68 (Block A).

The epoxide end-capped Pluronic F68 was reacted with PCL-diol (2.3 g) in 15 ml THF in the presence of Zn(BF₄)₂ at 37° C with stirring for 48 hours. Gradient precipitation in heptane was used to separate the resulting copolymer from non-reacted free PCL. The precipitated copolymer was collected by centrifugation and dried. The resulting hydroxy-terminated ABA block copolymer is HO-PCL-EX252-F68-EX252-PCL-OH, designated as PCL/F68/PCL in Table 9, is shown below:

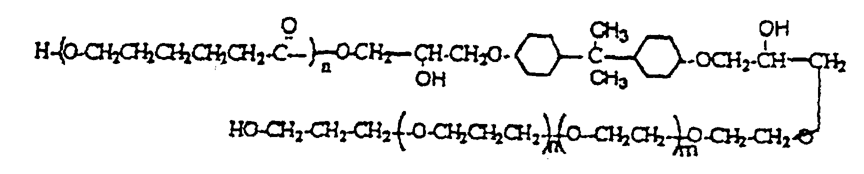

The general appearance and physical properties of the ABA and BAB triblock copolymers formulated in Example 17 are shown in Table 13. The corresponding hydroxy-terminated BAB block copolymer, HO-F68-EX252-PCL-EX252-F68-OH, is designated as F68/PCL/F68 in Table 13. The "/" marks indicate epoxy linkages in accordance with the present invention.

Using this scheme of designation, the hydroxy-terminated BAB triblock copolymer compound 36 on Fig. 7 is PEG/PCL(E)/PEG, where "(E)" indicates that the PCL is expanded with epoxy linkages as set forth in Example 15. Of course, the terminology PEG/PCL/PEG would indicted an hydroxy-terminated BAB triblock copolymer without additional expansion of the PCL component. The corresponding ABA triblock copolymer, HO-PCL-EX252-PEG-EX252-PCL-OH, or PCL/PEG/PCL, is shown below.

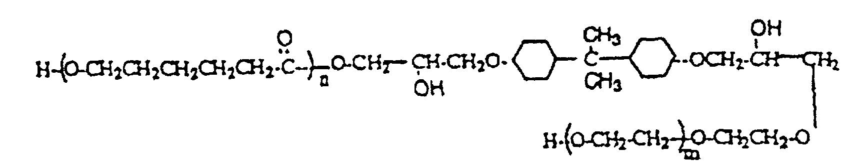

**Table 13**

| Polymer Type | Morphology | Water Solubility | Film-Forming Property |
|---|---|---|---|
| PCL/PEG/PCL | crystallizable powder | insoluble | strong, flexible |
| PEG/PCL/PCL | crystallizable powder | swells | flexible, breaks in water |
| PCL/F68/PCL | crystallizable powder | insoluble | strong, flexible |
| F68/PCL/F68 | crystallizable powder | insoluble | flexible |
| PCL/127/PCL | crystallizable powder | swells | brittle film |
| PCL/PPO/PCL | sticky wax | insoluble | does not form film |

Referring to Table 13, the most useful polymers, from the viewpoint of drug delivery, are the copolymers made from PCL and PEG or Pluronic F68. Polymers which do not crystallize, such as those containing a high level of PPO, have poor mechanical strength and are sticky. Polymers having a large hydrophilic segment, such as the polymer from PCL and Pluronic F127, are difficuh to separate from the aqueous phase and will not maintain a solid shape in contact with water, or body fluids. Successful drug delivery devices comprise polymers which are solid at body temperature, slowly dissolve or erode in the presence of body fluids, and non-inflammatory and non-toxic to tissues/cells. Other advantageous characteristics would include high drug loading efficiency, the ability to be derivatized, stability, and, in certain embodiments, the ability to be easily suspended in an injectable fluid medium.

In order to demonstrate that the reaction scheme of Fig. 7 produces ABA triblock copolymers as alleged, NMR spectra of the PCL/F68 and PCL/PEG copolymers were measured on a Bruker AM-360 apparatus using CDCl₃ as the solvent. A comparison of the proton NMR spectra of the starting materials and the final copolymer verified the molecular structure.

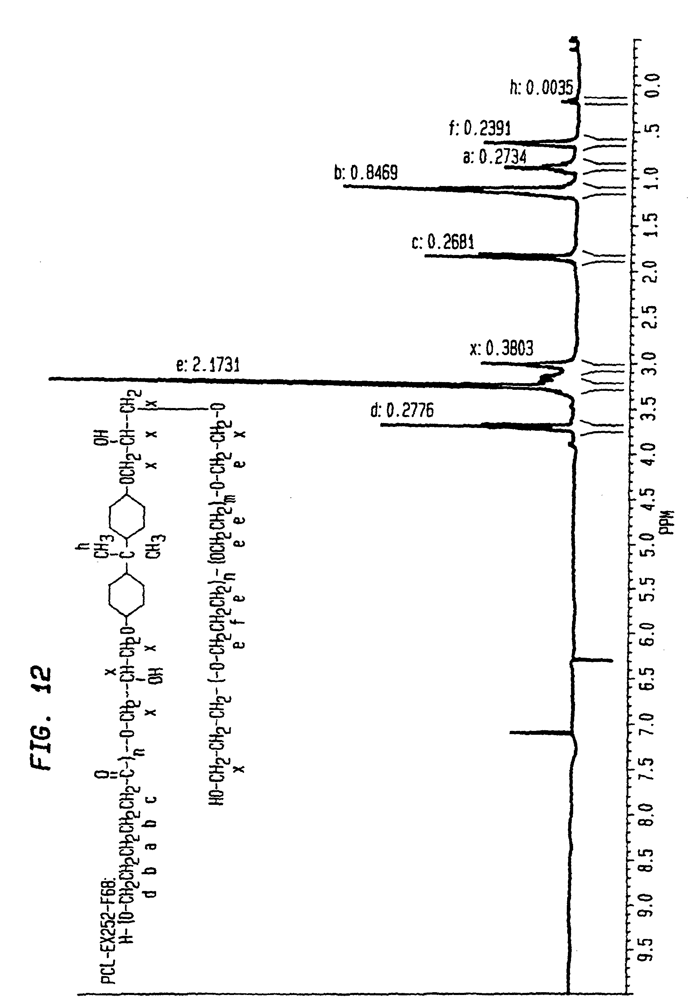

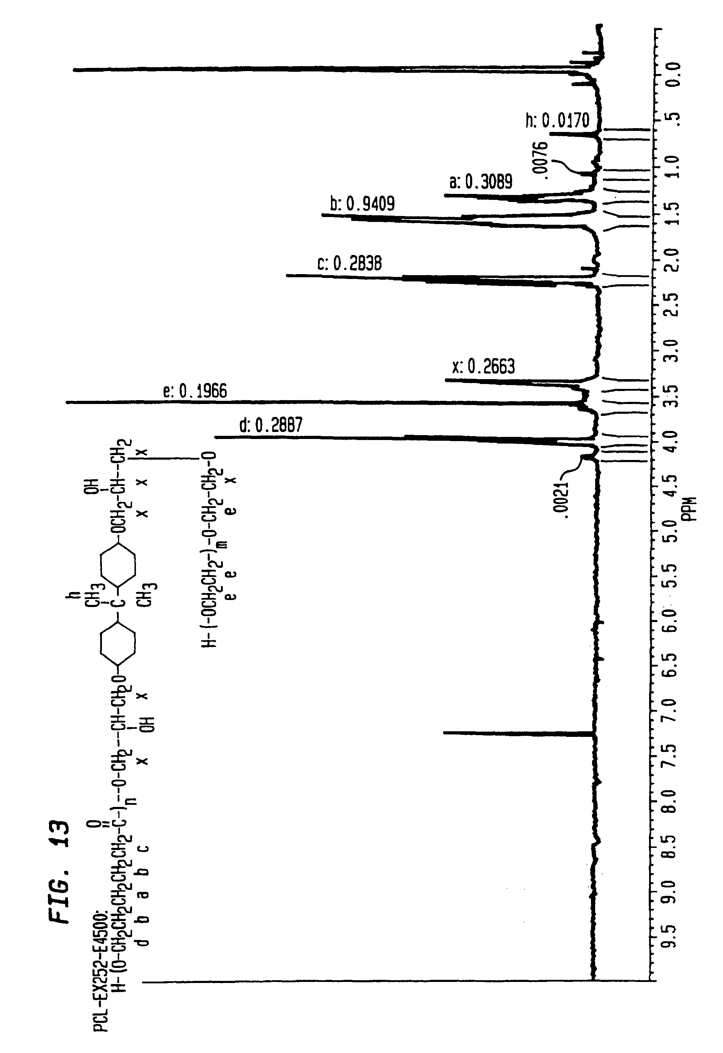

For the NMR study reported herein, the reaction compounds were PCL/PEG or PCL/F68 (see Figs. 13 and 12, respectively). However, when the polymers are used for drug delivery, further reactions may be carried out to form the triblock copolymers, PCL/PEG/PCL or PCL/F68/PCL.

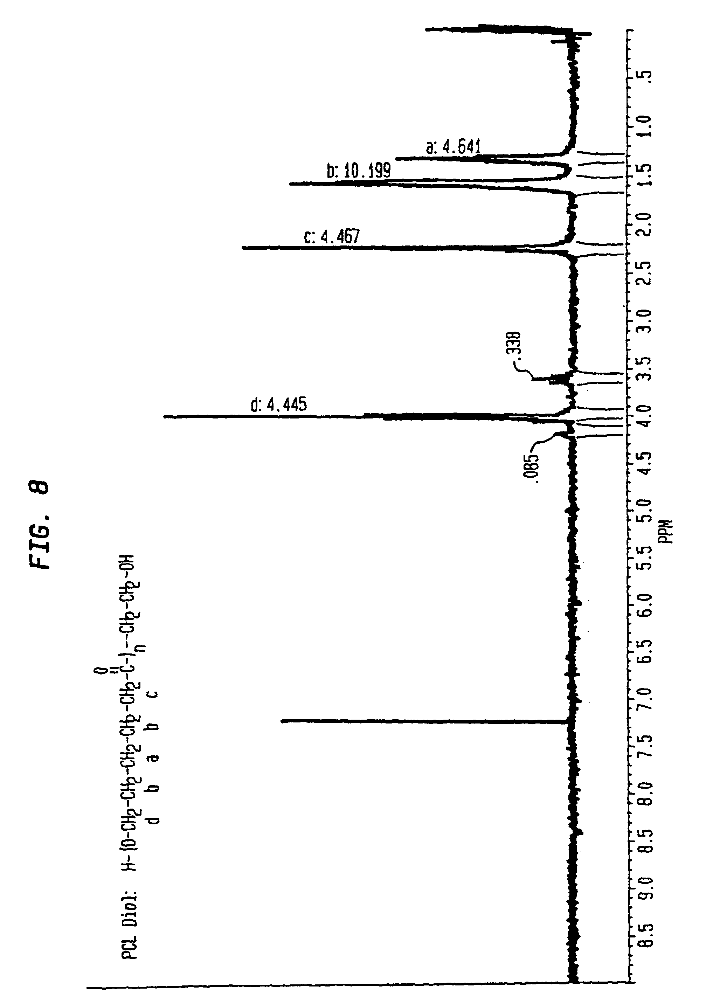

Figs. 8-11 show the spectra of the starting materials PCL-diol, Pluronic F68, PEG E4500, and Denacol EX252, respectively. The spectrum of the PCL/F68 copolymer is shown in Fig. 12 and matches the proposed molecular structure shown hereinabove. Comparing the chemical shifts in the starting materials, PCL, F68, and EX252, to the shifts observed on Fig. 12, it is certain that there are PCL segments (chemical shifts at positions a, b, c, d) and F68 segments (chemical shifts at positions e and f) in the final product. A small peak at δ 0.7 ppm which has the lowest intensity should be the shift of proton h in the -CH₃ groups in Denacol EX252. The reaction between epoxide groups and hydroxyl end-groups was confirmed by the chemical shift at δ 3.401 ppm (proton x) which represents the protons in the linking bonds resulting from the reaction. The -CH₂OH end groups in the final copolymer gave a shift at 3.415 ppm.

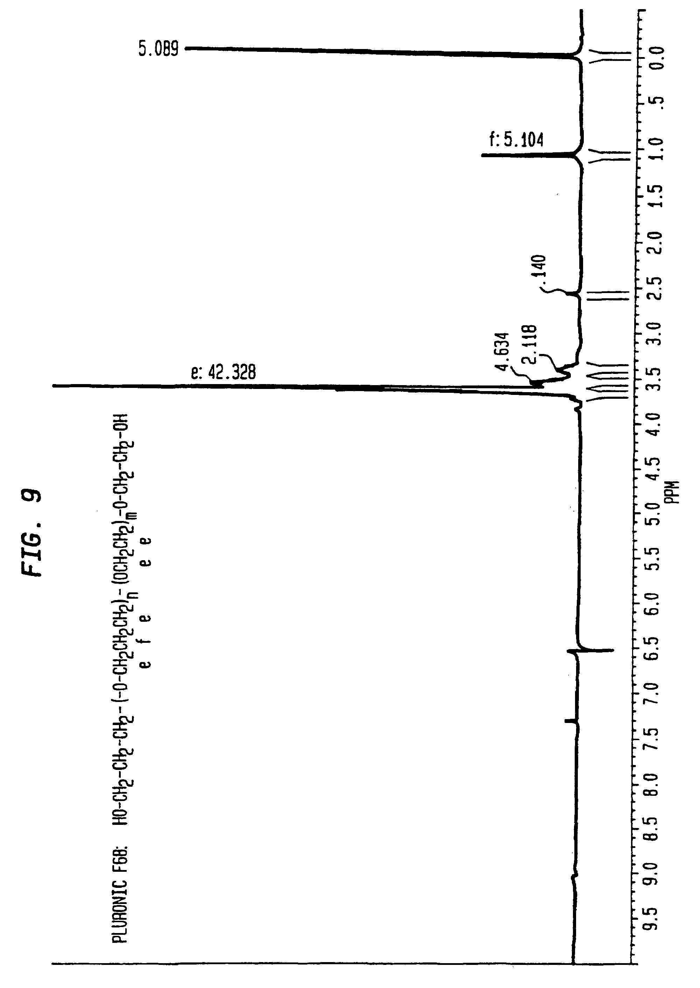

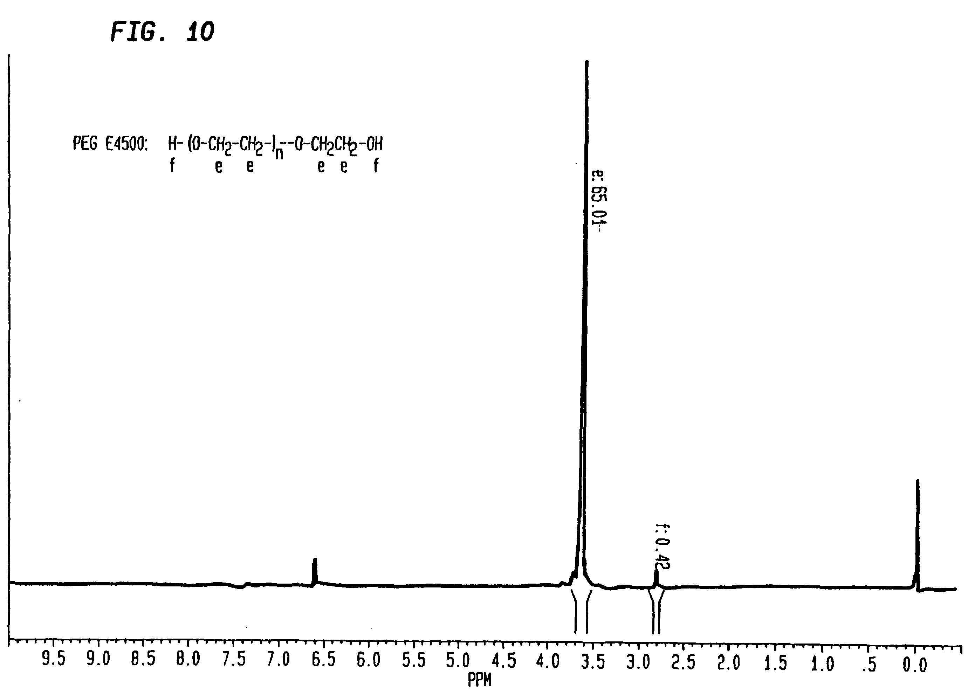

The spectrum of the block copolymer PCL/PEG is shown in Fig. 13. This spectrum shows the same shifts as in Fig. 12 except for proton f which represents the difference between Pluronic F68 and PEG E4500 as shown in the spectra of Figs. 9 and 10. The PCL/PEG block copolymer shown in Fig. 13 had a 75:25 molar ratio of PCL to PEG.

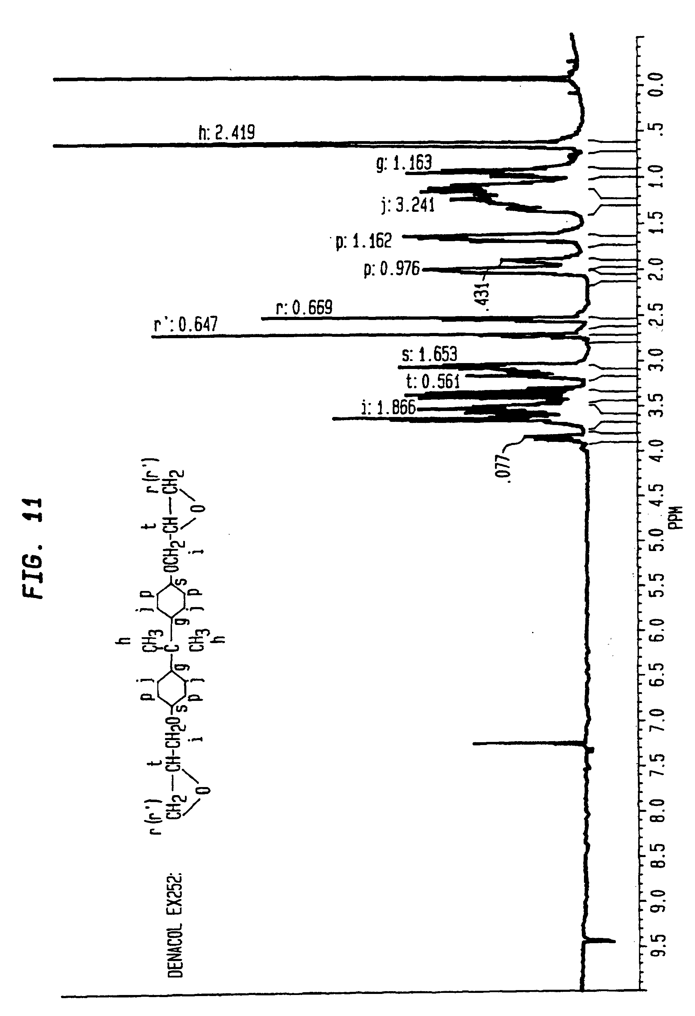

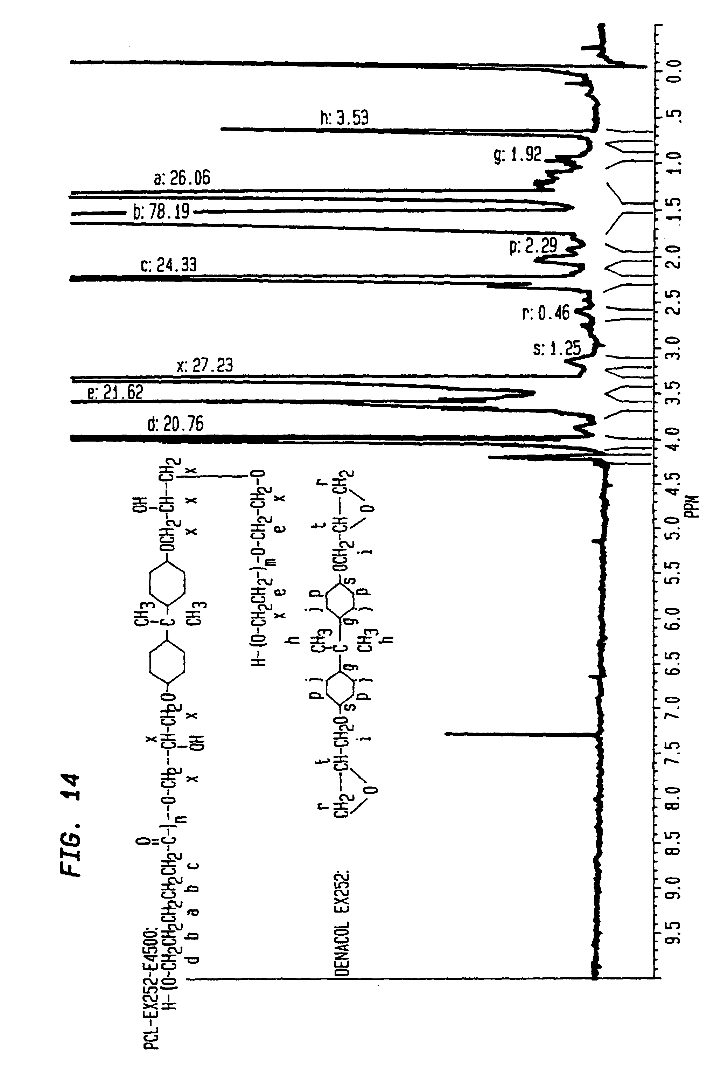

In the spectrum of Fig. 14, the PCL/PEG copolymer had a 60:40 molar ratio of PCL to PEG, and therefore, contained a greater proportion of PEG than the PCL/PEG copolymer shown in Fig. 13. The chemical shifts caused by the protons in Denacol EX252 which are extremely weak due to their relatively very small amounts, were deliberately enlarged. The chemical shift at 0.71 ppm (protons h) represents 6 protons in the -CH₃ groups in Denacol EX252 and peak r at 2.64 ppm is the shift which comes from the two protons of -CH₂ in the epoxide end group in Denacol EX252. After the epoxide reacted with the polymer diols, the intensity of this proton r was greatly reduced. It can be verified by the intensity ratio of h/r. Before reaction, the ratio is 3.6 as shown in Fig. 11. The ratio changed to 7.7 after the formation of the copolymer (Fig. 14). There is a trace amount of unreacted epoxide in the copolymer. This indicates that it is possible that one of the epoxide groups can be reacted with the -OH end groups of the poly-diol while leaving the other epoxide group free so that an epoxide-capped copolymer would be formed if excess Denacol EX252 is used.

### Example 18:

Heparin and albumin were chemically linked with the terminal hydroxyl groups of the block copolymer through use of multi-functional epoxide compounds, illustratively Denacol EX521. In this embodiment, Denacol EX521, with five epoxide groups per molecule, was used as a linking reagent instead of the difunctional Denacol EX252 so that more free epoxide groups would be available for coupling reactions. An excess of Denacol EX521 was reacted with the terminal hydroxyl groups of the polymer particles to form epoxide-capped ends. The coupling of heparin or albumin to the PCL-based polymer particles is the same reaction between the free epoxide group on the polymer ends and amino, hydroxyl, or other functional groups in albumin and heparin molecules, as described hereinabove in the section on epoxy-derivatization.

Triblock ABA and BAB copolymers of PCL and PEG or F68 of the type described in Example 17 were used to make nanoparticles. A specific illustrative preparation scheme is as follows: 100 mg polymer was dissolved in 5 ml methylene chloride and 1 ml acetone. This polymer solution was added, with sonication at 55 Watts of energy output, into 20 ml distilled water. Sonication was continued for a total of 10 minutes to form an oil-in-water emulsion. Organic solvent was evaporated at room temperature with stirring for 16 hours. Nanoparticles were recovered by ultracentrifugation at 145,000 g, resuspended, and lyophilized.

In a specific illustrative embodiment for the surface modification of PCL-based nanoparticles, 50 mg polymer nanoparticles were suspended in 10 ml pH 5.0 borate buffer (0.05 M). An excess of Denacol EX 521 (0.8 g) was dissolved in 5 ml of the same buffer and added into the polymer particle suspension. A catalyst, zinc tetrafluoroborate (Zn(BF₄)₂; 14 mg), was added with stirring. The reaction mixture was shaken at 37° C for 30 minutes. The particles were collected by centrifugation and the excess epoxide compound was removed by washing the separated particles with water. The result was epoxide-capped polymer particles.

The epoxide-capped polymer particles were resuspended in 10 ml borate buffer and 20 mg heparin or albumin was added with stirring. The reaction was permitted to continue for 5 to 10 hours at 37° C. The final product was collected by centrifugation. Free heparin or albumin was removed by washing the nanoparticles three times with water.

In order to measure the amount of heparin or albumin coupled to the polymer particles, radiolabeled heparin (³H-heparin) and albumin (¹⁴C-albumin) were used in the coupling reaction. About 5 mg coupled particles were dissolved in 5 ml of methylene chloride. The organic solution was washed three times with water (7 ml). The concentration of heparin or albumin in the combined aqueous extracts was measured by liquid scintillation counting and the amount of total heparin or albumin in the polymer particles was calculated from a calibration plot.

Table 14 shows the results of coupling albumin (BSA) to various block copolymer particles. The nanoparticles made of the polymer PCUEX252/PCL is the expanded PCL-diol, compound 33 of Fig. 7.

**Table 14**

| Specimen | Amount of BSA (mg) | BSA % (wlw) coupled to polymer | Efficiency of BSA Coupling (%) |
|---|---|---|---|
| PCL/F127/PCL | 1.37 | 15.40 | 38.50 |
| PEG/PCL/PEG | 1.19 | 11.37 | 28.43 |
| PCL/PEG/PCL | 1.25 | 13.17 | 32.43 |
| PEG/PCL/PEG/PCL/PEG | 1.36 | 13.22 | 33.05 |
| PCL/F68/PCL | 0.82 | 6.46 | 16.15 |
| PCL/EX252/PCL | 0.33 | 3.51 | 8.78 |

Referring to Table 14, it is obvious that the amount of albumin coupled to the nanoparticles varies with the hydrophobicity of the polymer. More hydrophilic polymers result in higher coupling. Since the coupling takes place at the end of the polymer molecule, the molecular weight of the polymer would be an important factor in coupling efficiency. The higher the molecular weight, the lower the amount of albumin that can be coupled. A person of ordinary skill in the art, in the practice of the invention, would have to balance the desired molecular weight required for mechanical strength against the biomolecular coupling required for a given application.

For solid dosage forms, *e.g.*, implants, requiring long-term release, a hydrophobic polymer is useful. Hydrophilic polymers are permeable to water or tissue fluid, and will consequently, bioerode more quickly. From the standpoint of making nanoparticles, the hydrophobic/hydrophilic balance should be adjusted so that the polymer can form nanoparticles without an external emulsifier. If the polymer is too hydrophilic in nature, or too hydrophobic, an emulsifier will be required to form nanoparticles. Further, if the polymer is too hydrophilic, it will be difficult to recover. Of course, hydrophilic polymers will entrap more hydrophilic drug and hydrophobic polymers will entrap more hydrophobic drug. A person of ordinary skill in the art can easily control these properties by determining the appropriate number of hydrophobic and hydrophilic segments, as well as their relative positions (*e.g*., BAB or ABA), in the multi-block polymers.

The stability of the albumin-coupled nanoparticles was tested in a diffusion chamber containing phosphate buffer, pH 7.4, at 37° C. Nanoparticles of PCL/F68/PCL made in accordance with Example 18 were suspended in buffer and continuously shaken. Periodically, samples of buffer was removed and replaced with fresh buffer. Radioactivity of the removed buffer samples was measured by liquid scintillation counting. In this manner, the stability of albumin (BSA) coupled to PCL/F68/PCL copolymer was monitored over a 60 day period and compared to a polymer comprising a physical mixture, or dispersion, of BSA with the PCL/F68/PCL nanoparticles. It is to be noted, that the physical mixture of albumin with nanoparticles is not considered to be part of the invention.

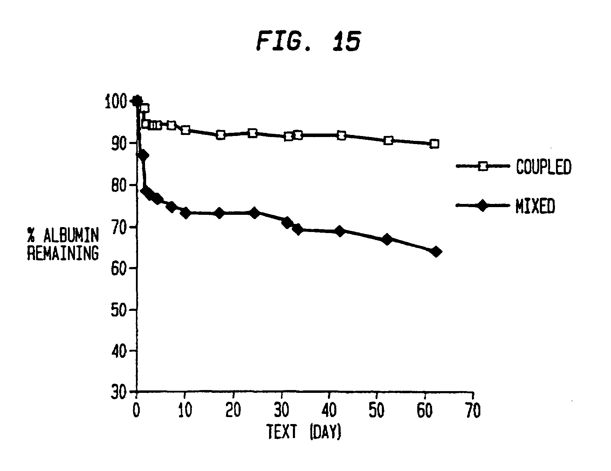

The results are shown in Fig. 15 which is a graphic representation of the percent of albumin remaining in the PCL/F68/PCL nanoparticles as function of time in days. Referring to Fig. 15, the chemically coupled albumin was very stable. More than 90% of the coupled albumin remained after 62 days of incubation. The physically mixed albumin/polymer specimen exhibited faster leakage than the coupled specimen during the first 5 days. The high molecular weight of albumin may impede its diffusion from the polymer particles.

Table 15 shows the results of coupling heparin to various block copolymer particles. Approximately 5% w/w heparin was coupled to particles of each identified copolymer.

**Table 15**

| Specimen | Amount of Heparin (mg) | Heparin % (w/w) coupled to polymer | Efficiency of Heparin Coupling (%) |
|---|---|---|---|
| PEG/PCL/PEG | 0.64 | 5.87 | 14.68 |
| PCL/F68/PCL | 0.51 | 4.95 | 12.38 |
| PCL/EX252/PCL | 0.46 | 5.05 | 12.63 |

Heparin-coupled nanoparticles were subjected to standard APTT testing. No clotting occurred over 200 seconds for dog plasma treated with heparin-coupled nanoparticles, confirming the antithrombogenic effect of the coupled heparin. In comparison, un-heparinized particles clotted within 20-30 seconds.

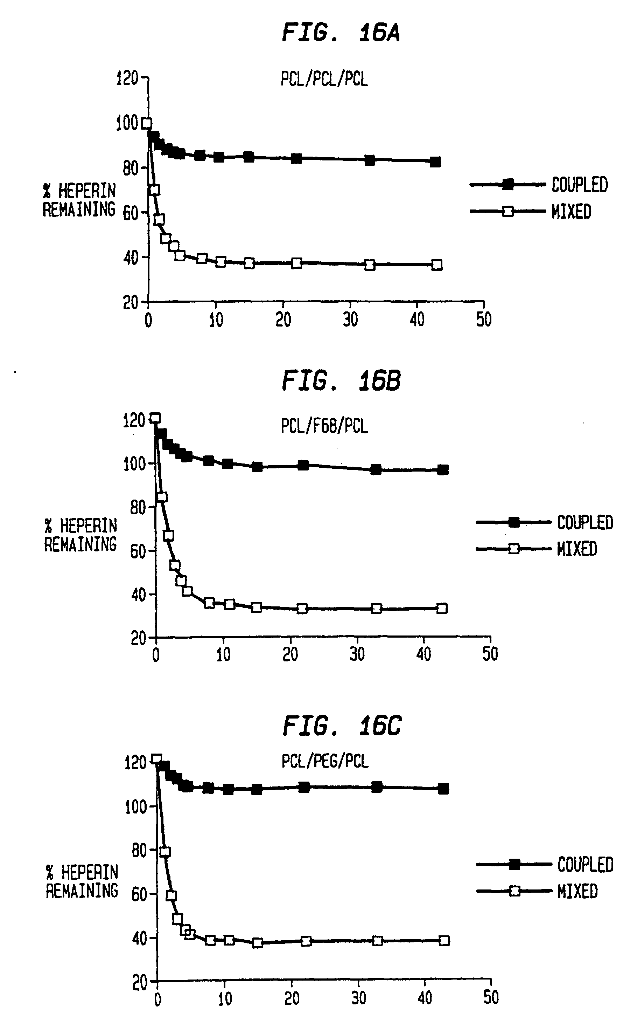

Figs. 16A through 16C are graphical representations of the stability of the heparin-coupled nanoparticles of Table 15 expressed as % bound heparin remaining over time in days. The chemically coupled heparin is substantially more stable than the physically mixed.- About 85% of the heparin remained in the chemically coupled particles after 43 days as compared to 15 % in the physically mixed samples.

### Example 19:

U86 and dexamethasone were incorporated into nanoparticles comprising PCL-based copolymers. The nanoparticles were prepared by the in-solvent emulsification-evaporation technique described above (see, Example 18). However, since the block copolymers contain both hydrophobic and hydrophilic features, a surfactant is not necessarily required to form the initial oil-in-water emulsion.

The PCL-based polymer and hydrophobic drug were dissolved in an organic solvent, methylene chloride. The organic phase was sonicated in an aqueous phase, which in this particular embodiment was a sodium phosphate buffer (pH 8.0), to form an oil-in-water emulsion. The organic solvent was evaporated at room temperature with stirring. The nanoparticles were recovered by ultracentrifugation and dried by lyophilization. The hydroxyl end groups on the block copolymers allowed heparin to couple on the particle surface.

In a specific illustrative embodiment, dexamethasone (35 mg) was dissolved in a combination of 0.5 ml acetone and 0.3 ml ethanol. The drug solution was mixed into a polymer solution (100 mg) dissolved in 5 ml methylene chloride. The organic phase, containing drug and polymer, was emulsified with sonification at 55 Watts of energy output, into 20 ml 1% PVA solution for 10 minutes over an ice bath to form an oil-in-water emulsion. The organic solvent was evaporated at room temperature for 16 hours. The nanoparticles, thus-formed, were recovered by ultracentrifugation, washed three times with water, and lyophilized.

Table 16 shows the mean particle size, drug loading, and heparin coupling to U86-containing nanoparticles. Anti-thrombogenic activity was confirmed by the APTT test which showed no clotting in greater than 200 seconds for the heparinized nanoparticles. The copolymer of F68/PCL/F68 formed the smallest particles due to the long free hydrophilic Pluronic F68 chain on both ends of the copolymer. PCL/PEG/PCL block copolymer also formed small particles.

**Table 16**

| Specimen | U86 loading (w/w) | Heparin Coupling (%) | Particle Size (nm) |
|---|---|---|---|
| F68/PCL/F68 | 12.8 | 3.86 | 131.2 |
| PCL/F68/PCL | 25.2 | 2.67 | 585.8 |
| PCL/PEG/PCL | 16.1 | 4.16 | 168.5 |

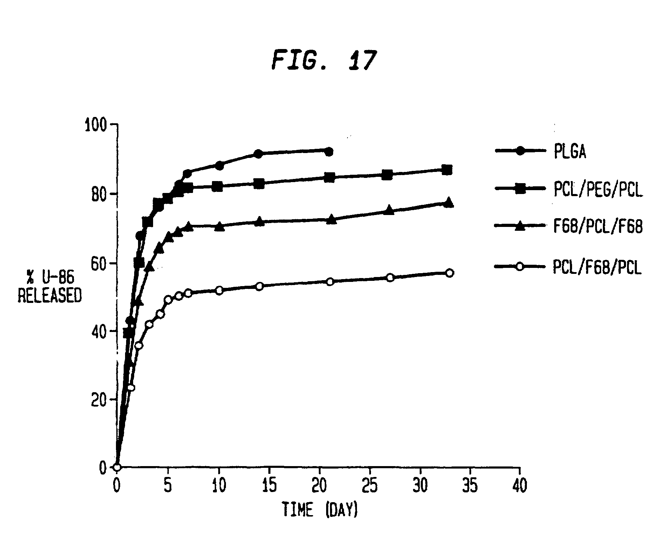

Fig. 17 is a graphical representation of the *in vitro* release of U86 from the heparinized nanoparticles expressed as % U86 released over time in days. Over a 33 day period, about 85% of the incorporated U86 is released from PCL/PEG/PCL, 75% from F68/PCL/F68, and 50% from PCL/F68/PCL. The nanoparticles remained intact after 33 days in the *in vitro* environment. It is hypothesized that release of U86 from the particles in the first 30 days was primarily by diffusion. The remaining U86 will be released more slowly as the polymer degrades. Fig. 17 also shows that PLGA nanoparticles release a greater amount of drug than the PCL-based triblock copolymers.

Dexamethasone-containing nanoparticles were made in accordance with this Example and incorporated into ABA-type copolymers identified on Table 17 below. Since ABA-type copolymers were used in this specific illustrative embodiment, and hence the end segments were hydrophobic, a surfactant, specifically 1% aqueous PVA solution, was employed to emulsify the medium. PCL homopolymer (PCL/EX252/PCL), the expanded PCL-diol which is compound 33 on Fig. 7, was also used to make dexamethasone-containing nanoparticles for comparative purposes.

Table 17 shows the particle size, drug loading and results of standard APTT tests of heparin-coupled, dexamethasone-containing nanoparticles. The PCL/F68/PCL nanoparticles were particularly small. All particles showed good anti-thrombogenic activity.

**Table 17**

| Specimen | Dexamethasone loading (w/w) | Particle Size (nm) | Thrombin Time (sec.) |
|---|---|---|---|
| PCL/PEG/PCL | 33.9 | 117.5 | >200 sec. |
| PCL/F68/PCL | 22.1 | 72.2 | >200 sec. |
| PCL/EX252/PCL | 28.7 | 177.0 | >200 sec. |

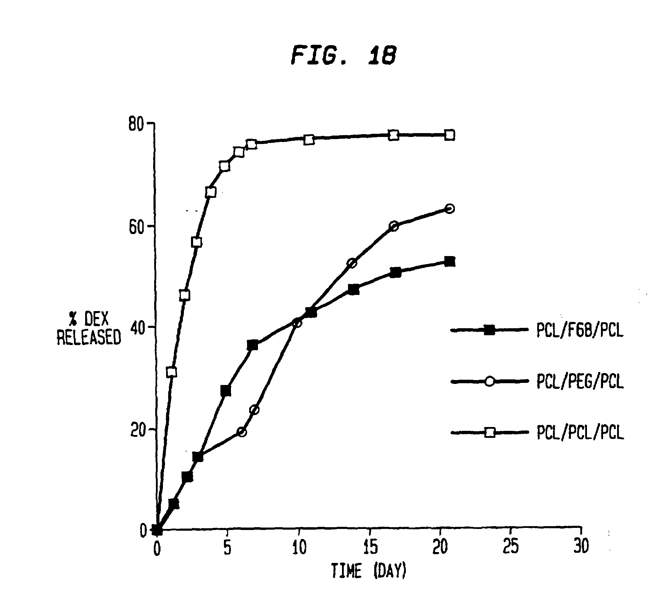

Fig. 18 is a graphical representation of the % dexamethasone released *in vitro* over time, in days, for the nanoparticles described on Table 17. Within 21 days, about 80% of the incorporated dexamethasone was released from PCL/F68/PCL, 65% from PCL/PEG/PCL, and 50% from the PCL homopolymer. Smaller particle size and lower drug loading resulted in quicker release in the first three days as demonstrated by the PCL/F68/PCL nanoparticle. On the other hand, larger particles with higher drug loading demonstrated longer periods of sustained release as shown by the results for the PCL/PEG/PCL and PCL/PCL/PCL nanoparticles.

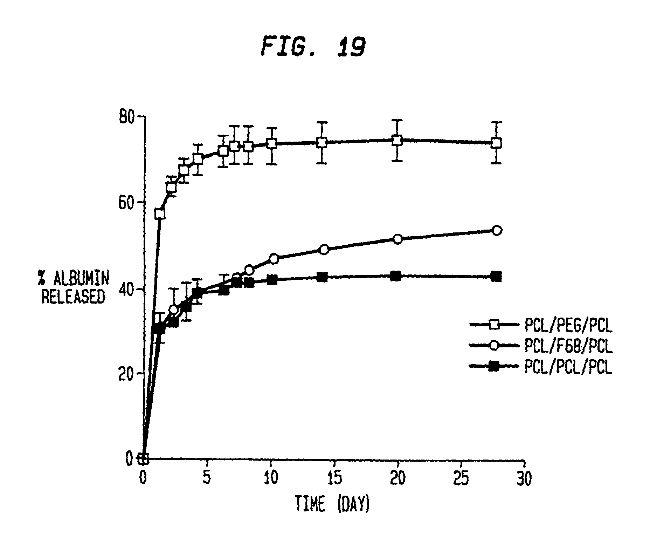

The block copolymers of the present invention can also be used as a matrix carrier for controlled release of biomacromolecules, such as albumin (BSA). Films containing 15% BSA were made from ABA-type block copolymers and PCL homopolymers by hot compression molding at 130° F and 1 ton of pressure. The resulting films of about 150 µm thickness were cut into 1 x 1 cm pieces and shaken in pH 7.4 phosphate buffer at 37° C. The amount of BSA released *in vitro* from the films was monitored by measuring absorbance at 595 nm using a BIO-RAD Protein Assay reagent (Bio-Rad Company, Hercules, CA). The results are shown in Fig. 19 which is a graphical representation of the *in vitro* release of BSA expressed as the % BSA released over time in days. Referring to Fig. 19, it is obvious that the release of albumin from PCL/PEG copolymers is much higher than the release from the PCL homopolymer. This suggests that the release of high molecular weight proteins, which are typically hydrophilic, from a copolymer matrix is positively related to its hydrophobicity.

Contact angle measurements, which relates to the interfacial tension between solid polymer particles and water, were made to assess the hydrophilicity/hydrophobicity of several hydroxy-terminated triblock copolymers of the present invention, PCL/F68/PCL and PCL/PEG/PCL, as a function of molar ratio of hydrophobic to hydrophilic components. The results are shown below in Table 18. If the contact angle is small, the polymer surface is hydrophilic and *vice versa*. Hydrophilicity/hydrophobicity may be an important parameter in the cellular uptake of the formed nanoparticles in practical embodiments, such as treatment or prevention of restenosis and immunization with orally administered vaccines. In the latter case, the uptake of hydrophobic particles, such as polystyrene particles, by the Peyer's patches is greater than the uptake of more hydrophilic particles, such as PLGA particles.

**Table 18**

| **CONTACT DATA** | | |
|---|---|---|
| **PCL/F68/PCL** | | |
| | Molar Ratio of F68 (%) | Contact Angle ± Standard Deviation F68 |
| 1 | 0.000 | 60.220 ± 0.280 |
| 2 | 10.000 | 49.730 ± 1.520 |
| 3 | 33.000 | 34.470 ± 1.360 |
| 4 | 40.000 | 24.330 ± 1.380 |
| 5 | 50.000 | 20.460 ± 1.470 |
| 6 | 58.000 | 16.140 ± 1.020 |

| **PCL/PEG/PCL** | | |
|---|---|---|
| | Molar Ratio of PEG (%) | Contact Angle ± Standard Deviation PEG |
| 1 | 0.000 | 60.220 ± 0.280 |
| 2 | 30.000 | 39.200 ± 1.110 |
| 3 | 50.000 | 30.020 ± 1.900 |
| 4 | 58.000 | 18.550 ± 1.320 |
| 5 | 80.000 | 10.780 ± 1.900 |

The foregoing demonstrates that the PCL block copolymers of the present invention can be formed into nanoparticles, heparin can be covalently bound to the surface to confer anticoagulant activity to the nanoparticles, and proteins andlor peptides can be bound to the surface and released therefrom. Of course, the PCL-based copolymers of the present invention are derivatizable, and can thus, be reacted with a variety of bioactive agents or surface modifiers. In some embodiments, no detergents are necessary for the formation of nanoparticles. Furthermore, the unique formulations permits a far wider range of breakdown duration times than possible with standard PCL. Breakdown times can range from less than an hour to months, and even as much as three years based on reports. See, for example, Darney, *et al*., Fertility and Sterility, Vol. 58, pp. 137-143 (1992); Darney, *et al*., Am. J. Obstet. Gynecol., Vol. 160, pp. 1292-1295 (1989); and Ory. *et al*., Am. J. Obstet. Gynecol., Vol. 145, pp. 600-604 (1983).

In addition to nanoparticles, it should be noted that the novel PCL-based copolymers of the present invention, and methods of making same, are applicable to the manufacture of microparticles, nanoparticles, coatings, and biodegradable monolithic drug depots or polymer matrices and/or devices, such as surgical sutures, catheter tips, urinary catheters, *etc*.

### III. Method of Use Embodiments:

The nanoparticle form is particularly suited for catheter-based local drug delivery at any site which can be accessed through the vasculature, or by other interventional means. Therefore, the nanoparticles of the present invention are contemplated for use in catheter-based delivery systems, particularly in interventional cardiology applications and systems and in the treatment of the vasculature. Active agents for these applications, include, without limitation, dexamethasone, corticosteroids, thrombolytic drugs, calcium channel blockers, anti-platelet action drugs, anti-proliferative agents, such as U86, cytoskeletal inhibitors, DNA, antiinflammatories, and immunosuppressants.

### (1) Prevention of Restenosis

In a specific method of use aspect of the invention, the nanoparticles are useful for local intravascular administration of smooth muscle inhibitors and antithrombogenic agents as part of an interventional cardiac or vascular catheterization procedure, such as a balloon angioplasty. Due to their small size, the nanoparticles may penetrate the arterial wall, for example, and freely enter extracellular spaces.

Nanoparticles are made particularly suitable for intravascular use by co-incorporation of one or more additives to reduce thrombogenicity and enhance extracellular matrix adhesion. The additives specifically contemplated for this purpose include detergents or surfactants such as polyvinyl alcohol, heparin, albumin, cytokines, and various lipids including phospholipids and fatty acids, or a combination thereof. Surface modification with the detergent, DMAB, produced the best results, in terms of retention, as shown in the experimental results reported hereinabove (see Tables 6 and 7). Modifying the surface charge of the nanoparticles, imparting mucoadhesive properties to the nanoparticles, and loading the nanoparticles with albumin further increased efficacy.

Model bioactive agents for this embodiment of the invention include the hydrophobic drugs, U86 and dexamethasone. However, in a specific advantageous embodiment, cytochalasin B was formulated into PLGA nanoparticles in accordance with Example 20 hereinbelow.

For treatment of restenosis of vascular smooth muscle cells, preferred therapeutic agents include protein kinase inhibitors, such as staurosporin or the like, smooth muscle migration and/or contraction inhibitors such as the cytochalasins, suramin, and nitric oxide-releasing compounds, such as nitroglycerin, or analogs or functional equivalents thereof. Cytochalasins are believed to inhibit both migration and contraction of vascular smooth muscle cells by interacting with actin. Specifically, the cytochalasins inhibit the polymerization of monomeric G-actin to polymeric F-actin, which, in turn, inhibits the migration and contraction of vascular smooth muscle cells by inhibiting cell functions requiring cytoplasmic microfilaments. The cytochalasins include mold metabolites exhibiting an inhibitory effect on target cellular metabolism, including prevention of contraction or migration of vascular smooth muscle cells. Cytochalasins are typically derived from phenylalanine, tryptophan, or leucine and are described more particularly in International application WO 94/16707 published on August 4, 1994; WO 94/07529 published on April 14, 1994; and Japanese Patent Nos 72 01,925; 72 14,219; 72 08,533; 72 23,394; 72 01924; and 72 04, 164. The text of the cited publications is incorporated and included herein by reference. Exemplary molecules include cytochalasin A-H and J-S: chaetoglobosin A-G, J, and K; deoxaphomin, proxiphomin, protophomin, zygosporin D-G, aspochalasin B-D and the like, as well as functional equivalents and derivatives. Cytochalasin B is used in this example as a model, and preferred, compound.

While the present example directly applies cytochalasin-bound nanoparticles to vascular tissue, it is to be understood that the invention clearly contemplates the surface modification of the nanoparticles so as to include binding proteins/peptides, such as vascular smooth muscle cell binding proteins, to target the nanoparticles. Vascular smooth muscle binding proteins include antibodies (*e.g*., monoclonal and polyclonal affinity-purified antibodies, F(ab')₂, Fab', Fab, and Fv fragments and/or complementary determining regions (CDR) of antibodies or functional equivalents thereof; growth factors, cytokines, and polypeptide hormones and the like; and macromolecules recognizing extracellular matrix receptors, such as integrin and fibronectin receptors. In addition, binding peptides for targeting the nanoparticles would include binding peptides for intercellular stroma and matrix located between and among vascular smooth muscle cells. These peptides are associated with epitopes on collagen, extracellular glycoproteins, such as tenascin, reticulum and elastic fibers and other intercellular matrix materials.

### Example 20:

150 mg PLGA was dissolved in 5 ml methylene chloride and 15 mg cytochalasin B (Sigma Chemical Co., St. Louis, MO) was dispersed in the polymer solution. Acetone (about 4 ml) was added drop-wise, with stirring, until a clear solution, or organic phase, was formed. The organic phase was emulsified in 20 ml 2.5% PVA solution with sonication to form an oil-in-water emulsion. The oil-in-water emulsion was stirred for 16 hours on a magnetic stir plate to evaporate the organic solvents. The resulting nanoparticles were recovered by ultracentrifugation, washed until free from un-entrapped cytochalasin B and lyophilized for 48 hours. A typical yield for this procedure is about 60%. The nanoparticles have about 7.08% w/w drug loading and an average particle size of 145.4 ± 44.1 nm.

In order to evaluate cellular uptake of cytochalasin B-loaded nanoparticles, a fluorescent dye, coumarin-6, was incorporated into the nanoparticle formulation of Example 20 Specifically, approximately 0.1% by weight coumarin-6 was dissolved into the organic phase prior to emulsification. The uptake of cytochalasin-B and subsequent retention by B054 primate smooth muscle cells (passage #25) in tissue culture. The target cells were plated out in 100 mm plates for 24 hours prior to use at 2.5 x 10⁵ cells/plate (a confluent monolayer for the culture cell). The target cells were exposed to 5 ml/plate cytochalasin B-containing nanoparticles made in accordance with this example (10 µg/ml in complete media) for one hour at 37° C. Then, the monolayer was washed two times with 10 ml complete media, and re-supplied with 10 ml complete media.

The cells were harvested by trypsin/EDTA cell removal, with low speed centrifugation. The cell pellet was resuspended in PBS/2% new born calf serum/0.05% sodium azide. The uptake of nanoparticles into the cells was quantified by two methods: by direct measurement of fluorescence by flow cytometry and by fluorescent spectrophotometric measurement of the extract of coumarin-6 from the cells with ethyl acetate. The results are given below in Table 19. Time "0" was harvest time, measurements were made after 2 hours and 24 hours of incubation at 37° C. The fluorescence data was collected in log scale and converted to linear via control samples. The linear values are reported FE value (fluors intensity).

**Table 19**

| Cellular Fluorescence (flow cytometry) | | | Coumarin in Extracts | |
|---|---|---|---|---|
| Time Posted (hrs) | Fluorescencee FE | % Retention | Coumarin-6 (ng) | % Retention |
| 0 | 871 | 100 | 1.63 | 100 |
| 2 | 255 | 29 | 0.56 | 34 |
| 24 | 145 | 16 | 0.29 | 18 |

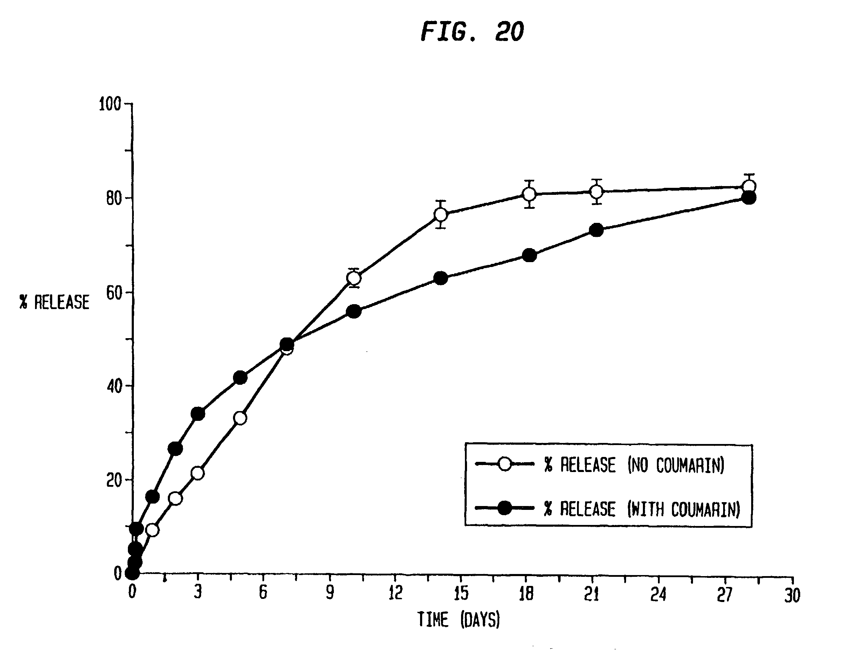

Release of cytochalasin-B was evaluated *in vitro* over a 30 day period in a double diffusion chamber in accordance with the technique described herein, *i.e*., 5 mg nanoparticles per ml physiological phosphate buffer (pH 7.4, 0.154 mM) at 37° C. The result are shown in Fig. 20 which is a graphic representation of the *in vitro* release of cytochalasin-B over time (in days) expressed as the percent of total cytochalasin-B released into the buffer from nanoparticles of the type made in Example 20. A sample of nanoparticles containing the fluorescent dye Coumarin-6 was also tested *in vitro* to ascertain whether the dye affected release of the active agent from the nanoparticles.

The therapeutically effective amount of nanoparticles will depend on several factors, including the binding affinity of any vascular smooth muscle binding protein associated with the nanoparticles, the atmospheric pressure applied during infusion, the time over which the therapeutic agent is applied and resides at the vascular site, the nature of the therapeutic agent employed, the rate of release of the therapeutic agent from the nanoparticles, the nature of the vascular trauma and therapy desired, and the intercellular and/or intracellular localization of the nanoparticles. For intravascular administration, the nanoparticles are suspended in a suspending medium suitable for injection, preferably in a concentration of 0.1 mg/ml or less to 300 mg/ml, and preferably in the range of 5 to 30 mg/ml. This concentration of nanoparticles is in excess of the therapeutically required amount and is still "fluid" for injection. For cytochalasin, a 10⁻³ M to 10⁻¹² M concentration at the site of administration in a blood vessel is preferred.

In a preferred embodiment of the invention, the nanoparticles formed by the methods described hereinabove can be regionally and selectively injected into a target zone with a custom angioplasty catheter developed for this purpose since blood flow must be interrupted during the injection process. Several custom catheters which would be suitable for the purpose are currently in the investigational stage. These are the Wolinsky catheter (C.R. Bard, Inc., Billerica, MA), the Dispatch catheter (Sci-Med, Minneapolis, MN), and the Cordis Arterial Infusion catheter (Cordis Corporation, Miami Lakes, FL). US Patent No. 4,824,436 describes a catheter which has the ability to form a blood-free chamber within the artery into which fluid, such as a solution of heparin, can be delivered under pressure. US Patent No. 5,049,132 describes yet another catheter adapted for delivery of a liquid therapeutic agent. Of course, conventional catheters can be otherwise modified by a person of ordinary skill in the art to discharge the novel drug delivery system to an arterial (or other organ) wall. Further, infusion needles, or any other means of injecting nanoparticles are specifically within the contemplation of the invention.

In a method of use, the nanoparticles are injected under pressure, illustratively 2 to 10 atm, with 3-6 being preferred, to the wall of the vessel preceding, during, or subsequent to the damaging intervention, such as angioplasty. In a preferred embodiment, the nanoparticles include heparin which confers antithrombogenic properties in addition to inhibiting smooth muscle cell proliferation. In addition, surface modification with the detergent DMAB produces excellent results with respect to retention at the site of administration. The nanoparticles adhere to the intramural tissue and slowly degrade to release therapeutic agent which may be smooth muscle inhibitors, including agents that modulate intracellular Ca⁺² and Ca⁺² binding proteins, receptor blockers for contractile agonists, inhibitors of the sodium/hydrogen antiporter, protease inhibitors, nitrovasodilators, phosphodiesterase inhibitors, phenothiazines, growth factor receptor antagonists, anti-mitotic agents, immunosuppressive agents, antisense oligonucleotides, and protein kinase inhibitors.

In an advantageous method aspect, inducing an osmotic shock to the vessel wall with a hypertonic solution prior to, or contemporaneously with, nanoparticle administration further enhances drug entry and extracellular matrix penetration.

Although disclosed in terms of prevention of restenosis following angioplasty, the method of the present invention can be applied to any balloon catheter procedure for such conditions as coronary artery disease, benign prostatic hypertrophy, malignant disorders of various tissues available to tubular access, occlusions in peripheral or cardiac vasculature, clearing and restoring prostatic and other intrusions in the urethra, opening fallopian tubes, and dilating esophageal strictures. Tissue injury and resulting proliferation of smooth muscle cells is often a contributing factor to complications from these procedures. Thus, the treatment of conditions wherein the target tissue or cell population is accessible by local administration, such as by catheter, infusion needle, surgical intervention, or the like, is within the contemplation of the invention.

Specifically included is the treatment of cancer with anticancer agents incorporated into nanoparticles made in accordance with the present invention. Of course, the anti-cancer-laden nanoparticles can be surface modified to target and/or enhance retention at the site. Anti-cancer agents include, but are not limited to, alkylating agents, such as mechlorethamine, cyclophosphamide, ifosfamide, mephalan, chlorambucil, hexamethylmelamine, thiotepa, busulfan, carmustine, lomustin, lomustine, semustine, steptozocin, dacarbazine; antimetabolites, such as methotrexate, fluorouracil, floxuridine, cytarabine, mercaptopurine, thioguanine, pentostatin; natural products, such as alkaloids *(e.g*., vinblastine or vincristine), toxins *(e.g*., etoposide or teniposide), antibiotics (*e.g*., such as dactinomycin, daunorubicin, bleomycin, plicamycin, mitomycin), and enzymes, (*e.g*., L-asparaginase); biological response modifiers, such as Interferon-α; hormones and antagonists, such as adrenocortocoids (*e.g*., dexamethasone), progestins, estrogens, anti-estrogens, androgens, gonadotropin releasing hormone analogs; miscellaneous agents, such as cisplastin, mitoxantrone, hydroxyurea, procarbazine or adrenocortical suppressants (*e.g*., mitotane or aminoglutethimide).

### (2) Sustained Release of Protein/Peptide Vaccine for Immunization

In this embodiment, the nanoparticles can be orally administered in an enteric capsule to be delivered to the gastrointestinal tract which will result in result in uptake by the intestinal mucosa and the Peyer's patch. This embodiment is useful for immunization with protein/peptide based vaccines, but can be adapted to deliver gene therapy to the Peyer's Patch lymphoid tissue.

Conventional methods of immunization generally require multiple injections at certain time intervals to achieve the desired protective immune response. Thus, multiple contacts with health care personnel are necessary. This is associated with a high "drop out" rate and a lack of cost-effectiveness, particularly in developing countries. It would be advantageous to provide an orally administered single dose vaccine immunization system which contains both an adequate priming dose as well as staged booster dose(s). In addition to securing compliance with the dosing schedule, such a dosage form would be less costly, and hence, more competitive. Cost would further be reduced for oral dosage forms which do not require needles, syringes, *etc*.

The sustained release of antigens from nanoparticles, and its subsequent processing by the macrophage and presentation to the immune system, results in an immune response. A single dose oral vaccine using capsule-protected nanoparticles of the type made in accordance with the present invention has been shown to achieve an immune response comparable to that of the conventional method of subcutaneous immunization with alum tetanus-toxoid. The capsule is designed to protect the nanoparticles and encapsulated antigen from gastric enzymes and acidic pH, and to release the enclosed antigen loaded nanoparticles in a burst in the ileum for optimal uptake by the gut-associated lymphoid tissue (and subsequent delivery to the mesenteric lymph nodes) in order to induce an immune response.

The capsule may comprise a protective time-release capsule of the type known in the prior art, and preferably is an osmotically controlled, time-release capsule of the type disclosed in USPN 5,229,895 issued on July 20, 1993, the disclosure of which is incorporated herein by reference. However, any capsule coated with enteric polymers can be used for the purpose. Such enteric polymers include cellulose acetate phthalate, shellac, Eudragit (sold by Rohm Pharmaceutical, Philadelphia, PA), *etc*. that bypass the acidic pH of the stomach and dissolve in the intestine. The time of release of the capsule contents depends upon the number of polymer coats and structure as is known in the art.

In the particular embodiment described herein, the nanoparticles are contained in a PORT™ system capsule (TSRL, Ann Arbor, MI) which is an oral drug delivery system designed to bypass the stomach and release a dose of drug to the gastrointestinal tract at specific times. The design of the PORT™ system is based on controlling the flux of water into a gelatin capsule via a polymer film coating which regulates water flux into the capsule. As the capsule travels down the gastrointestinal tract, pressure builds inside the capsule from the influx of water and forces the contents out in a pulse. The influx of water is regulated by varying the thickness of the polymer film coating on a gelatin capsule wall. The coatings used in this particular embodiment were cellulose acetate which regulates water flux into the capsule and cellulose acetate phthalate which resists stomach acid, but dissolves at intestinal pH. As the amount of coating applied to capsule is increased, the permeability and water flux decreases. The decrease in water flux decreases the rate of pressure build-up within the capsule, thereby prolonging the time of the pulse. The pulse times can illustratively range from 4 to 9 hours for film coatings of 4 to 11%.

In addition to containment in a controlled-release capsule, the nanoparticles can be adapted to have staged, variable breakdown periods to achieve priming and booster doses. Formulation of a biodegradable polymeric non-antigen-containing sealing coat(s) which delays hydrolysis of the biodegradable polymer, and surface modification as described herein, are several of the techniques which may be used to vary the breakdown rate.

Although the following example is directed to the use of nanoparticles for the delivery of tetanus-toxoid vaccine as the model protein-based vaccine, it is to be understood that the system may be useful for delivery of other vaccines, or combinations of vaccines, to achieve long-term protective immune responses against any vaccine-preventable disease. Illustrative examples are bacterial vaccines such as tetanus, cholera toxin, hepatitis B, acellular pertussis, Staphylococcus enterotoxin B, pneumococcus, Staphylococcus and Streptococcus antigens, and others, including combined diphtheria, pertussis, and tetanus (DPT); E. Coli (enteropathogenic); and viral vaccine proteins, such as all AIDS antigens, viral proteins (*e.g*., influenza virus proteins, adenovirus, and others); live virus in microcapsules (e.g., attenuated poliovirus), Hepatitis viral components, Rotavirus components.

Orally administered controlled release nanoparticles can induce a secretory immune response (IgA) in addition to a systemic immune response (IgG). This would be particularly useful for the prevention of respiratory, vaginal, and gut-associated mucosal infectious diseases.

### Example 21:

Tetanus-toxoid (provided by the Serum Institute, Pune, India ) was loaded into PLGA in the water-in-oil-in-water emulsification technique of Example 10 hereinabove. The technique produced a 57% entrapment efficiency with 12% antigen loading. Particle size distribution studies revealed a uniform particle distribution with a mean particle diameter of 154.3 ∓ 82.7 nm. The *in vitro* release rate of tetanus toxoid from the PLGA nanoparticles into a phosphate buffered saline at 37° C approximates first order kinetics.

More particularly, tetanus toxoid and a viscosity enhancer, Pluronic F-125 (BASF, Parsippany, NJ), are dissolved in water. PLGA (50:50, molecular weight 90,000, inherent viscosity, 1.07; Birmingham Polymers, Inc, Birmingham, Alabama) is dissolved in methylene chloride (3% w/v). The tetanus toxoid solution and the PLGA solutions are sonicated to form a water-in-oil primary emulsion. The primary emulsion is then emulsified into an aqueous solution of PVA (2.5% w/v) to form a water-in-oil-in-water emulsion. The organic solvent is then evaporated, the nanoparticles are recovered by ultracentrifugation, washed three times with water, resuspended in water and lyophilized.

### Example 22:

Nanoparticles incorporating BSA and 0.05% Rhodamine dye were administered to a group of rats (male, Sprague-Dawley, 230-250 mg) in order to detect their presence in the intestinal mucosa and Peyer's patch lymphoid tissue. The nanoparticles used in this study had a particle size of 150 ∓ 48.5 nm. Fluorescent microscopy revealed significant uptake of the nanoparticles in the Peyer's Patch lymphoid tissue.

### Example 23:

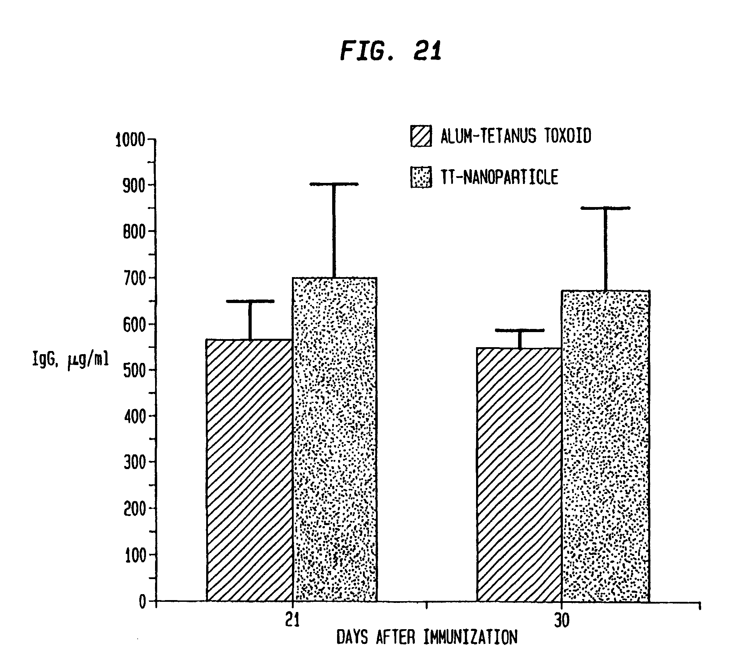

The use of the nanoparticles of the present invention as a drug delivery device for vaccines has been demonstrated by studies in rats. Tetanus Toxoid loaded nanoparticles (15 *Lf*) were prepared and subcutaneously injected in rats. The immune response, as measured by IgG, µg/ml, was compared to the immune response in rats to which conventional Alum-Tetanus Toxoid conjugate (Pasteur-Merieux through US supplier, Connaught Laboratories, Inc., Swiftwater, PA; 5 *Lf*) had been subcutaneously administered. The results are shown on Fig. 21 which is a graphic representation of the immune response, as measure by IgG, µg/ml, at 21 days post-immunization and 30 days post-immunization.

The immune response in the short-term was virtually identical. The nanoparticles continue to release Tetanus Toxoid for 30 days, thus prolonging the sensitization-exposure period and enhancing the long term immune response. Further, the results demonstrated that the immunogenicity of Tetanus Toxoid is not adversely affected by the nanoparticle formulation procedures.

As demonstrated above, the nanoparticles of the present invention can be adapted for oral administration, as well as intravascular or subcutaneous administration, for sustained release of drugs or vaccines or used as an immunological adjuvant for immunization. Vaccines, as well as gene therapy for the paraintestinal lymphoid system, can be orally administered.

In addition to the foregoing, nanoparticles suitable for vaccination can be administered via the following routes: intramuscular, subcutaneous, oral, nasal, intraperitoneal, rectal, and vaginal.

### (3) Gene Therapy

The nanoparticles can be used to deliver genetic material in a targeted manner. In this application, the nanoparticles can be formulated for administration via the oral route or the mucous membrane. The nanoparticles are capable of sustained administration of gene therapy, particularly to the lymphoid system surrounding the ileum as described hereinabove.

However, nanoparticles containing genetic material can also be devised and targeted for site-specific delivery to other cells or tissue types by injection and/or implantation. Also specifically contemplated are genetic material suitable for the DNA or anti-sense treatment of cardiovascular disease, including platelet-derived growth factor, transforming growth factors, alpha and beta, fibroblast growth factors (acidic and basic), angiotensin II, heparin-binding epidermal growth factor-like molecules, Interleukin-1, alpha and beta, Interleukin-6, insulin-like growth factors, oncogenes (c-myb, c-myo, fos, and others), proliferating cell nuclear antigen, cell adhesion molecules (intracellular adhesion molecules, vascular cell adhesion molecules, and others), and platelet surface antigens (IIb /IIIa and others).

In another illustrative embodiment, the nanoparticles of the present invention may be used as a carrier for nucleic acids, such as an osteotropic gene or gene segment. The nanoparticles have the capability of transferring nucleic acids into bone cells and tissues for promoting bone growth and regeneration. In one specific embodiment, an osteotropic gene or gene segment is transferred into bone progenitor cells to stimulate progenitor cells and promote increased bone formation. The DNA-carrying nanoparticles may be injected to the site, which may be bone or skeletal connective tissues, such as tendons, cartilage, and ligaments. Specific examples include bone morphogenic proteins (BMP2 and 4 and others), transforming growth factor, such as TGF-β1-3, activin, phosphoproteins, osteonectin, osteopontin, bone sialoprotein, osteoalcin and other vitamin-k dependent proteins, glycoproteins, such as aggrecan, glycan, and others, and collagen (I, II, and others). Further specific examples are described in copending US patent application numbers 08/199,780 filed on February 18, 1994 and. 08/316,650 filed on September 30, 1994, assigned to the assignee hereof, the disclosures of which are incorporated by reference herein.

Regulatory factors involved in bone repair are known to include systemic hormones, cytokines, growth factors, and other molecules that regulate growth and differentiation. Various osteoinductive agents have been purified and shown to be polypeptide growth-factor-like molecules. These stimulatory factors are referred to as bone morphogenetic or morphogenic proteins (BMPs), and have also been termed osteogenic bone inductive proteins or osteogenic proteins (OPs). Several BMP genes have now been cloned and the common designations are BMP-1 through BMP-8.

BMPs 2-8 are generally thought to be osteogenic, although BMP-1 is a more generalized morphogen (Shimell, *et al*., 1991). BMP-3 is also called osteogenin (Luyten, *et al*., 1989) and BMP-7 is also called OP-1 (Ozkaynak, *et al*., 1990). BMPs are related to, or part of, the transforming growth factor-β (TGF-β) superfamily, and both TGF-β1 and TGF-β2 also regulates osteoblast function (Seitz, *et al*., 1992. Several BMP (or OP) nucleotide sequences and polypeptides have been described in US Patents, *e.g.*, 4,795,804; 4,877,864; 4,968,590; 5,108,753; including specifically BMP-1 disclosed in 5,108,922; BMP-2A in 5,166,058 and 5,103,649; BMP-2B in 5.013, 649; BMP-3 in 5,116,738; BMP-5 in 5,106,748; BMP-6 in 5,187,076; BMP-7 in 5,108,753, and 5,141,905; and OP-1, COP-5 and COP-7 in 5,011,691. In addition, an article by Wozny, *et al*. is incorporated herein by reference to describe BMP molecular clones and their activities. The cited literature, including the patent literature specifically, also teaches how to prepare an osteotropic gene segment or cDNA.

Other growth factors or hormones that have been reported to have capacity to stimulate new bone formation include acidic fibroblast growth factor, estrogen, macrophage colony stimulating factor, and calcium regulatory agents such as parathyroid hormone. The use of bone stimulating proteins and polypeptides, particularly recombinant BMPs, has also been investigated.

In the instant invention, nucleic acid segments are transferred into bone progenitor cells or tissues at the site *in vivo*. The nucleic acid segment may be DNA (double or single-stranded) or RNA (*e.g*., mRNA, tRNA, rRNA); it may be a "coding segment", and antisense nucleic acid molecule. Thus, the nucleic acid segments may be genomic sequences, including exons and introns alone or together, or coding cDNA regions, or in fact any construct that one desires to transfer to a bone progenitor cell and virtually any form, such as naked DNA or RNA, including linear nucleic acid molecules and plasmids, or as a functional insert within the genomes of various recombinant viruses, including viruses with DNA genomes and retroviruses.

The invention may be employed to promote expression of a desired gene in bone cells or tissues and to impart a particular desired phenotype to the cells. This expression could be increased expression of a gene that is normally expressed , or it could be used to express a gene that is not normally associated with bone progenitor cells in their natural environment. Alternatively, the invention may be used to suppress the expression of a gene that is naturally expressed in such cells and tissues, and again, to change or alter the phenotype. Gene suppression may be a way of expressing a gene that encodes a protein that exerts a down-regulatory function, or it may utilize antisense technology.

An osteotropic gene is a gene or DNA coding region that encodes a protein, polypeptide, or peptide that is capable of promoting, or assisting in the promotion of, bone formation, or one that increase the rate of primary bone growth or healing. In addition, an osteotropic gene may be capable of stimulating the growth or regeneration of skeletal connective tissues, such as tendon, cartilage, and ligament. Bone progenitor cells refer to any or all of those cells that have the capacity to ultimately form, or contribute to the formation of, new bone tissue. They specifically include various cells in different stages of differentiation, such as stem cell, macrophages, fibroblasts, vascular cells, osteoblast, chondroblasts, osteoclasts, and the like. Osteotropic genes and the proteins that they encode include, for example, systemic hormones, such as parathyroid hormone (PTH) and estrogen; many different growth factors and cytokines; chemotactic or adhesive peptides or polypeptides; molecules such as activin (US Patent No. 5,208,219, incorporated herein by reference), specific bone morphogenetic proteins (BMPs); and growth factor receptor genes.

Examples of suitable osteotropic growth factors include the transforming growth factor (TGF) family, specifically TGFs 1-4, and particularly TGF-α, TGF-β1, TGF-β2 (US Patent Nos. 5,168,051; 4,886,747; and 4,742,033, each incorporated herein by reference); and fibroblast growth factors (FGF), such as acidic FGF and kFGF; granulocyte/macrophage colony stimulating factors (GMCSF); epidermal growth factor (EGF); platelet derived growth factor (PDGF); insulin-like growth factors (IGF), including IGF-I and IGF-II; and leukemia inhibitory factor (LIF), also known as HILDA and DIA.

Preferred osteotropic genes and DNA segments are those of the TGF superfamily, such as TGF-α, TGF-β1, TGF-β2, and the members of the BMP family of genes. Of course, the original source of a recombinant gene or DNA segment need not be of the same species as the animal to be treated. In this regard, it is contemplated that any recombinant PTH, TGF, or BMP gene may be employed, such as those from human, mouse, and bovine sources. Gene and DNA segment refer to a DNA molecule that has been isolated free of total genomic DNA of the species from which it was obtained. Included within the term DNA segment are DNA segments and smaller fragments of such segments, and also recombinant vectors, including for example, plasmids, cosmids, phage, retroviruses, adenoviruses, and the like.

The nanoparticles of the present invention may comprise one or more osteotropic genes or nucleic acid segments, in combination, or in combination with other proteins, peptides, or pharmaceutically active agents, andlor surface modifying agents.

### Example 24:

In a specific embodiment illustrating use of nanoparticles of the present invention for delivery of DNA, or DNA fragments, luciferase marker DNA was incorporated into PLGA nanoparticles in accordance with the principles of the invention.

COS cells (mouse kidney epithelium) were transfected *in vitro* using the pGL2 plasmid expression vector which encodes luciferase. A standard transfection protocol was used. In brief, COS cells plated the day before were exposed to DNA (luciferase, Promega, Los Angeles, CA) for 2.5 hours in a standard cell culture medium, which medium lacked serum. The cells were washed and then cultured in a medium with 10% serum supplementation for 60 additional hours.

In an experiment designed to evaluate the sustained release of DNA, 20 mg of plasmid DNA was complexed with DEAE-dextran and compared to DNA enclosed in PLGA nanoparticles at a concentration of either 10 µg/ml or 20 µg/ml.

In order to make nanoparticles, PLGA (90 mg) was dissolved in 3 ml chloroform. Nuclease-free BSA (30 mg) and DNA (2 mg) were dissolved in 300 µl nuclease-free Tris - EDTA which is Tris buffer (Tris(hydroxymethyl)aminomethane; 10 mM, pH 7.4) containing 0.1 mM EDTA. The DNA-containing solution was emulsified with the PLGA polymer solution by sonication over an ice bath for 8 minutes using a microtip probe sonicator at 55 Watts of energy output. The resulting water-in-oil emulsion was further emulsified into 25 ml of 2% w/v PVA (M Wt. 30-70 K) solution in Tris-EDTA buffer saturated with chloroform using the sonicator probe at 55 Watts. The result was a water-in-oil-water emulsion. The water-in-oil-water emulsion was stirred for 18 hours with a magnetic stirrer in an open container, and then for 2 additional hours under vacuum to completely evaporate the organic solvent. Nanoparticles, thus formed, were recovered by ultracentrifugation, washed three times with Tris-EDTA buffer, and lyophilized for 48 hours. The resulting nanoparticles were stored desiccated.

Since DNA is water-soluble, it is entrapped by the emulsion procedure and distributed throughout the polymer matrix. Targeting of DNA-containing nanoparticles can be accomplished as described hereinabove with appropriate surface modifying agents, such as ferritin, antibodies which are specific to target cells, marker proteins for receptors on target cells, or the provision of a characteristic lipid coating, among others.

It should be noted specifically, that the Tris-EDTA buffer used in this specific illustrative embodiment, has antinuclease properties which prevent DNA breakdown during processing. In addition to Tris-EDTA, any other buffer or combination of buffers containing a calcium complexing or chelating agent, such as dithizone, nitrolotriacetic acid, citrates, oxalates, tartrates, and dimercaprol, is suitable for use in the practice of the invention. Calcium is a necessary cofactor in the breakdown of DNA with nucleases, therefore calcium complexing agents which competitively remove calcium ions mitigate against the loss of DNA by this mechanism. In addition to the use of calcium complexing buffers, certain proteins, such as histones, protamine or polylysine, bind nuclease and thereby block its damaging effect on the DNA. It is also advantageous to conduct the entire nanoparticle production procedure in a nuclease-free environment, such as by using nuclease-free reagents, such as nuclease-free serum albumin (available from Sigma Chemical Co., St. Louis, MO).

Luciferase activity of the nanoparticles was determined by a substrate utilization assay using a commercially available kit (Luciferase Assay System, Promega, Los Angeles, CA) substantially according to the protocol supplied by the manufacturer. In brief, cells were homogenized in 2 ml buffer (50 mM Tris acetate, pH 7.4, 1 mM EDTA, 1 mM dithiothreitol, 10% glycerol, 1 mg/ml BSA). Immediately thereafter, 0.5 ml of cell culture lysis reagent (Promega) was added to the homogenate, mixed well, and incubated at room temperature for 10 minutes. To measure background activity (counts per minute, CPM), 100 µl of clarified homogenate was added to a clean microcentrifuge tube and luciferase activity was determined by scintillation counting for 1 minute at room temperature (1219 RackBeta Scintillation counter, LKB, supplied through Wallace, Inc., Gaithersburg, MD, all channels open). The same procedure was used to measure background CPM of 1 ml luciferase substrate stock solution. Once background activity was determined, homogenate and substrate were mixed and counted immediately. Enzyme activity values were normalized to 1 µl of total protein.

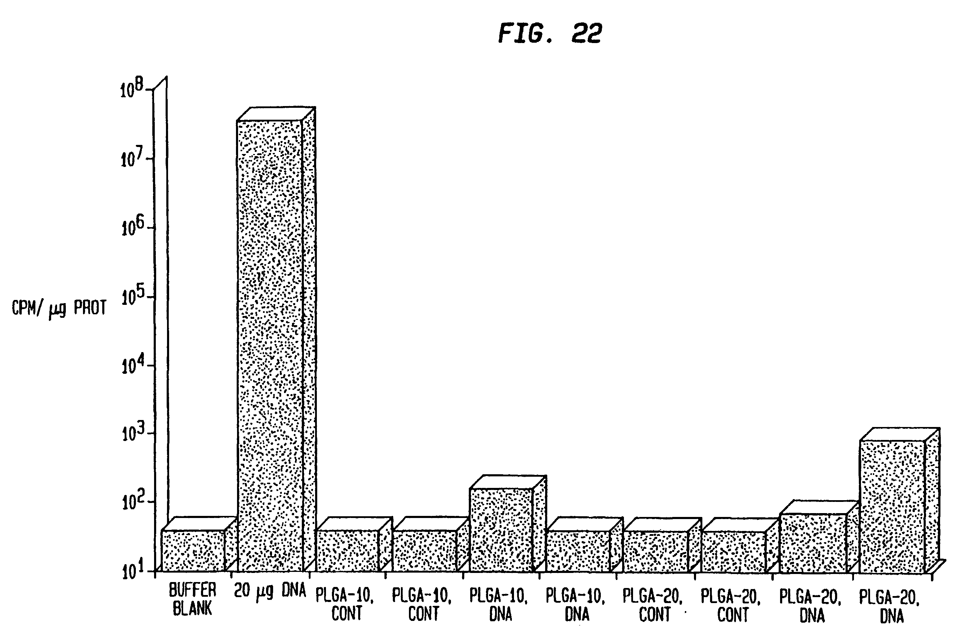

The results are shown graphically in Fig. 22 which is a plot of luciferase activity as CPM/µg protein for each specimen. The total amount of DNA contained in each batch of nanoparticles was considerably less than the control comparison for this experiment. Thus, the group designated PLGA-10 DNA contained 40 ng of DNA total and the group designated PLGA-20 DNA contained 80 ng of DNA. Furthermore, the sustained release of all the DNA from the nanoparticles would have actually occurred after 30 days as shown in the *in vitro* release studies conducted with the model protein, BSA. Thus, the 2.5 hours exposure to the nanoparticles constitutes a severe test of the efficacy of the nanoparticles since only minute amounts of DNA were released. Nevertheless, Fig. 22 shows significant expression above background of luciferase in three of the four DNA-containing nanoparticle groups.

In a still further illustrative embodiment of the invention, a method of making nanoparticles has been developed which does not use sonification. It has been discovered that sonication may damage genetic material. The damaging effect is magnified with larger genes. Therefore, a technique has been developed using an excess of organic solvents, such as DMSO or chloroform, and a detergent to obtain nanoparticles without the use of sonification.

### Example 25:

In a specific illustrative embodiment, DNA (luciferase, 2 mg) and nuclease-free BSA (30 mg) are dissolved in 300 µl Tris-EDTA buffer to form an aqueous phase. The aqueous phase is homogenized into a PLGA polymer solution dissolved in chloroform (90 mg PLGA in 3 ml chloroform) containing 1% w/v Span-20 to form a water-in-oil emulsion. The primary emulsion is further emulsified by homogenization for 30 minutes into a 2% w/v solution of PVA in nuclease-free Tris-EDTA buffer which has been saturated with chloroform. The result is a multiple emulsion, or a water-in-oil-in-water emulsion. The organic solvent is evaporated at room temperature by stirring, uncovered, over a magnetic stirring plate for 18 hours. Then, a vacuum is applied for an additional 2 hours. The resulting nanoparticles are recovered by ultracentrifugation, washed three times with Tris-EDTA buffer and lyophilized.

Nanoparticles which include osteotropic genes and/or other materials to stimulate bone growth, may be advantageously suspending in a gelling medium which is applied to the site of need. The nanoparticles, which may be in a gelling medium, may also be intimately mixed with another material, specifically a bone filler, such as bone cement, dental adhesives, hydroxyapatite, and bone ceramics, to hold the nanoparticles at the site of application.

Although the invention has been disclosed in terms of biodegradable polymers, in the specific embodiment directed to therapy to facilitate bone growth, nanoparticles which are, at least in part, insoluble and non-degradable are contemplated. Such nanoparticles could contain insoluble calcium phosphate crystalline mineral components, for example, to render them osteoconductive, *i.e*., capable of facilitating new mineral formation. Such insoluble nanoparticles would be integrated into the renewed bone structure. Specifically included are all calcium phosphate mineral phases, including octacalcium phosphate, amorphous calcium phosphate, tricalcium phosphate, carbonate-apatite, and fluorapatites, as well as ceramics of all of the aforementioned. Further, calcium bisphosphonates, or other crystalline salts or free acids or mono-, bis- or polyphosphonates, would be useful as fillers and surface modifying agents. Synergistic combinations include ferric or aluminum salts of bisphosphonates.

In addition to the foregoing, it is to be understood that the nanoparticles of the present invention would find widespread application in the delivery of bioactive agents in general. The purpose of the delivery of bioactive agents may range from therapeutic to diagnostic (imaging agents), to cosmetic or nutritional. Nanoparticle-based delivery of gene therapy is expected to improve transfection of DNA over a prolonged period of time.

Although the invention has been described in terms of specific embodiments and applications, persons skilled in the art can, in light of this teaching, generate additional embodiments without exceeding the scope or departing from the spirit of the claimed invention. Accordingly, it is to be understood that the drawing and description in this disclosure are proffered to facilitate comprehension of the invention, and should not be construed to limit the scope thereof.

## Claims

1. A nanoparticle having an average diameter of less than 300 nm and comprising a biocompatible, biodegradable polymer core, at least one bioactive agent, and at least one multifunctional epoxide linking compound covalently linked to the biodegradable polymer.

2. A nanoparticle according to claim 1, wherein the biodegradable polymer is a synthetic polymer.

3. A nanoparticle according to claim 2, wherein the synthetic polymer is selected from polyesters, polyethers, polyanhydrides, polyalkylcyanoacrylates, polyacrylamides, poly(orthoesters), polyphosphazenes, polyamino acids, biodegradable polyurethanes, and copolymers thereof.

4. A nanoparticle according to claim 2, wherein the biodegradable polymer is a hydroxy- or epoxy-terminated multiblock copolymer comprising at least one hydrophobic segment and at least one hydrophilic segment, the hydrophobic and hydrophilic segments being connected by multifunctional epoxide linking compounds, and having a molecular weight of 6,000 to 100,000 as measured by gel permeation chromatography and/or intrinsic viscosity.

5. A nanoparticle according to any one of claims 1 to 4, wherein the multifunctional epoxide linking compound is a polyfunctional polyglycerol polyglycidyl ether.

6. A nanoparticle according to claim 4 or claim 5, wherein the hydrophobic segment is selected from polycaprolactones, polylactides, polyglycolides, polylactic-polyglycolic acid copolymers, biodegradable polyurethanes, polyanhydrides, and polyamino acids.

7. A nanoparticle according to any one of claims 4 to 6, wherein the hydrophilic segment is a polyether selected from polyethylene glycol, polaxomers, and poly(propylene oxide).

8. A nanoparticle according to any one of claims 4 to 7, wherein the multiblock copolymer is selected from ABA, BAB, multiblock (AB)ₙ and (BA)ₙ type polymers, and combinations thereof, wherein the A block is polycaprolactone and the B block is selected from polyethylene glycol, poloxamers, and poly(propylene oxide).

9. A nanoparticle according to claim 8, wherein the multiblock copolymer is selected from HO-PEG-EX252-PCL-EX252-PCL-EX252-PEG-OH; HO-F68-EX252-PCL-EX252-PCL-F68-OH; HO-PCL-EX252-F68-EX252-PCL-OH; HO-PCL-EX252-PEG-EX252-PCL-OH; and HO-PCL-EX252-PPO-EX252-PCL-OH.

10. A nanoparticle according to claim 2, wherein the biodegradable polymer is a polylactic polyglycolic copolymer having a molecular weight of 30,000 to 700,000 and an intrinsic viscosity of 0.5 to 10.5.

11. A nanoparticle according to any one of claims 4 to 9, further comprising a bioactive agent and/or surface modifying agent covalently attached to the multifunctional epoxide compound.

12. A nanoparticle according to claims 1, wherein the biodegradable polymer is a naturally-derived polymer.

13. A nanoparticle according to any one of claims 1 to 12, wherein the bioactive agent is at least one pharmaceutical agent.

14. A nanoparticle according to claim 13, wherein the pharmaceutical agent is selected from cardiovascular agents, anticancer agents, peptide or protein-based vaccines and nucleic acids.

15. A nanoparticle according to claim 14, wherein the cardiovascular agent is selected from stimulators, inhibitors, antithrombins, calcium channel blockers, antitensin converting enzyme (ACE) inhibitors, immunosuppressants, fish oils, growth factor antagonists, cytoskeletal inhibitors, antiinflammatory agents, thrombolytic agents, antiproliferatives, genetic material suitable for the DNA or anti-sense treatment of cardiovascular disease, protein kinase inhibitors, smooth muscle migration and/or contraction inhibitors, and nitric oxide-releasing compounds.

16. A nanoparticle according to claim 15, wherein the cadiovascular agent is cytochalasin B.

17. A nanoparticle according to claim 14, wherein the anticancer agent is selected from alkylating agents, antimetabolites, natural products (*e.g*., alkaloids), toxins, antibiotics, enzymes, biological response modifiers, hormones, antagonists, and genetic material suitable for the treatment of cancer.

18. A nanoparticle according to claim 14, wherein the nucleic acid is an osteotropic gene or gene segment, or oligonucleotide.

19. A nanoparticle according to claim 18, wherein the osteotropic gene or gene segment is selected from bone morphogenic proteins (BMP2 and 4 and others), transforming growth factor, such as TGF-β1-3, activin, phosphoproteins, osteonectin, osteopontin, bone sialoprotein, osteocalcin, vitamin-k dependent proteins, glycoproteins, and collagen (at least I and II).

20. A nanoparticle according to claim 18 or claim 19, further including a protein to block nuclease activity.

21. A nanoparticle according to any one of claims 1 to 20, wherein the surface modifying agent is selected from synthetic polymers, biopolymers, low molecular weight oligomers, natural products, surfactants, and multifunctional epoxide compounds.

22. A nanoparticle according to claim 21, wherein the synthetic polymer is selected from carboxymethyl cellulose, cellulose, cellulose acetate, cellulose phthalate. polyethylene glycol, polyvinyl alcohol. hydroxypropylmethyl cellulose phthalate. hydroxypropyl cellulose, sodium or calcium salts of carboxymethyl cellulose, noncrystalline cellulose, polaxomers, poloxamines, dextrans, DEAE-dextran, polyvinyl pyrolidone, polystyrene, and silicates.

23. A nanoparticle according to claim 21, wherein the natural product is selected from proteins, peptides, sugar-containing compounds, lipids, acacia, gelatin, casein, heparin, albumins, myoglobins, hemoglobins, fibrinogens, tragacanth, sorbitol, mannitol, polysaccharides, pectin, lecithin, phospholipids, cholesterol, beeswax, wool fat, sulfonated oils, and resin soap.

24. A nanoparticle according to claim 21, wherein the surfactant is selected from non-ionic, anionic, and cationic surfactants, including polyoxyethylene sorbitan fatty acid esters, sorbitan fatty acid esters, fatty alcohols, alkyl aryl polyether sulfonates, and dicetyl ester of sodium sulfonsuccinic acid;
sodium dodecyl sulfate, sodium and potassium salts of fatty acids, polyoxyl stearate, polyyoxylethylene lauryl ether, sorbitan sesquioleate, triethanolamine, fatty acids, and glycerol esters of fatty acids; and didodecyldimethyl ammonium bromide, cetyl trimethyl ammonium bromide, benzalkonium chloride, hexadecyl trimethyl ammonium chloride, dimethyldodecylaminopropane and N-cetyl-N-ethyl morpholinium ethosufate.

25. A nanoparticle according to any one of claims 1 to 24, further including a suspending medium.

26. A nanoparticle according to any one of claims 1 to 25, further including an encapsulation.

27. A method of making a nanoparticle according to claim 1 containing a hydrophobic bioactive agent the method comprising the steps of:
(a) dissolving biocompatible, biodegradable polymer in an organic solvent;
(b) dissolving the bioactive agent in an organic solvent, the combined polymer and bioactive agent-containing solutions comprising an organic phase;
(c) adding the organic phase to an aqueous phase;
(d) sonicating the combined organic phase and aqueous phase at a temperature below the melting point of the polymer and at an energy sufficient to form a stable emulsion;
(e) evaporating the organic solvent from the stable emulsion;
(f) separating resulting nanoparticles from the remaining aqueous phase; and
(g) modifying the surface of the nanoparticle by contacting the nanoparticle, if necessary or desired after partially hydrolyzing the surface thereof, with a reactive multifunctional epoxide thereby to form an epoxy-activated nanoparticle.

28. A method of making a nanoparticle according to claim 1 containing a hydrophilic bioactive agent, the method comprising the steps of:
(a) dissolving a biodegradable, biocompatible polymer in a nonpolar organic solvent;
(b) dissolving the hydrophilic bioactive agent in a semipolar organic solvent or a combination of polar and semipolar organic solvents, the combined polymer and bioactive agent-containing solutions comprising an organic phase;
(c) adding the organic phase to an aqueous phase;
(d) sonicating the combined organic phase and aqueous phase at a temperature below the melting point of the polymer and at an energy sufficient to form a stable emulsion;
(e) evaporating the organic solvent from the stable emulsion;
(f) separating resulting nanoparticles, the remaining aqueous phase; and
(g) modifying the surface of the nanoparticle by contacting the nanoparticle, if necessary or desired after partially hydrolyzing the surface thereof, with a reactive multifunctional epoxide thereby to form an epoxy-activated nanoparticle.

29. A method according to claim 28, wherein the organic phase further includes an agent to favor partitioning of the hydrophilic bioactive agent into the organic phase upon solidification of the resulting nanoparticles.

30. A method according to any one of claims 27 to 29, wherein the aqueous phase is an aqueous solution of an emulsifying agent.

31. A method of making a nanoparticle according to claim 1 containing a water-soluble protein/peptide-containing bioactive agent, the method comprising the steps of:
(a) dissolving the water-soluble protein/peptide-containing bioactive agent in an aqueous solution to form a first aqueous phase;
(b) dissolving at least one biocompatible, biodegradable polymer in a nonpolar organic solvent to form an organic phase;
(c) admixing the first aqueous phase with the organic phase to form a primary emulsion;
(d) emulsifying the primary emulsion into a second aqueous phase to form a water in oil in water emulsion;
(e) evaporating the organic solvent from the water in oil in water emulsion;
(f) separating the resulting nanoparticles from the remaining aqueous phase; and
(g) modifying the surface of the nanoparticles by contacting the nanoparticles, if necessary or desired after partially hydrolyzing the surface thereof, with a reactive multifunctional epoxide thereby to form an epoxy-activated nanoparticle.

32. A method according to claim 31, wherein step (c) includes sonicating with energy sufficient to form a stable primary emulsion.

33. A method according to claim 31 or claim 32, wherein the second aqueous phase is an aqueous solution of an emulsifying agent.

34. A method according to claim 33, wherein the aqueous solution contains 1 to 3% w/v emulsifying agent.

35. A method of making ultrasmall nanoparticles having an average diameter of 10-50 nm according to claim 1, the method comprising the steps of:
(a) dissolving at least one biocompatible, biodegradable polymer and at least one bicactive agent in a co-solvent comprising a nonpolar organic solvent and a semi-polar organic solvent to form a first organic phase;
(b) adding the first organic phase to an aqueous phase containing at least one emulsifying agent to form an emulsion;
(c) sonicating the emulsion with a sonicator probe at an energy output of about 65 Watts at a temperature below the melting point of the biocompatible, biodegradable polymer to yield a stable emulsion;
(d) recovering the nanoparticles; and
(e) modifying the surface of the nanoparticles by contacting the nanoparticles, if necessary or desired after partially hydrolyzing the surface thereof, with a reactive multifunctional epoxide thereby to form an epoxy-activated nanoparticle.

36. A method of making ultrasmall nanoparticles having an average diameter of 10-50 nm according to claim 1, the method comprising the steps of:
(a) dissolving a water-soluble bioactive agent in an aqueous solution to form a first aqueous phase;
(b) dissolving at least one biocompatible, biodegradable polymer in a co-solvent comprising a nonpolar organic solvent and a semi-polar organic solvent to form a first organic phase;
(c) adding the first aqueous phase to the first organic phase to form a primary emulsion;
(d) adding the primary emulsion into a second aqueous solution to form a water-in-oil-in-water emulsion;
(c) sonicating the water-in-oil-in-water emulsion with a sonicator probe at an energy output of about 65 Watts at a temperature below the melting point of the biocompatible, biodegradable polymer to yield a stable emulsion;
(d) recovering the nanoparticles; and
(e) modifying the surface of the nanoparticles by contacting the nanoparticles; if necessary or desired after partially hydrolyzing the surface thereof, with a reactive multifunctional epoxide thereby to form an epoxy-activated nanoparticle.

37. A method according to any one of claims 27 to 36, wherein the reactive multifunctional epoxide is selected from butane- and ethane-di-glycidyl ethers, erythritol anhydride, polyfunctional polyglycerol polyglycidyl ethers, and epichlorhydrin.

38. A method according to any one of claims 27 to 37, comprising the further step of reacting the epoxy-activated nanoparticles with reactrve groups on one or more bioactive agents and/or surface modifying agents to form epoxy-derivatized nanoparticles.

39. A method of making a nanoparticle according to claim 1, the method comprising the steps of:
(a) preparing an emulsion comprising at least one biocompatible, biodegradable polymer, at least one bioactive agent, and at least one organic solvent and water;
(b) sonicating the emulsion at a temperature below the melting point of the biocompatible, biodegradable polymer and at an energy sufficient to form a stable emulsion;
(c) evaporating the organic solvent to yield nanoparticles; and
(d) contacting the nanoparticles with a multifunctional epoxide compound under conditions wherein the multifunctional epoxide compound reacts with the biocompatible, biodegradable polymer to yield epoxide activated nanoparticles.

40. A method according to claim 39, further comprising the step of contacting the epoxide activated nanoparticles with at least one bioactive agent and/or surface modifying agent under conditions wherein the epoxide activated nanoparticles react with the bioactive agent or surface modifying agent to form epoxy derivatized nanoparticles.

41. A method according to claim 39 or claim 40, wherein the emulsion further comprises at least one emulsifying agent.

42. A method according to claim 41, wherein the nanoparticles are partially hydrolyzed to create reactive groups on the biocompatible, biodegradable polymer prior to the step of contacting the nanoparticles with the multifunctional epoxide compound.

43. A multiblock copolymer having hydrophobic and hydrophilic segments connected by epoxy linkages and being hydroxy-terminated or epoxide-terminated and having a molecular weight between about 6,000 to 100,000 as measured by gel permeation chromatography and intrinsic viscosity.

44. A multiblock copolymer according to claim 43, wherein the hydrophobic segment is selected from polycaprolactone, polylactides, polyglycolides, polylactic-polyglycolic acid copolymers, biodegradable polyurethanes, polyanhydrides, and polyamino acids.

45. A multiblock copolymer according to claim 43 or claim 44, wherein the hydrophilic segment is a polyether selected from polyethylene glycol, polaxomers, and poly(propylene oxide).

46. A multiblock copolymer according to any one of claims 43 to 45, which are ABA, BAB, multiblock (AB)ₙ or (BA) ₙtype, and combinations thereof, wherein the A block is polycaprolactone and the B block is a polyether selected from polyethylene glycol, poloxamers, and poly(propylene oxide).

47. A multiblock copolymer according to any one of claims 43 to 45, wherein a hydrophobic segment and/or the hydrophilic segment is expanded, so that multiple molecules are linked together by epoxy linkages.

48. A method of making block copolymers having hydrophilic and hydrophobic segments, the method comprising the steps of:
(a) dissolving a first polymer-diol in an organic solvent;
(b) adding a multifunctional epoxide in excess to the dissolved first polymer-diol so that one of the epoxide groups of the multifunctional epoxide reacts with hydroxyl groups en the ends of the first polymer-diol to form an epoxide end-capped first polymer (block A); and
(c) adding an excess of a second polymer-diol (block B) to the epoxide end-capped first polymer block A to form a hydroxyl-terminated BAB-type triblock copolymer.

49. A method according to claim 48, wherein there is provided the further step(s) of expanding the molecular weight of a polymer-diol, prior to use in steps (a) and/or (b), by reacting an excess of the polymer-diol with a polyfunctional epoxide.

50. A method according to claim 48 or claim 49, wherein steps (b) and (c) are repeated to form multiblock copolymers.

51. A method according to any one of claims 48 to 51, comprising the further step of reacting the BAB-type triblock copolymer or multiblock polymer with a multifunctional epoxide to form an epoxide end-capped polymer.

52. A method according to claim 51, comprising the further step of reacting the epoxide end-capped polymer with a bioactive agent and/or surface modifying agent having at least one functional group thereon which reacts with epoxide groups to covalently attach the bioactive agent and/or surface modifying agent to the epoxide end-capped polymer.

53. A method according to any one of claims 48 to 52, comprising the further step of washing or reacting the polymer to block further epoxide reactivity.

54. A method according to any one of claims 48 to 53, wherein the multifunctional epoxide is selected from butane- and ethane-di-glycidyl ethers, erythritol anhydride, polyfunctional polyglycerol polyglycidyl ethers, and epichlorhydrin.

55. A method according to any one of claims 48 to 54, wherein the first polymer-diol is a hydrophobic polymer-diol and the second polymer-diol is a hydrophilic polymer-diol.

56. A method according to any one of claims 48 to 54, wherein the first polymer-diol is a hydrophilic polymer-diol and the second polymer-diol is a hydrophobic polymer-diol.

57. A method according to claim 55 or claim 56, wherein the hydrophobic polymer-diol is selected from polycaprolactones, polylactides, polyglycolides, and polylactic-polyglycolic acid copolymer.

58. A method according to any one of claims 55 to 57, wherein the hydrophilic polymer-diol is selected from polyethylene glycol, polaxomers, and poly(propylene oxide).

59. A method of modifying the surface of a polymer of the type having a reactive end group, the method comprising the steps of:
contacting the polymer with a multifunctional epoxide compound in the presence of a catalyst to form an epoxide-coupled polymer, and
reacting the epoxide-coupled polymer with a bioactive agent and/or surface modifying agent having at least one functional group thereon which reacts with epoxide groups to covalently link the bioactive agent and/or surface modifying agent to the polymer.

## Patentansprüche

1. Nanopartikel, welches einen mittleren Durchmesser von weniger als 300 nm aufweist, und einen biokompatiblen, bioabbaubaren Polymerkern, mindestens ein bioaktives Mittel und mindestens eine Epoxid-Kopplungsverbindung, welche kovalent mit dem bioabbaubaren Polymer verbunden ist, umfasst.

2. Nanopartikel nach Anspruch 1, wobei das bioabbaubare Polymer ein synthetisches Polymer ist.

3. Nanopartikel nach Anspruch 2, wobei das synthetisches Polymer aus Polyestern, Polyethern, Polyanhydriden, Polyalkylcyanoacrylaten, Polyacrylamiden, Poly(orthoestern), Polyphosphazenes, Polyaminosäuren, bioabbaubaren Polyurethanen und Copolymeren hiervon ausgewählt ist.

4. Nanopartikel nach Anspruch 2, wobei das bioabbaubare Polymer ein Hydroxy- oder Epoxy-terminiertes Multiblock-Copolymer ist, welches mindestens ein hydrophobes Segment und mindestens ein hydrophiles Segment umfasst, wobei die hydrophoben und hydrophilen Segmente durch multifunktionelle Epoxid-Kopplungsverbindungen verbunden sind und welches ein mittels Gelpermeationschromatographie und/oder innere Viskosität bestimmtes Molekulargewicht von 6.000 bis 100.000 aufweist.

5. Nanopartikel nach einem der Ansprüche 1 bis 4, wobei die multifunktionelle Epoxid-Kopplungsverbindung ein polyfunktioneller Polyglycerol-Polyglycidylether ist.

6. Nanopartikel nach Anspruch 4 oder 5, wobei das hydrophobe Segment aus Polycaprolactonen, Polylactiden, Polyglycoliden, Polymilchsäure-Polyglycolsäure-Copolymeren, bioabbaubaren Polyurethanen, Polyanhydriden und Polyaminosäuren ausgewählt ist.

7. Nanopartikel nach einem der Ansprüche 4 bis 6, wobei das hydrophile Segment ein Polyether ist, der aus Polyethylenglycol, Poloxameren und Poly(propylenoxid) ausgewählt ist.

8. Nanopartikel nach einem der Ansprüche 4 bis 7, wobei das Multiblock-Copolymer aus ABA, BAB, Multiblock (AB)ₐ und (BA)ₙ-Typ-Polymeren sowie Kombination hiervon ausgewählt ist, wobei der A-Block Polycaprolacton ist und der B-Block aus Polyethylenglycol, Poloxameren und Poly(propylenoxid) ausgewählt ist.

9. Nanopartikel nach Anspruch 8, wobei das Multiblock-Copolymer aus HO-PEG-EX252-PCL-EX252-PCL-EX252-PEG-OH, HO-F68-EX252-PCL-EX252-PCL-F68-OH, HO-PCL-EX252-F68-EX252-PCL-OH, HO-PCL-EX252-PEG-EX252-PCL-OH und HO-PCL-EX252-PPO-EX252-PCL-OH ausgewählt ist.

10. Nanopartikel nach Anspruch 2, wobei das bioabbaubare Polymer ein Polymilchsäure-Polyglycol-Copolymer mit einem Molekulargewicht von 30.000 bis 700.000 und einer inneren Viskosität von 0,5 bis 10,5 ist.

11. Nanopartikel nach einem der Ansprüche 4 bis 9, welches weiterhin ein bioaktives Mittel und/oder ein mit der multifunktionellen Epoxidverbindung kovalent verbundenes oberflächenmodifizierendes Mittel umfasst.

12. Nanopartikel nach Anspruch 1, wobei das bioabbaubare Polymer ein natürlich abgeleitetes Polymer ist.

13. Nanopartikel nach einem der Ansprüche 1 bis 12, wobei das bioaktive Mittel mindestens ein pharmazeutisches Mittel ist.

14. Nanopartikel nach Anspruch 13, wobei das pharmazeutische Mittel aus Herzkreislaufmitteln, Antikrebsmitteln, Vakzinen auf Peptid- oder Protein-Basis und Nucleinsäuren ausgewählt ist.

15. Nanopartikel nach Anspruch 14, wobei die Herzkreislaufmittel aus Stimulatoren, Inhibitoren, Antithrombinen, Calciumkanalblockern, Antitensin-umwandelndes Enzym (ACE)-Inhibitoren, Immunsuppressiva, Fischölen, Wachstumsfaktor-Antagonisten, Cytoskelett-Inhibitoren, Antientzündungsmitteln, Thrombolysemitteln, Antiproliferativa, allgemein Material, das für die DNA- oder Antisense-Behandlung von Herzkreislauferkrankungen geeignet ist, Proteinkinase-Inhibitoren, Inhibitoren der Migration und/oder Kontraktion glatter Muskeln und Stickstoffoxidfreisetzenden Verbindungen ausgewählt ist.

16. Nanopartikel nach Anspruch 15, wobei das Herzkreislaufmittel Cytochalasin B ist.

17. Nanopartikel nach Anspruch 14, wobei das Antikrebsmittel aus Alkylierungsmitteln, Antimetaboliten, natürlichen Produkten (z. B. Alkaloiden), Toxinen, Antibiotika, Enzymen, Modifikatoren der biologischen Antwort, Hormonen, Antagonisten und genetischem Material, das für die Behandlung von Krebs geeignet ist, ausgewählt ist.

18. Nanopartikel nach Anspruch 14, wobei die Nucleinsäure ein osteotropes Gen oder Gensegment oder Oligonucleotid ist.

19. Nanopartikel nach Anspruch 18, wobei das osteotrope Gen oder Gensegment aus morphogenen Knochenproteinen (BMP2 und 4 und andere), einem transformierenden Wachstumsfaktor wie TGF-β1-3, Activin, Phosphoproteinen, Osteonectin, Osteopontin, Knochen-Sialoprotein, Osteocalcin, Vitamin-Kabhängigen Proteinen, Glykoproteinen und Collagen (zumindest I und II) ausgewählt ist.

20. Nanopartikel nach Anspruch 18 oder 19, welches weiterhin ein Protein zum Blockieren einer Nucleaseaktivität enthält.

21. Nanopartikel nach einem der Ansprüche 1 bis 20, wobei das oberflächenmodifizierende Mittel aus synthetischen Polymeren, Biopolymeren, Oligomeren mit niedrigem Molekulargewicht, natürlichen Produkten, oberflächenaktiven Mitteln und multifunktionellen Epoxidverbindungen ausgewählt ist.

22. Nanopartikel nach Anspruch 21, wobei das synthetische Polymer aus Carboxymethylcellulose, Cellulose, Celluloseacetat, Cellulosephthalat, Polyethlenglycol, Polyvinylalkohol, Hydroxypropylmethylcellulosephthalat, Hydroypropylcellulose, Natrium- oder Calciumsalzen von Carboxymethylcellulose, nichtkristalliner Cellulose, Poloxameren, Poloxaminen, Dextranen, DEAE-Dextran, Polyvinylpyrrolidon, Polystyrol und Silicaten ausgewählt ist.

23. Nanopartikel nach Anspruch 21, wobei das natürliche Produkt aus Proteinen, Peptiden, Zucker-enthaltenden Verbindungen, Lipiden, Acacia, Gelatine, Casein, Heparin, Albuminen, Myoglobinen, Hemoglobinen, Fibrinogenen, Tragant, Sorbitol, Mannitol, Polysacchariden, Pectin, Lecithin, Phospholipiden, Cholesterol, Bienenwachs, Wollfett, sulfonierten Ölen und Harzseife ausgewählt ist.

24. Nanopartikel nach Anspruch 21, wobei das oberflächenaktive Mittel aus nichtionischen, anionischen und kationischen oberflächenaktiven Mitteln ausgewählt ist, einschließlich Polyoxyethylen-Sorbitanfettsäureestern, Sorbitanfettsäureestern, Fettalkoholen, Alkylaryl-Polyethersulfonaten und Diethylester der Natriumsulfonsuccinsäure; Natriumdodecylsulfat, Natrium- und Caliumsalzen von Fettsäuren, Polyoxylstearat, Polyoxyethylenlaurylether, Sorbitansesquioleat, Triethanolaminen, Fettsäuren und Glycerolestern von Fettsäuren; und Didodecyldimethylammoniumbromid, Cetyltrimethylammoniumbromid, Benzalkoniumchlorid, Hexadecyltrimethylammoniumchlorid, Dimethyldodecylaminopropan und N-Cetyl-N-ethylmorpholiniumethosulfat.

25. Nanopartikel nach einem der Ansprüche 1 bis 24, welches weiterhin ein Suspensionsmedium enthält.

26. Nanopartikel nach einem der Ansprüche 1 bis 25, welches weiterhin eine Umhüllung aufweist.

27. Verfahren zur Herstellung eines Nanopartikels nach Anspruch 1, welches ein hydrophobes bioaktives Mittelenthält, wobei das Verfahren die Schritte aufweist:
(a) Lösen eines biokompatiblen, bioabbaubaren Polymers in einem organischen Lösungsmittel;
(b) Lösen des bioaktiven Mittels in einem organischen Lösungsmittel, wobei die vereinigten Lösungen, die das Polymer und das bioaktive Mittel enthalten, eine organische Phase umfassen;
(c) Zugabe der organischen Phase zu einer wässerigen Phase;
(d) Beschallen der vereinigten organischen Phase und wässerigen Phase bei einer Temperatur unterhalb des Schmelzpunkts des Polymers und bei einer Energie, die zur Bildung einer stabilen Emulsion ausreicht;
(e) Verdampfen des organischen Lösungsmittels aus der stabilen Emulsion;
(f) Abtrennen sich ergebender Nanopartikel von der verbleibenden wässerigen Phase; und
(g) Modifizieren der Oberfläche des Nanopartikels durch Kontaktieren des Nanopartikels, wenn notwendig oder erwünscht nach teilweiser Hydrolyse der Oberfläche hiervon, mit einem reaktiven multifunktionellen Epoxid, um so ein Epoxyaktiviertes Nanopartikel zu bilden.

28. Verfahren zur Herstellung eines Nanopartikels nach Anspruch 1, welches ein hydrophiles inaktives Mittel enthält, wobei das Verfahren die Schritte aufweist:
(a) Lösen eines bioabbaubaren, biokompatiblen Polymers in einem nichtpolaren organischen Lösungsmittel;
(b) Lösen des hydrophilen bioaktiven Mittels in einem semipolaren organischen Lösungsmittel oder einer Kombination eines polaren und semipolaren organischen Lösungsmittels, wobei die vereinigten Lösungen, die das Polymer und das bioaktive Mittel enthalten, eine organische Phase umfassen;
(c) Zugabe der organischen Phase zu einer wässerigen Phase;
(d) Beschallen der vereinigten organischen Phase und wässerigen Phase bei einer Temperatur unterhalb des Schmelzpunkts des Polymer und bei einer Energie, die zur Bildung einer stabilen Emulsion ausreicht;
(e) Verdampfen des organischen Lösungsmittels aus der stabilen Emulsion;
(f) Abtrennen sich ergebender Nanopartikel von der verbleibenden wässerigen Phase; und
(g) Modifizieren der Oberfläche des Nanopartikels durch Kontaktieren des Nanopartikels, wenn notwendig oder erwünscht nach teilweiser Hydrolyse der Oberfläche hiervon, mit einem reaktiven multifunktionellen Epoxid, um so ein Epoxyaktiviertes Nanoteilchen zu bilden.

29. Verfahren nach Anspruch 28, wobei die organische Phase weiterhin ein Mittel enthält, um die Abtrennung des hydrophilen bioaktiven Mittels in die organische Phase bei der Verfestigung der sich ergebenden Nanopartikel zu begünstigen.

30. Verfahren nach einem der Ansprüche 27 bis 29, wobei die wässerige Phase eine wässerige Lösung eines Emulgiermittels ist.

31. Verfahren zur Herstellung eines Nanopartikels nach Anspruch 1, welches ein wasserlösliches Protein/Peptidenthaltendes bioaktives Mittel enthält, wobei das Verfahren die Schritte aufweist:
(a) Lösen des wasserlöslichen Protein/Peptidenthaltenden bioaktiven Mittels in einer wässerigen Lösung um eine erste wässerige Phase zu bilden;
(b) Lösen mindestens eines biokompatiblen, bioabbaubaren Polymers in einem nichtpolaren organischen Lösungsmittel, um eine organische Phase zu bilden;
(c) Mischen der ersten wässerigen Phase mit der organischen Phase, um eine Primäremulsion zu binden;
(d) Emulgieren der Primäremulsion in eine zweite wässerige Phase, um eine Wasser-in-Öl-in-Wasser-Emulsion zu bilden;
(e) Verdampfen des organischen Lösungsmittels aus der Wasser-in-Öl-in-Wasser-Emulsion;
(f) Abtrennen sich ergebender Nanopartikel von der verbleibenden wässerigen Phase; und
(g) Modifizieren der Oberfläche der Nanopartikel durch Kontaktieren der Nanopartikel, wenn notwendig oder erwünscht nach teilweiser Hydrolyse der Oberfläche hiervon, mit einem reaktiven multifunktionellen Epoxid, um so ein Epoxyaktiviertes Nanopartikel zu bilden.

32. Verfahren nach Anspruch 31, wobei der Schritt (c) das Beschallen mit Energie beinhaltet, die zur Bildung einer stabilen Primäremulsion ausreicht.

33. Verfahren nach Anspruch 31 oder 32, wobei die zweite wässerige Phase eine wässerige Lösung eines Emulgiermittels ist.

34. Verfahren nach Anspruch 33, wobei die wässerige Lösung 1 bis 3% Gew./Vol. Emulgiermittel enthält.

35. Verfahren zur Herstellung ultrakleiner Nanopartikel mit einem mittleren Durchmesser von 10-50 nm gemäß Anspruch 1, wobei das Verfahren die Schritte aufweist:
(a) Lösen mindestens eines biokompatiblen, bioabbaubaren Polymers und mindestens eines bioaktiven Mittels in einem Co-Lösungsmittel, welches ein nichtpolares organisches Lösungsmittel und ein semipolares organisches Lösungsmittel umfasst, um eine erste organische Phase zu bilden;
(b) Zugabe der ersten organischen Phase zu einer wässrigen Phase, welche mindestens ein Emulgiermittelenthält, um eine Emulsion zu bilden;
(c) Beschallen der Emulsion mit einer Beschallungssonde bei einer Energieabgabe von ungefähr 65 Watt bei einer Temperatur unterhalb des Schmelzpunkts des biokompatiblen, bioabbaubaren Polymers, um eine stabile Emulsion zu erhalten;
(d) Rückgewinnen der Nanopartikel; und
(e) Modifizieren der Oberfläche der Nanopartikel durch Kontaktieren der Nanopartikel, wenn notwendig oder erwünscht nach teilweiser Hydrolyse der Oberfläche hiervon, mit einem reaktiven multifunktionellen Epoxid, um so ein Epoxyaktiviertes Nanoteilchen zu bilden.

36. Verfahren zur Herstellung ultrakleiner Nanopartikel mit einem mittleren Durchmesser von 10-50 nm gemäß Anspruch 1, wobei das Verfahren die Schritte aufweist:
(a) Lösen eines wasserlöslichen bioaktiven Mittels in einer wässerigen Lösung, um eine erste wässerige Phase zu bilden,
(b) Lösen mindestens eines biokompatiblen, bioabbaubaren Polymers in einem Co-Lösungsmittel, welches ein nichtpolares organisches Lösungsmittel und ein semipolares organisches Lösungsmittel umfasst, um eine erste organische Phase zu bilden;
(c) Zugabe der ersten wässerigen Phase zu der ersten organischen Phase, um eine Primäremulsion zu bilden;
(d) Zugabe der Primäremulsion in eine zweite wässerige Lösung, um eine Wasser-in-Öl-in-Wasser-Emulsion zu bilden;
(e) Beschallen der Wasser-in-Öl-in-Wasser-Emulsion mit einer Beschallungssonde bei einer Energieabgabe von ungefähr 65 Watt bei einer Temperatur unterhalb des Schmelzpunkts des biokompatiblen, bioabbaubaren Polymers, um eine stabile Emulsion zu erhalten;
(f) Rückgewinnen der Nanopartikel; und
(g) Modifizieren der Oberfläche der Nanopartikel durch Kontaktieren der Nanopartikel, wenn notwendig oder erwünscht nach teilweiser Hydrolyse der Oberfläche hiervon, mit einem reaktiven multifunktionellen Epoxid, um so ein Epoxyaktiviertes Nanopartikel zu bilden.

37. Verfahren nach einem der Ansprüche 27 bis 36, wobei das reaktive multifunktionelle Epoxid aus Butan- und Ethandi-glycidylethern, Erythritolanhydrid, polyfunktionellen Polyglycerolpolyglycidylethern und Epichlorhydrin ausgewählt ist.

38. Verfahren nach einem der Ansprüche 27 bis 37, welches den weiteren Schritt der Umsetzung der Epoxyaktivierten Nanoteilchen mit reaktiven Gruppen mit ein oder mehreren bioaktiven Mitteln und/oder oberflächenmodifizierenden Mitteln umfasst, um Epoxy-derivatisierte Nanopartikel zu bilden.

39. Verfahren zur Herstellung eines Nanopartikels nach Anspruch 1, wobei das Verfahren die Schritte aufweist:
(a) Zubereiten einer Emulsion, welche mindestens ein biokompatibles, bioabbaubares Polymer, mindestens ein bioaktives Mittel und mindestens ein organisches Lösungsmittel und Wasser umfasst;
(b) Beschallen der Emulsion bei einer Temperatur unterhalb des Schmelzpunkts des biokompatiblen, bioabbaubaren Polymers und bei einer ausreichenden Energie, um eine stabile Emulsion zu bilden;
(c) Verdampfen des organischen Lösungsmittels um Nanopartikel zu erhalten; und
(d) Kontaktieren der Nanopartikel mit einer multifunktionellen Epoxid-Verbindung unter Bedingungen, bei denen die multifunktionelle Epoxidverbindung mit dem biokompatiblen, bioabbaubaren Polymer reagiert, um Epoxidaktivierte Nanopartikel zu erhalten.

40. Verfahren nach Anspruch 39, welches weiterhin den Schritt des Kontaktierens der Epoxid-aktivierten Nanopartikel mit mindestens einem bioaktiven Mittel und/oder oberflächenmodifizierenden Mittel unter Bedingungen umfasst, unter denen die Epoxid-aktivierten Nanopartikel mit dem bioaktiven Mittel oder dem oberflächenmodifizierenden Mittel reagieren, um Epoxy-derivatisierte Nanopartikel zu bilden.

41. Verfahren nach Anspruch 39 oder 40, wobei die Emulsion weiterhin mindestens ein Emulgiermittel umfasst.

42. Verfahren nach Anspruch 41, wobei die Nanoteilchen teilweise hydrolysiert sind, um an dem biokompatiblen, bioabbaubaren Polymer vor dem Schritt des Kontaktierens der Nanopartikel mit der multifunktionellen Epoxidverbindung reaktive Gruppen zu bilden.

43. Multiblock-Copolymer mit hydrophoben und hydrophilen Segmenten, die durch Epoxy-Bindungen verbunden und Hydroxy-terminiert oder Epoxid-terminiert sind und ein mittels Gelpermeationschromatographie und innere Viskosität bestimmtes Molekulargewicht von ungefähr 6.000 bis 100.000 aufweisen.

44. Multiblock-Copolymer nach Anspruch 43, wobei eine das hydrophobe Segment aus Polycaprolacton, Polylactiden, Polyglycoliden, Polymilchsäure-Polyglycolsäure-Copolymeren, bioabbaubaren Polyurethanen, Polyanhydriden und Polyaminosäuren ausgewählt ist.

45. Multiblock-Copolymer nach Anspruch 43 oder 44, wobei das hydrophile Segment ein Polyether ist, der aus Polyethylenglykol, Poloxameren und Poly(propylenoxid) ausgewählt ist.

46. Multiblock-Copolymer nach einem der Ansprüche 43 bis 45, welches ABA, BAB, Multiblock (AB)ₙ oder (BA)ₙ-Typen und Kombinationen hiervon sind, wobei der A-Block Polycaprolacton und der B-Block ein Polyether ist, der aus Polyethylenglycol, Poloxameren und Poly(propylenoxid) ausgewählt ist.

47. Multiblock-Copolymer nach einem der Ansprüche 43 bis 45, wobei ein hydrophobes Segment und/oder das hydrophile Segment expandiert ist, so dass viele Moleküle durch Epoxid-Bindungen aneinander gebunden sind.

48. Verfahren zur Herstellung von Block-Copolymeren mit hydrophilen und hydrophoben Segmenten, wobei das Verfahren die Schritte aufweist:
(a) Lösen eines ersten Polymer-Diols in einem organischen Lösungsmittel;
(b) Zugabe eines multifunktionellen Epoxids im Überschuss zu dem gelösten ersten Polymer-Diol, so dass eine der Epoxidgruppen des multifunktionellen Epoxids mit Hydroxylgruppen an den Enden des ersten Polymer-Diols reagiert, um ein erstes Polymer mit Epoxid-Endgruppen zu bilden (Block A); und
(c) Zugabe eines Überschusses eines zweiten Polymer-Diols (Block B) zu dem ersten Polymerblock A mit Hydroxylendgruppen, um ein hydroxyl-terminiertes Dreiblock-Copolymer vom BAB-Typ zu bilden.

49. Verfahren nach Anspruch 48, wobei ein weiterer Schritt/weitere Schritte der Expansion des Molekulargewichts eines Polymer-Diols vor der Verwendung in den Schritten (a) und/oder (b) vorgesehen ist, indem ein Überschuss des Polymer-Diols mit einem polyfunktionellen Epoxid umgesetzt wird.

50. Verfahren nach Anspruch 48 oder 49, wobei die Schritte (b) und (c) wiederholt werden, um Multiblock-Copolymere zu bilden.

51. Verfahren nach einem der Ansprüche 48 bis 51, welches den weiteren Schritt der Umsetzung des BAB-Typ-Dreiblock-Copolymers oder Multiblock-Polymers mit einem multifunktionellen Epoxid umfasst, um ein Polymer mit Epoxid-Endgruppen zu bilden.

52. Verfahren nach Anspruch 51, welches den weiteren Schritt der Umsetzung des Polymers mit Epoxid-Endgruppen mit einem bioaktiven Mittel und/oder einem oberflächenmodifizierenden Mittel mit mindestens einer funktionellen Gruppe daran, welche mit Epoxidgruppen reagiert, umfasst, um das bioaktive Mittel und/oder oberflächenmodifizierende Mittel an das Polymer mit Epoxid-Endgruppen zu binden.

53. Verfahren nach einem der Ansprüche 48 bis 52, welches den weiteren Schritt des Waschens oder des Umsetzens des Polymers umfasst, um weitere Epoxid-Reaktivität zu blockieren.

54. Verfahren nach einem der Ansprüche 48 bis 53, wobei das multifunktionelle Epoxid aus Butan- und Ethandiglycidylethern, Erythritolanhydrid, polyfunktionellen Polyglycerol-Polyglycidylethern und Epichlorhydrin ausgewählt ist.

55. Verfahren nach einem der Ansprüche 48 bis 54, wobei das erste Polymer-Diol ein hydrophobes Polymer-Diol ist, und das zweite Polymer-Diol ein hydrophiles Polymer-Diol ist.

56. Verfahren nach einem der Ansprüche 48 bis 54, wobei das erste Polymer-Diol ein hydrophiles Polymer-Diol und das zweite Polymer-Diol ein hydrophobes Polymer-Diol ist.

57. Verfahren nach Anspruch 55 oder 56, wobei das hydrophobe Polymer-Diol aus Polycaprolactonen, Polylactiden, Polyglycoliden und Polymilchsäure-Polyglycolsäure-Copolymeren ausgewählt ist.

58. Verfahren nach einem der Ansprüche 55 bis 57, wobei das hydrophile Polymer-Diol aus Polyethylenglycol, Poloxameren und Poly(propylenoxid) ausgewählt ist.

59. Verfahren zum Modifizieren der Oberfläche eines Polymers des Typs mit einer reaktiven Endgruppe, wobei das Verfahren die Schritte aufweist:
Kontaktieren des Polymeren mit einer multifunktionellen Epoxidverbindung in der Gegenwart eines Katalysators, um ein Epoxid-gekoppeltes Polymer zu bilden; und
Umsetzen des Epoxid-gekoppelten Polymers mit einem bioaktiven Mittel und/oder oberflächenmodifizierenden Mittel mit mindestens einer funktionellen Gruppe daran, welche mit Epoxidgruppen reagiert, um das bioaktive Mittel und/oder oberflächenmodifizierende Mittel kovalent an das Polymer zu binden.

## Revendications

1. Nanoparticule ayant un diamètre moyen inférieur à 300 nm et comprenant un noyau polymère biocompatible, biodégradable, au moins un agent actif au plan biologique et au moins un composé de liaison polyfonctionnel de type époxyde lié par covalence au polymère biodégradable,

2. Nanoparticule selon la revendication 1, dans laquelle le polymère biodégradable est un polymère synthétique.

3. Nanoparticule selon la revendication 2, dans laquelle le polymère synthétique est choisi parmi les polyesters, les polyéthers, les polyanhydrides, les poly(canoacrylates d'alkyle), les polyacrylamides, les polyorthoesters, les polyphosphazènes, les polyaminoacides, les polyuréthanes biodégradables et les copolymères de ceux-ci.

4. Nanoparticule selon la revendication 2, dans laquelle le polymère biodégradable est un copolymère multibloc à terminaisons hydroxy ou époxy comprenant au moins un segment hydrophobe et au moins un segment hydrophile, les segments hydrophobes et hydrophiles étant reliés par des composés de liaison polyfonctionnels de type époxyde, et ayant une masse moléculaire de 6 000 à 100 000 mesurée par chromatographie par perméation de gel et/ou par la viscosité intrinsèque.

5. Nanoparticule selon l'une quelconque des revendications 1 à 4, dans lequel le composé de liaison polyfonctionnel de type époxyde est un polyglycérolpolyglycidyléther polyfonctionnel.

6. Nanoparticule selon la revendication 4 ou la revendication 5, dans laquelle le segment hydrophobe est choisi parmi les polycaprolactones, les polylactides, les polyglycolides, les copolymères poly(acide lactique)-pcly(acide glycolique), les polyuréthanes biodégradables, les polyanhydrides et les polyaminoacides.

7. Nanoparticule selon l'une quelconque des revendications 4 à 6, dans laquelle le segment hydrophile est un polyéther choisi parmi le polyéthylèneglycol, les polaxomères et le poly(oxyde de propylène).

8. Nanoparticule selon l'une quelconque des revendications 4 à 7, dans lequel le copolymère multibloc est choisi parmi les polymères de type ABA, BAB, (AB)ₐ et (BA)ₙ multiblocs, et les combinaisons de ceux-ci, cù le bloc A est une polycaprolactone et le bloc B est choisi parmi le polyéthylèneglycol, les polaxomères et le poly(oxyde de propylène).

9. Nanoparticule selon la revendication 8, dans laquelle le copolymère multibloc est choisi parmi HO-PEG-EX352-PCL-EX252-PCL-EX252-PEG-OH ; HO-F68-EX252-PCL-EX252-PCL-F68-OH ; OH-PCL-EX252-F68-EX252-PCL-OH ; HO-PCL-EX252-PEG-EX252-PCL-OH ; et HO-PCL-EX252-PPO-EX252-PCL-OH.

10. Nanoparticule selon la revendication 2, dans laquelle le polymère biodégradable est un copolymère polylactique-polyglycolique ayant une masse moléculaire de 30 000 à 700 000 et une viscosité intrinsèque de C,5 à 10,5.

11. Nanoparticule selon l'une quelconque des revendications 4 à 9, comprenant en outre un agent actif au plan biologique et/ou un agent de modification de surface fixé par covalence au composé époxyde polyfonctionnel.

12. Nanoparticule selon la revendication 1, dans laquelle le polymère biodégradable est un polymère existant à l'état naturel.

13. Nanoparticule selon l'une quelconque des revendications 1 à 12, dans laquelle l'agent actif au plan biologique est au moins un agent pharmaceutique.

14. Nanoparticule selon la revendication 13, dans laquelle l'agent pharmaceutique est choisi parmi les agents cardiovasculaires, les vaccins à base de peptides ou de protéines et les acides nucléiques.

15. Nanoparticule selon la revendication 14, dans laquelle l'agent cardiovasculaire est choisi parmi les stimulateurs, les inhibiteurs, les antithrombines, les agents bloquant les canaux du calcium, les inhibiteurs de l'enzyme de transformation de l'antitensine (ACE), les immunodépresseurs, les huiles de poisson, les antagonistes du facteur de croissance, les inhibiteurs de cytocélétal, les agents anti-inflammatoires, les agents thrombolytiques, les agents anti-prolifératifs, une substance générique convenant au traitement de l'ADN ou d'anti-sens d'une maladie cardiovasculaire, les inhibiteurs de la protéinekinase, les inhibiteurs de la migration et/ou de la contraction des muscles lisses et les composés libérant de l'oxyde nitrique.

16. Nanoparticule selon la revendication 15, dans laquelle l'agent cardiovasculaire est la cytochalasine B.

17. Nanoparticule selon la revendication 14, dans laquelle l'agent anticancéreux est choisi parmi les agents alkylants, les antimétabolites, les produits naturels (par exemple les alcaloïdes), les toxines, les antibiotiques, les enzymes, les agents de modification d'une réponse biologique, les hormones, les antagonistes et une substance générique convenant au traitement du cancer.

18. Nanoparticule selon la revendication 14, dans laquelle l'acide nucléique est un gène ou un segment de gène ostéotropique ou un oligonucléotide.

19. Nanoparticule selon la revendication 18, dans laquelle le gène ou le segment-de gène ostéotropique est choisi parmi les protéines mophogéniques osseuses (BMP2 et 4 et d'autres), le facteur de croissance par transformation tel que TGF-β1-3, l'activine, les phosphoprotéines, l'ostéonectine, l'ostéopontine, la sialoprotéine osseuse, l'ostéocalcine, les protéines dépendant de la vitamine k, les glycoprotéines et le collagène (au moins I et II).

20. Nanoparticule selon la revendication 18 ou la revendication 19, comprenant en outre une protéine bloquant l'activité de la nucléase.

21. Nanoparticule selon l'une quelconque des revendications 1 à 20, dans laquelle l'agent de modification de surface est choisi parmi les polymères synthétiques, les biopolymères, les oligomères de faible masse moléculaire, les produits naturels, les tensioactifs et les composés époxyde polyfonctionnels.

22. Nanoparticule selon la revendication 21, dans laquelle le polymère synthétique est choisi parmi la carboxyméthylcellulose, la cellulose, l'acétate de cellulose, le phtalate de cellulose, le polyéthylèneglycol, le polyalcool vinylique), le phtalate d'hydroxypropylméthylcellulose, l'hydroxypropylcellulose, les sels de sodium ou de Calcium de carboxyméthylcellulose, la cellulose non cristalline, les polaxomères, les poloxamines, les dextranes, le DEAE-dextrane, la polyvinylpyrrolidone, le polystyrène et les silicates.

23. Nanoparticule selon la revendication 21, dans laquelle le produit naturel est choisi parmi les protéines, les peptides, les composés contenant du sucre, les lipides, l'acacia, la gélatine, la caséine, l'héparine, les albumines, les moyglobines, les hémoglobines, les fibrinogènes, la gomme adragante, le sorbitol, le mannitol, les polysaccharides, la pectine, la lécithine, les phospholipides, le cholestérol, la cire d'abeille, la graisse de laine, les huiles sulfonées et le savon de colophane.

24. Nanoparticule selon la revendication 21, dans laquelle le tensioactif est choisi parmi les tensioactifs non ioniques, anioniques et cationiques, y compris
les esters d'acides gras de sorbitane polyéthoxylés, les esters d'acides gras de sorbitane, les alcools gras, les alkylarylpolyéthersulfonates et le dioctylester de l'acide sulfosuccinique de sodium ;
le dodécylsulfate de sodium, les sels de sodium et de potassium d'acides gras, le poly(stéarate d'oxyle), le lauryléther polyéthoxylé, le sesquicléate de sorbitane, la triéthanolamine, les acides gras et les glycérolesters d'acides gras ; et
le bromure de didodécyldiméthylammonium, le bromure de cétyltriméthylammonium, le chlorure de benzalkonium, le chlorure d'hexadécyltriméthylammonium, le diméthyldodécylaminopropane et l'éthosulfate de N-cétyl-N-éthylmorpholinium.

25. Nanoparticule selon l'une quelconque des revendications 1 à 24, comprenant en outre un milieu de mise en suspension.

26. Nanoparticule selon l'une quelconque des revendications 1 à 25, comprenant en outre un agent d'encapsulation.

27. Procédé de fabrication d'une nanoparticule selon la revendication 1 contenant un agent hydrophobe actif au plan biologique, le procédé comprenant les étapes consistant à :
(a) dissoudre un polymère biocompatible, biodégradable dans un solvant organique ;
(b) dissoudre l'agent actif au plan biologique dans un solvant organique, les solutions contenant le polymère et l'agent actif au plan biologique combinées comprenant une phase organique ;
(c) ajouter la phase organique à une phase aqueuse ;
(d) soumettre à des ultrasons la phase organique et la phase aqueuse combinées à une température inférieure au point de fusion du polymère et à une énergie suffisante pour former une émulsion stable ;
(e) évaporer le solvant organique de l'émulsion stable ;
(f) séparer les nanoparticules résultantes de la phase aqueuse restante ; et
(g) modifier la surface des nanoparticules par mise en contact des nanoparticules, si nécessaire ou le cas échéant, après une hydrolyse partielle de la surface de celles-ci, avec un ccmposé époxyde polyfonctionnel réactif, pour former des nanoparticules époxy-activées.

28. Procédé de fabrication d'une nanoparticule selon la revendication 1 contenant un agent hydrophile actif au plan biologique, le procédé comprenant les étapes consistant à :
(a) dissoudre un polymère biocompatible, biodégradable dans un solvant organique non polaire ;
(b) dissoudre l'agent actif au plan biologique hydrophile dans un solvant organique semi-polaire ou dans une combinaison de solvants organiques polaires et semi-polaires, les solutions contenant le polymère et l'agent actif au plan biologique combinées comprenant une phase organique ;
(c) ajouter la phase organique à une phase aqueuse ;
(d) soumettre à des ultrasons la phase organique et la phase aqueuse combinées à une température inférieure au point de fusion du polymère et à une énergie suffisante pour former une émulsion stable ;
(e) évaporer le solvant organique de l'émulsion stable ;
(f) séparer les nanoparticules résultantes de la phase aqueuse restante ; et
(g) modifier la surface des nanoparticules par mise en contact des nanoparticules, si nécessaire ou le cas échéant, après une hydrolyse partielle de la surface de celles-ci, avec un composé époxyde polyfonctionnel réactif, pour former des nanoparticules époxy-activées.

29. Procédé selon la revendication 28, dans lequel la phase organique comprend en outre un agent favorisant le partage de l'agent actif au plan biologique hydrophile dans la phase organique lors de la solidification des nanoparticules résultantes.

30. Procédé selon l'une quelconque des revendications 27 à 29, dans lequel la phase aqueuse est une solution aqueuse d'un agent émulsifiant.

31. Procédé de fabrication d'une nanoparticule selon la revendication 1 contenant un agent actif au plan biologique contenant une grotéine/peptide soluble dans l'eau, le procédé comprenant les étapes consistant à :
(a) dissoudre un agent actif au plan biologique contenant une protéine/peptide soluble dans l'eau dans une solution aqueuse pour former une première phase aqueuse ;
(b) dissoudre au moins un polymère biocompatible, biodégradable, dans un solvant organique non polaire pour former une phase organique ;
(c) mélanger la première phase aqueuse avec la phase organique pour former une émulsion principale ;
(d) émulsifier l'émulsion principale dans une deuxième phase aqueuse pour former une émulsion eau dans huile dans eau ;
(e) évaporer le solvant organique de l'émulsion eau dans huile dans eau ;
(f) séparer les nanoparticules résultantes de la phase aqueuse restante ; et
(g) modifier la surface des nanoparticules par mise en contact des nanoparticules, si nécessaire ou le cas échéant, après une hydrolyse partielle de la surface de celles-ci, avec un composé époxyde polyfonctionnel réactif, pour former des nanoparticules époxy-activées.

32. Procédé selon la revendication 31, dans lequel l'étape (c) comprend un passage aux ultrasons avec une énergie suffisante pour former une émulsion principale stable.

33. Procédé selon la revendication 31 ou la revendication 32, dans lequel la deuxième phase aqueuse est une solution aqueuse d'un agent émulsifiant.

34. Procédé selon la revendication 33, dans lequel la solution aqueuse contient 1 à 3 % en poids/volume d'un agent émulsifiant.

35. Procédé de fabrication de nanoparticules extrêmement petites ayant un diamètre moyen de 10 à 50 nm selon la revendication 1, le procédé comprenant les étapes consistant à :
(a) dissoudre au moins un polymère biocompatible, biodégradable et au moins un agent actif au plan biologique dans un solvant commun comprenant un solvant organique non polaire et un solvant organique semi-polaire pour former une première phase organique ;
(b) ajouter la première phase organique à une phase aqueuse contenant au moins un agent émulsifiant pour former une émulsion ;
(c) passer aux ultrasons l'émulsion avec une sonde de dispositif de passage aux ultrasons à une sortie d'énergie d'environ 65 Watts à une température inférieure au point de fusion du polymère biocompatible, biodégradable pour produire une émulsion stable ;
(d) récupérer les nanoparticules ; et
(e) modifier la surface des nanoparticules par mise en contact des nanoparticules, si nécessaire ou le cas échéant, après une hydrolyse partielle de la surface de celles-ci, avec un composé époxyde polyfonctionnel réactif, pour former des nanoparticules époxy-activées.

36. Procédé de fabrication de nanoparticules extrêmement petites ayant un diamètre moyen de 10 à 50 nm selon la revendication 1, le procédé comprenant les étapes consistant à :
(a) dissoudre un agent actif au plan biologique soluble dans l'eau dans une solution aqueuse pour former une première solution aqueuse ;
(b) dissoudre au moins un polymère biccompatible, biodégradable dans un solvant commun comprenant un solvant organique non polaire et un solvant organique semi-polaire, pour former une première phase organique ;
(c) ajouter la première phase aqueuse à la première phase organique pour former une émulsion principale ;
(d) ajouter l'émulsion principale à une deuxième solution aqueuse pour former une émulsion eau dans huile dans eau ;
(d) soumettre à des ultrasons l'émulsicn eau dans huile dans eau avec une sonde de dispositif de passage aux ultrasons à une sortie d'énergie d'environ 65 watts à une température inféxieure au point de fusion du polymère biocompatible, biodégradable, pour produire une émulsion stable ;
(d) récupérer les nanoparticules ; et
(e) modifier la surface des nanoparticules par mise en contact des nanoparticules, si nécessaire ou le cas échéant, après une hydrolyse partielle de la surface de celles-ci, avec un composé époxyde polyfonctionnel réactif, pour former des nanoparticules époxy-activées.

37. Procédé selon l'une quelconque des revendications 27 à 36, dans lequel l'époxyde polyfonctionnel réactif est choisi parmi les butane- et les éthane-diglycidyléthers, l'anhydride d'érythritol, les polyglycérolpolyglycidyléthers polyfonctionnels et l'épichlorohydrine.

38. Procédé selon l'une quelconque des revendications 27 à 37, comprenant en outre la réaction des nanoparticules époxy-activées avec des groupes réactifs sur un ou plusieurs agents actifs au plan biologiques et/ou agents de modification de surface pour former des nanoparticules à fonctions époxy.

39. Procédé de fabrication d'une nanoparticule selon la revendication 1, le procédé comprenant les étapes consistant à :
(a) préparer une émulsion comprenant au moins un polymère biocompatible, biodégradable, au moins un agent actif au plan biologique et au moins un solvant organique et de l'eau ;
(b) soumettre aux ultrasons l'émulsion à une température inférieure au point de fusion du polymère biocompatible, biodégradable et à une énergie suffisante pour former une émulsion stable ;
(c) évaporer le solvant organique pour produire des nanoparticules ; et
(d) mettre en contact les nanoparticules avec un composé époxyde polyfonctionnel dans des conditions dans lesquelles le composé époxyde polyfonctionnel réagit avec le polymère biocompatible, biodégradable pour produire des nanoparticules époxyde-activées.

40. Procédé selon la revendication 39, comprenant en outre l'étape de mise en contact des nanoparticules époxyde-activées avec au moins un agent actif au plan biologique et/ou un agent de modification de surface dans des conditions dans lesquelles les nanoparticules époxyde-activées réagissent avec l'agent actif au plan biologique ou avec l'agent de modification de surface pour former des nanoparticules à fonctions époxy.

41. Procédé selon la revendication 39 ou la revendication 40, dans lequel l'émulsion comprend en outre au moins un agent émulsifiant.

42. Procédé selon la revendication 41, dans lequel les nanoparticules sont partiellement hydrolysées pour créer des groupes réactifs sur le polymère biocompatible, biodégradable avant l'étape de mise en contact des nanoparticules avec le composé époxyde polyfonctionnel.

43. Copolymère multibloc ayant des segments hydrophobes et hydrophiles reliés par des liaisons épcxy et étant à terminaisons hydroxy ou à terminaisons époxyde et ayant une masse moléculaire d'environ 6 000 à 100 000 mesurée par chromatographie par perméation de gel et/ou par la viscosité intrinsèque.

44. Copolymère multibloc selon la revendication 43, dans lequel le segment hydrophobe est choisi parmi les polycaprolactones, les polylactides, les polyglycolides, les copolymères poly(acide lactique)-poly(acide glycolique), les polyuréthanes biodégradables, les polyanhydrides et les polyaminoacides.

45. Copolymère multibloc selon la revendication 43 ou la revendication 44, dans lequel le segment hydrophile est un polyéther choisi parmi le polyéthylèneglycol, les polaxomères et le poly(oxyde de propylène).

46. Copolymère multibloc selon l'une quelconque des ravendications 43 à 45, qui est un polymère de type ABA, BAB, (Ag)ₐ et (BA)ₙ multibloc, et les combinaisons de ceux-ci, où le bloc A est une polycaprolactone et le bloc B est choisi parmi le polyéthylèneglycol, les polaxomères et le poly(oxyde de propylène).

47. Copolymère multibloc selon l'une quelconque des revendications 43 à 45, dans lequel un segment hydrophobe et/ou un segment hydrophile sont dilaté de sorte que plusieurs molécules soient liées les unes aux autres par des liaisons époxy.

48. Procédé de fabrication de copolymères séquencés ayant des segments hydrophiles et hydrophobes, le procédé comprenant les étapes consistant à :
(a) dissoudre un premier polymère-diol dans un solvant organique ;
(b) ajouter un composé époxyde polyfonctionnel en excès au premier polymère-diol dissous de sorte que l'un des groupes époxyde du composé époxyde polyfonctionnel réagisse avec les groupes hydroxyle aux extrémités du premier polymère-diol pour former un premier polymère à coiffe terminale époxyde (bloc A) ; et
(c) ajouter un excès d'un deuxième polymère-diol (bloc B) au premier polymère à coiffe terminale époxyde bloc A pour former un copolymère tribloc de type B-A-B à terminaisons hydroxyle.

49. Procédé selon la revendication 48, qui comprend en outre le ou les étapes d'accroissement de la masse moléculaire d'un polymère-diol, avant son utilisation dans les étapes (a) et/ou (b), par réaction d'un excès du polymère-diol avec un composé époxyde polyfonctionnel.

50. Procédé selon la revendication 48 ou la revendication 49, dans lequel les étapes (b) et (c) sont répétées pour former des copolymères multiblocs.

51. Procédé selon l'une quelconque des revendications 48 à 51, comprenant l'étape supplémentaire consistant à faire réagir le copolymère tribloc de type B-A-B ou le polymère multibloc avec un composé époxyde polyfonctionnel pour former un polymère à coiffe terminale époxyde.

52. Procédé selon la revendication 51, comprenant l'étape supplémentaire consistant à faire réagir le polymère à coiffe terminale époxyde avec un agent actif au plan biologique et/ou un agent de modification de surface contenant au moins un groupe fonctionnel qui réagit avec les groupes époxyde pour fixer par covalence l'agent actif au plan biologique et/ou l'agent de modification de surface au polymère à coiffe terminale époxyde.

53. Procédé selon l'une quelconque des revendications 48 à 52, comprenant l'étape supplémentaire consistant à laver ou à faire réagir le polymère pour davantage bloquer la réactivité époxyde.

54. Procédé selon l'une quelconque des revendications 48 à 53, dans lequel le composé époxyde polyfonctionnel est choisi parmi les butane- et les éthane-diglycidyléthers, l'anhydride d'érythritol, les polyglycérolpolyglycidyl-éthers polyfonctionnels et l'épichlorohydrine.

55. Procédé selon l'une quelconque des revendications 48 à 54, dans lequel le premier polymère-diol est un polymère-diol hydrophobe et le deuxième polymère-diol est un polymère-diol hydrophile.

56. Procédé selon l'une quelconque des revendications 48 à 54, dans lequel le premier polymère-diol est un polymère-diol hydrophile et le deuxième polymère-diol est un polymère-diol hydrophobe.

57. Procédé selon la revendication 55 ou la revendication 56, dans lequel le polymère-diol hydrophile est choisi parmi les polycaprolactones, les polylactides, les polyglycolides et un copolymére poly(acide lactique)-poly(acide glycolique).

58. Procédé selon l'une quelconque des revendications 55 à 57, dans lequel le polymère-diol hydrophile est choisi parmi le polyéthylèneglycol, les polaxomères et le poly(oxyde de propylène).

59. Procédé de modification de la surface d'un polymère du type contenant un groupe terminal réactif, le procédé comprenant les étapes consistant à :
mettre en contact le polymère avec un composé époxyde polyfonctionnel en présence d'un catalyseur pour former un polymère coupé à un époxyde ; et
faire réagir le polymère couplé à un époxyde avec un agent actif au plan biologique et/ou un agent de modification de surface contenant au moins un groupe fonctionnel qui réagit avec les groupes époxyde pour lier par covalence l'agent actif au plan biologique et/ou l'agent de modification de surface au polymère.
